# EUROPÄISCHE PATENTANMELDUNG

(11) **EP 1 686 127 A1**
(43) Veröffentlichungstag der Anmeldung: **02.08.2006**
(21) Anmeldenummer: 05022495.5
(22) Anmeldetag: 02.10.1997
(51) Int. Cl.: C07D 405/04, A61K 31/415, C07D 405/14, C07D 409/14, C07D 401/04, C07D 403/04, C07D 413/04, C07D 417/04, C07D 409/04, C07D 263/32

(54) **Neue Heterocyclylmethyl-substituierte Pyrazolderivate und ihre Verwendung zur Behandlung von Herz-Kreislauf-Erkrankungen**

(30) Priorität: 14.10.1996 DE 19642319; 14.10.1996 DE 19642320; 14.10.1996 DE 19642322; 14.10.1996 DE 19642323
(62) Teilanmeldung aus: 97912102.7
(71) Anmelder: Bayer HealthCare AG, 51368 Leverkusen (DE)
(72) Erfinder: Straub, Alexander, Dr., 42117 Wuppertal (DE); Fürstner, Chantal, Dr., 45478 Mühlheim (DE); Niewöhner, Ulrich, Dr., deceased (DE); Jaetsch, Thomas, Dr., 50668 Köln (DE); Feurer, Achim, Dr., 69259 Wilhelmsfeld (DE); Kast, Raimund, Dr., 42349 Wuppertal (DE); Stasch, Johannes-Peter, Dr., 42651 Solingen (DE); Perzborn, Elisabeth, Dr., 42327 Wuppertal (DE); Hütter, Joachim, Dr., 42349 Wuppertal (DE); Dembowsky, Klaus, Dr., 81249 München (DE); Arlt, Dieter, Dr., 32657 Lemgo (DE)

(57) **Zusammenfassung**

Die vorliegende Erfindung betrifft neue Heterocyclylmethyl-substituierte Pyrazol-Derivate, Verfahren zu ihrer Herstellung und ihre Verwendung als Arzneimittel, insbesondere als Arzneimittel zur Behandlung von Herz-Kreislauf-Erkrankungen.

## Beschreibung

Die vorliegende Erfindung betrifft neue Heterocyclylmethyl-substituierte Pyrazol-Derivate, Verfahren zu ihrer Herstellung und ihre Verwendung als Arzneimittel, insbesondere als Arzneimittel zur Behandlung von Herz-Kreislauf-Erkrankungen.

Es ist bereits bekannt, da 1-Benzyl-3-(substituierte heteroaryl)-kondensierte Pyrazol-Derivate die stimulierte Thrombozytenaggregation in vitro inhibieren (vgl. EP 667 345 A1).

### I

Die vorliegende Erfindung betrifft in der mit I (römisch eins) bezeichneten Ausführungsform neue Heterocyclylmethyl-substituierte Pyrazolderivate der allgemeinen Formel (I-I), 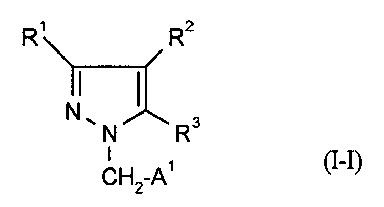 in welcher
- R¹: für einen 5- gliedrigen aromatischen Heterocyclus mit 1 Heteroatom aus der Reihe S, N und/oder O oder für Phenyl steht, die gegebenenfalls bis zu 3-fach gleich oder verschieden durch Formyl, Carboxyl, Mercaptyl, Hydroxy, geradkettiges oder verzweigtes Acyl, Alkylthio, Alkoxy oder Alkoxycarbonyl mit jeweils bis zu 6 Kohlenstoffatomen, Nitro, Cyano, Halogen, Phenyl oder geradkettiges oder verzweigtes Alkyl mit bis zu 6 Kohlen-stoffatomen substituiert sind, das seinerseits durch Hydroxy, Amino, Carboxyl, geradkettiges oder verzweigtes Acyl, Alkoxy, Alkoxycarbonyl oder Acylamino mit jeweils bis zu 5 Kohlenstoffatomen oder durch einen Rest der Formel -OR⁴ substituiert sein kann,
worin
- R⁴: geradkettiges oder verzweigtes Acyl mit bis zu 5 Kohlenstoffatomen oder eine Gruppe der Formel -SiR⁵R⁶R⁷ bedeutet,
worin
- R⁵, R⁶ und R⁷: gleich oder verschieden sind und Aryl mit 6 bis 10 Kohlenstoffatomen oder Alkyl mit bis zu 6 Kohlenstoffatomen bedeuten,
und/oder durch einen Rest der Formel 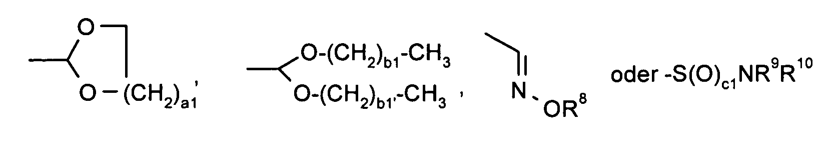 substituiert sind, worin
- b1 und b1': gleich oder verschieden sind und eine Zahl 0, 1, 2 oder 3 bedeuten,
- a1: eine Zahl 1, 2 oder 3 bedeutet,
- R⁸: Wasserstoff oder geradkettiges oder verzweigtes Alkyl mit bis zu 4 Kohlenstoff-atomen bedeutet,
- c1: eine Zahl 1 oder 2 bedeutet und
- R⁹ und R¹⁰: gleich oder verschieden sind und Wasserstoff oder geradkettiges oder verzweigtes Alkyl mit bis zu 10 Kohlenstoffatomen bedeutet, das gegebenenfalls durch Cycloalkyl mit 3 bis 8 Kohlenstoffatomen oder durch Aryl mit 6 bis 10 Kohlen-stoffatomen substituiert ist, das seinerseits durch Halogen substituiert sein kann oder Aryl mit 6 bis 10 Kohlenstoffatomen bedeuten, das gegebenenfalls durch Halogen substituiert ist oder Cycloalkyl mit 3 bis 7 Kohlenstoffatomen bedeuten oder
worin
- R¹¹: Wasserstoff, geradkettiges oder verzweigtes Alkyl mit zu 4 Kohlenstoffatomen oder einen Rest der Formel 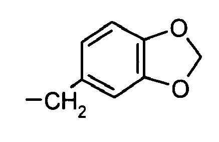
bedeutet,
oder Benzyl oder Phenyl bedeutet, wobei die Ringsysteme gegebenenfalls durch Halogen substituiert sind,
- R² und R³: unter Einbezug der Doppelbindung einen 5-gliedrigen aromatischen Heterocyclus mit einem Heteroatom aus der Reihe S, N und/oder O oder einen Phenylring bilden, die
gegebenenfalls bis zu 3-fach gleich oder verschieden durch Formyl, Carboxyl, Hydroxy, Amino, geradkettiges oder verzweigtes Acyl, Alkoxy oder Alkoxycarbonyl mit jeweils bis zu 6 Kohlenstoffatomen, Nitro, Cyano, Azido, Halogen, Phenyl oder geradkettiges oder ver-zweigtes Alkyl mit bis zu 6 Kohlenstoffatomen substituiert sind, wobei das Alkyl seinerseits durch Hydroxy, Amino, Carboxyl, geradkettiges oder verzweigtes Acyl, Alkoxy oder Alkoxycarbonyl mit jeweils bis zu 5 Kohlenstoffatomen substituiert sein kann,
und/oder gegebenenfalls durch einen Rest der Formel -S(O)_{c1'}NR^{9'}R^{10'} substituiert sind, worin c_{1'}, R^{9'} und R^{10'} die oben angegebene Bedeutung von c₁, R⁹ und R¹⁰ haben und mit dieser gleich oder verschieden sind,
- A¹: für einen 5- bis 6-gliedrigen aromatischen oder gesättigten Heterocyclus mit bis zu 3 Hetero-atomen aus der Reihe S, N und/oder O steht, der gegebenenfalls bis zu 3-fach gleich oder verschieden durch Mercaptyl, Hydroxy, Formyl, Carboxyl, geradkettiges oder verzweigtes Acyl, Alkylthio, Alkyloxyacyl, Alkoxy oder Alkoxycarbonyl mit jeweils bis zu 6 Kohlen-stoffatomen, Nitro, Cyano, Trifluormethyl, Azido, Halogen, Phenyl oder geradkettiges oder verzweigtes Alkyl mit bis zu 6 Kohlenstoffatomen substituiert ist, das seinerseits durch Hydroxy, Carboxyl, geradkettiges oder verzweigtes Acyl, Alkoxy oder Alkoxycarbonyl mit jeweils bis zu 5 Kohlenstoffatomen substituiert sein kann,
und/oder durch eine Gruppe der Formel -(CO)_{d1}-NR¹²R¹³ substituiert ist,
worin
- d1: eine Zahl 0 oder 1 bedeutet,
- R¹² und R¹³: gleich oder verschieden sind und Wasserstoff, Phenyl, Benzyl oder geradkettiges oder verzweigtes Alkyl oder Acyl mit jeweils bis zu 5 Kohlenstoffatomen bedeuten,
und deren isomere Formen, Salze und deren N-Oxide.

Die erfindungsgemäßen Verbindungen der allgemeinen Formel (I-I) können auch in Form ihrer Salze vorliegen. Im Allgemeinen seien hier Salze mit organischen oder anorganischen Basen oder Säuren genannt.

Im Rahmen der Ausführungsform (I) vorliegenden Erfindung werden physiologisch unbedenkliche Salze bevorzugt. Physiologisch unbedenkliche Salze der Heterocyclylmethyl-substituierten Pyrazol-Derivate können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen.

Heterocyclus steht im Rahmen der Ausführungsform I der Erfindung und in Abhängigkeit der oben aufgeführten Substituenten im Allgemeinen für einen 5- bis 6-gliedrigen Heterocyclus, der im Fall R¹ 1 Heteroatom im 5-Ring und im Fall A bis zu 3 Heteroatome aus der Reihe S, N und/oder O enthalten kann. Beispielsweise seien genannt: Pyridazinyl, Pyridyl, Pyrimidyl, Thienyl, Furyl, Morpholinyl, Pyrrolyl, Thiazolyl, Oxazolyl, Imidazolyl, Tetrahydropyranyl oder Tetrahydrofuranyl. Bevorzugt sind Furyl, Pyridyl, Thienyl, Pyrrolyl, Pyrimidyl, Pyridazinyl, Morpholinyl, Tetrahydropyranyl oder Tetrahydrofuranyl.

Bevorzugt sind erfindungsgemäße Verbindungen der allgemeinen Formel (I-I),
in welcher
- R¹: für Furyl, Pyrrolyl, Thienyl oder Phenyl steht, die gegebenenfalls bis zu 2-fach gleich oder verschieden durch Formyl, Carboxyl, Hydroxy, geradkettiges oder verzweigtes Acyl, Alkoxy oder Alkoxycarbonyl mit jeweils bis zu 5 Kohlenstoffatomen, Nitro, Cyano, Azido, Fluor, Chlor, Brom, Phenyl oder geradkettiges oder verzweigtes Alkyl mit bis zu 5 Kohlen-stoffatomen substituiert sind, das seinerseits durch Hydroxy, Amino, Carboxyl, geradkettiges oder verzweigtes Acyl, Alkoxy, Alkoxycarbonyl oder Acylamino mit jeweils bis zu 4 Kohlenstoffatomen oder durch einen Rest der Formel -OR⁴ substituiert sein kann,
worin
- R⁴: geradkettiges oder verzweigtes Acyl mit bis zu 4 Kohlenstoffatomen bedeutet,
und/oder durch einen Rest der Formel 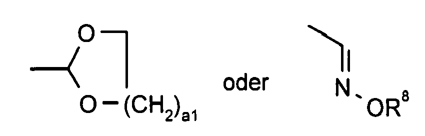 substituiert sind,
worin
- a1: eine Zahl 1, 2 oder 3 bedeutet,
- R⁸: Wasserstoff oder geradkettiges oder verzweigtes Alkyl mit bis zu 3 Kohlenstoff atomen bedeutet,
- R² und R³: unter Einbezug der Doppelbindung einen Furyl-, Thienyl- oder Phenylring bilden, die gegebenenfalls bis zu 3-fach gleich oder verschieden durch Formyl, Carboxyl, Hydroxy, Amino, geradkettiges oder verzweigtes Acyl, Alkoxy oder Alkoxycarbonyl mit jeweils bis zu 5 Kohlenstoffatomen, Nitro, Cyano, Azido, Fluor, Chlor, Brom, Phenyl oder geradkettiges oder verzweigtes Alkyl mit bis zu 5 Kohlenstoffatomen substituiert sind, das seinerseits durch Hydroxy, Amino, Carboxyl, geradkettiges oder verzweigtes Acyl, Alkoxy oder Alkoxycarbonyl mit jeweils bis zu 4 Kohlenstoffatomen substituiert sein kann,
- A¹: für Tetrahydropyranyl, Thienyl, Furyl, Tetrahydrofuranyl, Pyrazinyl, Morpholinyl, Pyrimidyl, Pyridazinyl oder Pyridyl steht, die gegebenenfalls bis zu 2-fach gleich oder verschieden durch Hydroxy, Formyl, Carboxyl, geradkettiges oder verzweigtes Acyl, Alkylthio, Alkyloxyacyl, Alkoxy oder Alkoxycarbonyl mit jeweils bis zu 4 Kohlenstoff-atomen, Fluor, Chlor, Brom, Nitro, Cyano, Trifluormethyl oder geradkettiges oder ver-zweigtes Alkyl mit bis zu 4 Kohlenstoffatomen substituiert sind, das seinerseits durch Hydroxy, Carboxyl, geradkettiges oder verzweigtes Acyl, Alkoxy oder Alkoxycarbonyl mit jeweils bis zu 4 Kohlenstoffatomen substituiert sein kann,
und/oder durch eine Gruppe der Formel -(CO)_{d1}-NR¹²R¹³ substituiert sind,
worin

- d1: eine Zahl 0 oder 1 bedeutet,
- R¹² und R¹³: gleich oder verschieden sind und Wasserstoff, Phenyl, Benzyl oder geradkettiges oder verzweigtes Alkyl oder Acyl mit jeweils bis zu 4 Kohlenstoffatomen bedeuten,
und deren isomere Formen und Salze und deren N-Oxide.

Besonders bevorzugt sind erfindungsgemäße Verbindungen der allgemeinen Formel (I-I),
in welcher
- R¹: für Furyl, Pyrryl, Thienyl oder Phenyl steht, die gegebenenfalls bis zu 2-fach gleich oder verschieden durch Formyl, geradkettiges oder verzweigtes Acyl, Alkoxy oder Alkoxy-carbonyl mit jeweils bis zu 4 Kohlenstoffatomen oder geradkettiges oder verzweigtes Alkyl mit bis zu 4 Kohlenstoffatomen substituiert sind, das seinerseits durch Hydroxy, Carboxyl, Amino, geradkettiges oder verzweigtes Acyl, Alkoxy, Alkoxycarbonyl oder Acylamino mit jeweils bis zu 3 Kohlenstoffatomen substituiert sein kann,
und/oder durch einen Rest der Formel 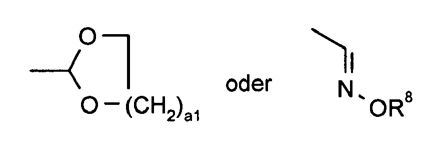
substituiert sind,
worin
- a1: eine Zahl 1 oder 2 bedeutet,
- R⁸: Wasserstoff oder Methyl bedeutet,

- R² und R³: unter Einbezug der Doppelbindung einen Furyl-, Thienyl- oder Phenylring bilden, die gegebenenfalls bis zu 2-fach gleich oder verschieden durch Formyl, Carboxyl, Hydroxy, Amino, geradkettiges oder verzweigtes Acyl, Alkoxy oder Alkoxycarbonyl mit jeweils bis zu 4 Kohlenstoffatomen, Nitro, Cyano, Fluor, Chlor, Phenyl oder geradkettiges oder verzweigtes Alkyl mit bis zu 3 Kohlenstoffatomen substituiert sind, das seinerseits durch Hydroxy, Amino, Carboxyl, geradkettiges oder verzweigtes Acyl, Alkoxy oder Alkoxycarbonyl mit jeweils bis zu 3 Kohlenstoffatomen substituiert sein kann,
- A¹: für Tetrahydropyranyl, Tetrahydrofuranyl, Thienyl, Pyrimidyl, Pyrazinyl, Pyridazinyl, Furyl oder Pyridyl steht, die gegebenenfalls bis zu 2-fach gleich oder verschieden durch Formyl, Carboxyl, geradkettiges oder verzweigtes Acyl, Alkylthio, Alkyloxyacyl, Alkoxy oder Alkoxycarbonyl mit jeweils bis zu 3 Kohlenstoffatomen, Fluor, Chlor, Brom, Nitro, Cyano, Trifluormethyl, oder geradkettiges oder verzweigtes Alkyl mit bis zu 3 Kohlenstoffatomen substituiert sind, das seinerseits durch Hydroxy, Carboxyl, geradkettiges oder verzweigtes Acyl, Alkoxy oder Alkoxycarbonyl mit jweils bis zu 3 Kohlenstoffatomen substituiert sein kann,
und/oder durch eine Gruppe der Formel -(CO)_{d1}-NR¹²R¹³ substituiert sind,
worin
- d1: eine Zahl 0 oder 1 bedeutet,
- R¹² und R¹³: gleich oder verschieden sind und Wasserstoff oder geradkettiges oder verzweigtes Alkyl oder Acyl mit jeweils bis zu 3 Kohlenstoffatomen bedeuten,
und deren isomere Formen und Salze und deren N-Oxide.

Ganz besonders bevorzugt sind erfindungsgemäße Verbindungen der allgemeinen Formel (I-I), in welcher
- R¹: für Furyl steht, das gegebenenfalls durch Formyl oder durch den Rest der Formel -CH₂-OH
oder 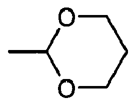 substituiert ist,
- R² und R³: unter Einbezug der Doppelbindung einen durch Phenyl, Fluor oder Nitro substituierten Phenylring bilden,
- A¹: für Furyl, Pyridyl, Pyrimidyl, Pyridazinyl, Thienyl, Tetrahydrofuranyl oder Tetrahydro-pyranyl steht, die gegebenenfalls durch Chlor, Brom, Methoxy, Methoxycarbonyl oder
Carboxyl substituiert sind
und deren Salze, isomere Formen und N-Oxide.

Die Erfindung betrifft weiterhin Verfahren zur Herstellung der erfindungsgemäßen Verbindungen der allgemeinen Formel (I-I), dadurch gekennzeichnet, dass man

### [A1] Verbindungen der allgemeinen Formel (I-II)

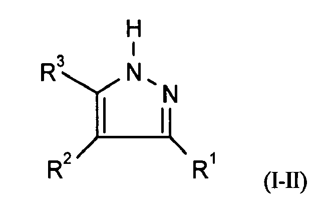

in welcher
R¹, R² und R³ die oben angegebene Bedeutung haben,
mit Verbindungen der allgemeinen Formel (I-III)

D¹-CH₂-A¹ (I-III)

in welcher
- A¹: die oben angegebene Bedeutung hat
und
- D¹: für Triflat oder Halogen, vorzugsweise für Brom steht,
in inerten Lösemitteln, gegebenenfalls in Anwesenheit einer Base umsetzt,
oder

### [B1] Verbindungen der allgemeinen Formel (I-IV)

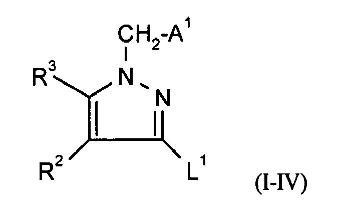

in welcher
A¹, R² und R³ die oben angegebene Bedeutung haben,
und
- L¹: für einen Rest der Formel -SnR¹⁴R¹⁵R¹⁶, ZnR¹⁷, Iod oder Triflat steht,
worin
- R¹⁴, R¹⁵ und R¹⁶: gleich oder verschieden sind und geradkettiges oder verzweigtes Alkyl mit bis zu 4 Kohlenstoffatomen bedeuten,
und
- R¹⁷: Halogen bedeutet,
mit Verbindungen der allgemeinen Formel (I-V)

R¹-T¹ (I-V)

in welcher
- R¹: die oben angegebene Bedeutung hat
und
im Fall L¹ = SnR¹⁴R¹⁵R¹⁶ oder ZnR¹⁷
- T¹: für Triflat oder für Halogen, vorzugsweise für Brom steht,
und
im Fall L¹ = Jod oder Triflat
- T¹: für einen Rest der Formel SnR^{14'}R^{15'}R^{16'}, ZnR^{17'} oder BR¹⁸R¹⁹steht,
worin
- R^{14'}, R^{15'}, R^{16'}, und R^{17'}: die oben angegebene Bedeutung von R¹⁴ , R¹⁵, R¹⁶ und R¹⁷ haben und mit dieser gleich oder verschieden sind,
- R¹⁸ und R¹⁹: gleich oder verschieden sind und Hydroxy, Aryloxy mit 6 bis 10 Kohlen-stoffatomen oder geradkettiges oder verzweigtes Alkyl oder Alkoxy mit jeweils bis zu 5 Kohlenstoffatomen bedeuten, oder gemeinsam einen 5- oder 6-gliedrigen carbocyclischen Ring bilden,
in einer palladiumkatalysierten Reaktion in inerten Lösemitteln umsetzt,
und im Fall der Reste -S(O)_{c1}NR⁹R¹⁰ und -S(O)_{c1},NR^{9'}R^{10'} ausgehend von den unsubstituierten Verbindungen der allgemeinen Formel (I-I) zunächst mit Thionylchlorid umsetzt und abschließend die Aminkomponente einsetzt
und gegebenenfalls die unter R¹, R², R³ und/oder A¹ aufgeführten Substituenten nach üblichen Methoden, vorzugsweise durch Reduktion, Oxidation, Abspaltung von Schutzgruppen und/oder nucleophiler Substitution variiert oder einführt.

Die erfindungsgemäßen Verfahren können durch folgendes Formelschema beispielhaft erläutert werden:

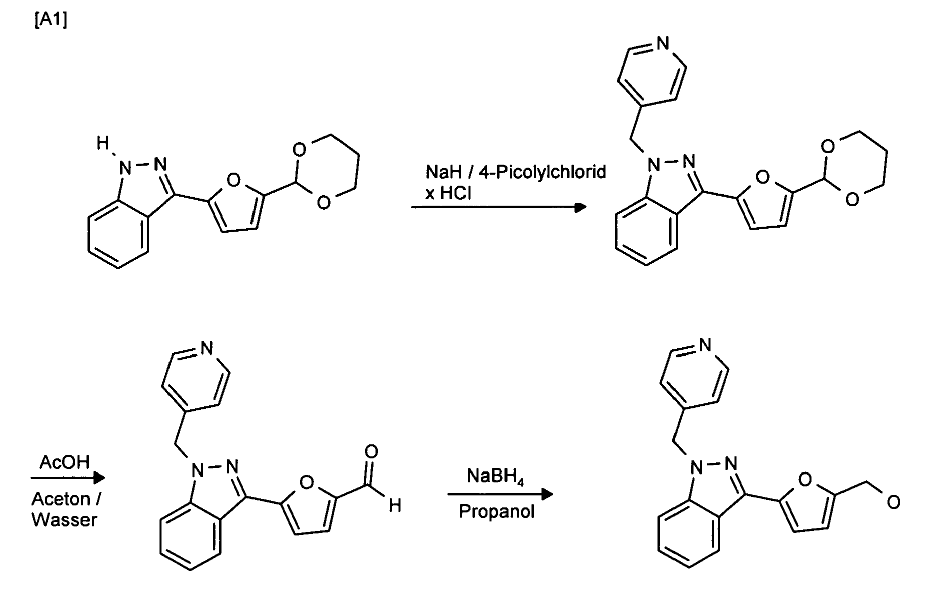

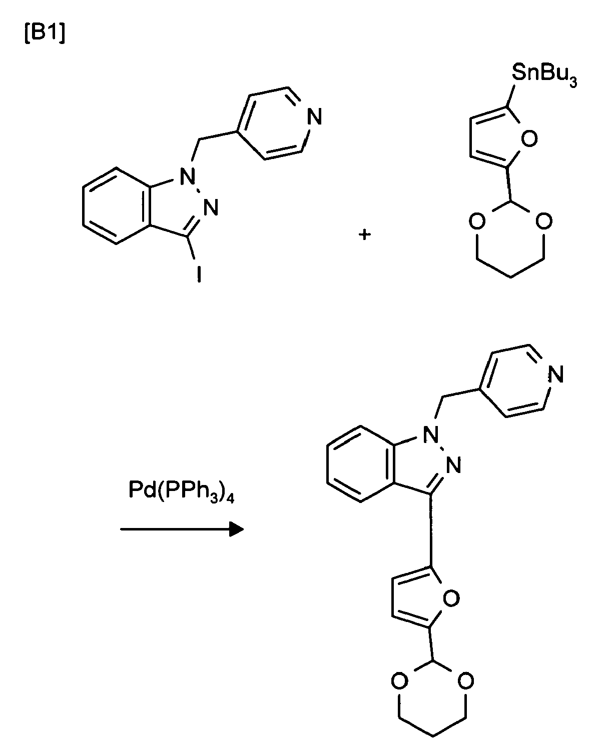

Als Lösemittel für die einzelnen Schritte des Verfahrens [A1] eignen sich hierbei inerte organische Lösemittel, die sich unter den Reaktionsbedingungen nicht verändern. Hierzu gehören Ether, wie Diethylether oder Tetrahydrofuran, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Tetrahydrofuran, Toluol oder Dimethylformamid.

Als Basen für das erfindungsgemäße Verfahren können im Allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium und deren Hydride wie Natriumhydrid einzusetzen. Bevorzugt sind Natrium- und Kaliumcarbonat, Triethylamin und Natriumhydrid.

Die Base wird in einer Menge von 1 mol bis 5 mol, bevorzugt von 1 mol bis 3 mol, bezogen auf 1 mol der Verbindung der allgemeinen Formel (I-II) eingesetzt.

Die Umsetzung wird im Allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im Allgemeinen arbeitet man bei Normaldruck.

Als Lösemittel für das Verfahren [B1] eignen sich hierbei inerte organische Lösemittel, die sich unter den Reaktionsbedingungen nicht verändern. Hierzu gehören Ether, wie Diethylether oder Tetrahydrofuran, DME, Dioxan, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Tetrahydrofuran, Dimethylformamid, Toluol, Dioxan oder Dimethoxyethan.

Die Reaktion wird im Allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im Allgemeinen arbeitet man bei Normaldruck.

Als Palladiumverbindungen im Rahmen der vorliegenden Erfidung eignen sich im Allgemeinen PdCl₂(P(C₆H₅)₃)₂, Palladium-bis-dibenzylidenaceton (Pd(dba)₂), [1,1'-Bis-(diphenylphosphino)-ferrocen]-Palladium(II)-chlorid (Pd(dppf)Cl₂) oder Pd(P(C₆H₅)₃)₄. Bevorzugt ist Pd(P(C₆H₅)₃)₄.

Die Verbindungen der allgemeinen Formeln (I-III) und (I-V) sind an sich bekannt oder nach üblichen Methoden herstellbar.

Die Verbindungen der allgemeinen Formel (I-II) sind teilweise bekannt oder neu und können dann hergestellt werden, indem man Verbindungen der allgemeinen Formel (I-VI) 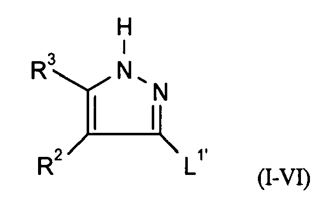 in welcher
- R² und R³: die oben angegebene Bedeutung haben
und
- L^{1'}: die oben angegebene Bedeutung von L¹ hat und mit dieser gleich oder verschieden ist,
mit Verbindungen der allgemeinen Formel (I-V) in Analogie zu dem oben aufgeführten Verfahren [B1] umsetzt.

Die Verbindungen der allgemeinen Formel (I-IV) sind teilweise bekannt oder im Fall der Stannyle neu und können dann beispielsweise hergestellt werden, indem man die Verbindungen der allgemeinen Formel (I-IVa) 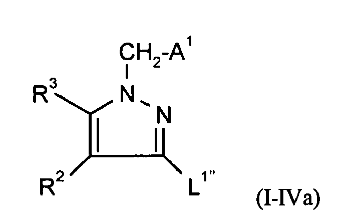 in welcher
- R², R³ und A¹: die oben angegebene Bedeutung haben,
- L^{1"}: für Triflat oder Halogen, vorzugsweise für Iod steht,
mit Verbindungen der allgemeinen Formel (I-VII)

(SnR¹⁴R¹⁵R¹⁶)₂ (I-VII)

in welcher
R¹⁴ , R¹⁵, R¹⁶ die oben angegebene Bedeutung haben
wie oben beschrieben palladiumkatalysiert umsetzt.

Die Verbindungen der allgemeinen Formeln (I-IVa) und (I-VII) sind an sich bekannt oder nach üblichen Methoden herstellbar.

Die Reduktionen werden im Allgemeinen mit Reduktionsmitteln, bevorzugt mit solchen, die für die Reduktion von Carbonyl zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat, Diisobutylaluminiumhydrid oder Lithiumaluminiumhydrid durchgeführt. Ganz besonders bevorzugt wird die Reduktion mit Diisobutylaluminiumhydrid und Natriumborhydrid durchgeführt.

Das Reduktionsmittel wird im Allgemeinen in einer Menge von 1 mol bis 6 mol, bevorzugt von 1 mol bis 4 mol bezogen auf 1 mol der zu reduzierenden Verbindungen, eingesetzt.

Die Reduktion verläuft im Allgemeinen in einem Temperaturbereich von -78°C bis +50°C, bevorzugt von -78°C bis 0°C, im Falle des DIBAH, 0°C, Raumtemperatur im Falle des NaBH₄, besonders bevorzugt bei -78°C, jeweils in Abhängigkeit von der Wahl des Reduktionsmittels sowie Lösemittel.

Die Reduktion verläuft im Allgemeinen bei Normaldruck, es ist aber auch möglich bei erhöhtem oder erniedrigtem Druck zu arbeiten.

Die Abspaltung der Schutzgruppe erfolgt im Allgemeinen in einem der oben aufgeführten Alkohole und/oder THF oder Aceton, vorzugsweise Methanol / THF in Anwesenheit von Salzsäure oder Trifluoressigsäure oder Toluolsulfonsäure in einem Temperaturbereich von 0°C bis 70°C, vorzugsweise bei Raumtemperatur und Normaldruck.

Im Fall, dass die Reste der Formeln -S(O)_{c1}NR⁹R¹⁰ und -S(O)_{c1}'NR^{9'}R^{10'} vorliegen, werden die entsprechenden unsubstituierten Verbindungen zunächst mit Thionylchlorid umgesetzt. In einem zweiten Schritt erfolgt die Umsetzung mit den Aminen in einem der oben aufgeführten Ethern, vorzugsweise Dioxan. Im Fall c1 = 2 wird anschließend eine Oxidation nach üblichen Methoden durchgeführt. Die Umsetzungen erfolgen in einem Temperaturbereich von 0°C bis 70°C und Normaldruck.

Darüber hinaus umfasst die Erfindung die Kombination der erfindungsgemäßen Verbindungen der allgemeinen Formel (I-I) mit organischen Nitraten und NO-Donatoren.

Organische Nitrate und NO-Donatoren im Rahmen der Erfindung sind im Allgemeinen Substanzen, die über die Freisetzung von NO bzw. NO-Species ihre therapeutische Wirkung entfalten. Bevorzugt sind Natriumnitroprussid (SNP), Nitroglycerin, Isosorbiddinitrat, Isosorbidmononitrat, Molsidomin und SIN-1 und ähnliche Stoffe.

Außerdem umfasst die Erfindung die Kombination mit Verbindungen, die den Abbau von cyclischen Guanosinmonophosphat (cGMP) inhibieren. Dies sind insbesondere Inhibitoren der Phosphodiesterasen 1, 2 und 5; Nomenklatur nach Beavo und Reifsnyder (1990) TIPS 11 S. 150 - 155. Durch diese Inhibitoren wird die Wirkung der erfindungsgemäßen Verbindungen potenziert und der gewünschte pharmakologische Effekt gesteigert.

Die erfindungsgemäßen Verbindungen der allgemeinen Formel (I-I) zeigen ein nicht vorhersehbares, wertvolles pharmakologisches Wirkspektrum.

Die erfindungsgemäßen Verbindungen der allgemeinen Formel (I-I) führen zu einer Gefäßrelaxation, zu einer Thrombozytenaggregationshemmung und zu einer Blutdrucksenkung sowie zu einer Steigerung des koronaren Blutflusses. Diese Wirkungen sind über eine direkte Stimulation der löslichen Guanylatzyklase und einem intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivate.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose und Krankheiten des Urogenitalsystems wie beispielsweise Prostatahypertrophie, erektile Dysfunktion und Inkontinenz eingesetzt werden.

Zur Feststellung der kardiovaskulären Wirkungen wurden folgende Untersuchungen durchgeführt: In in vitro-Untersuchungen an Zellen vaskulären Ursprungs wurde der Einfluss auf die Guanylatzyklaseabhängige cGMP-Bildung mit und ohne NO-Donor geprüft. Die antiaggregatorischen Eigenschaften wurden an mit kollagenstimulierten menschlichen Thrombozyten gezeigt. Die gefäßrelaxierende Wirkung wurde an mit Phenylephrin vorkontrahierten Kaninchenaortenringen bestimmt. Die blutdrucksenkende Wirkung wurde an narkotisierten Ratten untersucht.

### Stimulation der löslichen Guanylatzyklase in primären Endothelzellen

Primäre Endothelzellen wurden aus Schweineaorten durch Behandlung mit Kollagenase-Lsg. isoliert. Anschließend wurden die Zellen in Kulturmedium bis zum Erreichen der Konfluenz kultiviert. Für die Untersuchungen wurden die Zellen passagiert, in Zellkulturplatten ausgesät und bis zum Erreichen der Konfluenz subkultiviert. Zur Stimulation der endothelialen Guanylatzyklase wurde das Kulturmedium abgesaugt und die Zellen einmal mit Ringerlösung gewaschen und in Stimulationspuffer mit oder ohne NO-Donor (Natrium-Nitroprussid, SNP, 1 µM) inkubiert. Im Anschluss daran wurden die Testsubstanzen (Endkonzentration 1 µM) zu den Zellen pipettiert. Nach Ende der 10-minütigen Inkubationszeit wurde die Pufferlösung abgesaugt und die Zellen 16 Stunden lang bei - 20°C lysiert. Anschließend wurde das intrazelluläre cGMP radioimmunologisch bestimmt.

### Tabelle A

| Beispiel Nr. | % cGMP-Steigerung |
|---|---|
| I-4 | > 1000 |
| I-10 | 217 |
| I-16 | > 1000 |
| I-17 | 200 |
| I-18 | > 1000 |
| I-22 | 146 |
| I-24 | 65 |

### Gefäßrelaxierende Wirkung in vitro

1,5 mm breite Ringe einer isolierten Kaninchen-Aorta werden einzeln unter einer Vorspannung in 5 ml-Organbäder mit 37°C warmer, carbogenbegaster Krebs-Henseleit-Lösung gebracht. Die Kontraktionskraft wird verstärkt und digitalisiert sowie parallel auf Linienschreiber registriert. Zur Erzeugung einer Kontraktion wird Phenylephrin dem Bad kumulativ in ansteigender Konzentration zugesetzt.

Nach mehreren Kontrollzyklen wird die zu untersuchende Substanz in jedem weiteren Durchgang in jeweils steigender Dosierung untersucht und mit der Höhe der im letzten Vordurchgang erreichten Kontraktion verglichen. Daraus wird die Konzentration errechnet, die erforderlich ist, um die Höhe des Kontrollwertes um 50 % zu reduzieren (IC₅₀). Das Standardapplikationsvolumen beträgt 5 µl.

### Tabelle 2

| Beispiel Nr. | Aorta IC₅₀ (µm) |
|---|---|
| I-4 | 8,0 |
| I-10 | 9,0 |
| I-16 | 9,1 |
| I-18 | 7,2 |
| I-19 | 15 |
| I-20 | 8,2 |
| I-21 | >27 |
| I-22 | 8,8 |
| I-23 | 2,9 |
| I-24 | 26 |

### Blutdruckmessungen an narkotisierten Ratten

Männliche Wistar-Ratten mit einem Körpergewicht von 300 - 350 g werden mit Thiopental (100 mg/kg i.p.) anästhesiert. Nach Tracheotomie wird in die Femoralarterie ein Katheter zur Blutdruckmessung eingeführt. Die zu prüfenden Substanzen werden als Suspension in Tyloselösung mittels Schlundsonde in verschiedenen Dosen oral verabreicht.

### Thrombozytenaggregationshemmung in vitro

Zur Bestimmung der thrombozytenaggregationshemmenden Wirkung wurde Blut von gesunden Probanden beiderlei Geschlechts verwendet. Als Antikoagulans wurden einem Teil 3,8 %iger wässriger Natriumzitratlösung 9 Teile Blut zugemischt. Mittels Zentrifugation erhält man aus diesem Blut plättchenreicheres Zitratplasma (PRP).

Für die Untersuchungen wurden 445 µl PRP und 5 µl der Wirkstofflösung bei 37°C im Wasserbad vorinkubiert. Anschließend wurde die Thrombozytenaggregation im Aggregometer bei 37°C bestimmt. Hierzu wurde die vorinkubierte Probe mit 50 µl Kollagen, einem aggregationsauslösenden Agens, versetzt, und die Veränderung der optischen Dichte erfasst. Zur quantitativen Ausweitung wurde der maximale Aggregationsresponse ermittelt und daraus die prozentuale Hemmung gegenüber der Kontrolle errechnet.

Die in der Ausführungsform I vorliegenden Erfindung beschriebenen Verbindungen stellen auch Wirkstoffe zur Bekämpfung von Krankheiten im Zentralnervensystem dar, die durch Störungen des NO/cGMP-Systems gekennzeichnet sind. Insbesondere sind sie geeignet zur Beseitigung kognitiver Defizite, zur Verbesserung von Lern- und Gedächtnisleistungen und zur Behandlung der Alzheimer'schen Krankheit. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuß- und Suchtmittelaufnahme.

Weiterhin eignen sich diese Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im Allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht.

### II

Die vorliegende Erfindung betrifft in der mit II (römisch zwei) bezeichneten Ausführungsform neue 1-Heterocyclyl-methyl-substituierte Pyrazole der allgemeinen Formel (II-I), 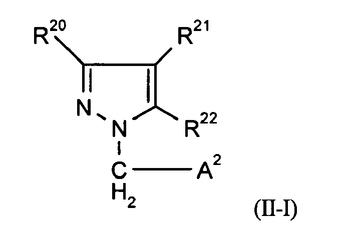 in welcher
- R²⁰: für einen 6-gliedrigen aromatischen Heterocyclus mit bis zu 3 Stickstoffatomen steht, der gegebenenfalls bis zu 3-fach gleich oder verschieden durch Formyl, Carboxyl, Hydroxy, Mercaptyl, geradkettiges oder verzweigtes Acyl, Alkoxy, Alkylthio oder Alkoxycarbonyl mit jeweils bis zu 6 Kohlenstoffatomen, Nitro, Cyano, Azido, Halogen, Phenyl und/oder durch eine Gruppe der Formel

-NR²³R²⁴

substituiert ist,
worin
- R²³ und R²⁴: gleich oder verschieden sind und Wasserstoff oder geradkettiges oder verzweigtes Acyl mit bis zu 6 Kohlenstoffatomen oder geradkettiges oder verzweigtes Alkyl mit bis zu 6 Kohlenstoffatomen bedeuten, das gegebenenfalls durch Cycloalkyl mit 3 bis 6 Kohlen-stoffatomen, Hydroxy, Amino oder durch geradkettiges oder verzweigtes Alkoxy, Acyl oder Alkoxycarbonyl mit jeweils bis zu 5 Kohlenstoffatomen substituiert ist,
oder
- R²³ und R²⁴: gemeinsam mit dem Stickstoffatom einen 3- bis 7-gliedrigen gesättigten oder partiell ungesättigten Heterocyclus bilden, der gegebenenfalls zusätzlich ein Sauerstoff- oder Schwefelatom oder einen Rest der Formel -NR²⁵ enthalten kann,
worin
- R²⁵: Wasserstoff oder geradkettiges oder verzweigtes Alkyl mit bis zu 4 Kohlenstoffatomen bedeutet,
und/oder durch geradkettiges oder verzweigtes Alkyl mit bis zu 6 Kohlenstoffatomen substituiert ist, das seinerseits durch Hydroxy, Amino, Halogen, Carboxyl, geradkettiges oder verzweigtes Acyl, Alkoxy, Alkoxycarbonyl oder Acylamino mit jeweils bis zu 5 Kohlenstoffatomen oder durch einen Rest der Formel -OR²⁶ substituiert sein kann,
worin
- R²⁶: geradkettiges oder verzweigtes Acyl mit bis zu 5 Kohlenstoffatomen oder eine Gruppe der Formel -SiR²⁷R²⁸R²⁹ bedeutet,
worin
- R²⁷, R²⁸ und R²⁹: gleich oder verschieden sind und Aryl mit 6 bis 10 Kohlenstoffatomen oder Alkyl mit bis zu 6 Kohlenstoffatomen bedeuten,
und/oder gegebenenfalls durch einen Rest der Formel 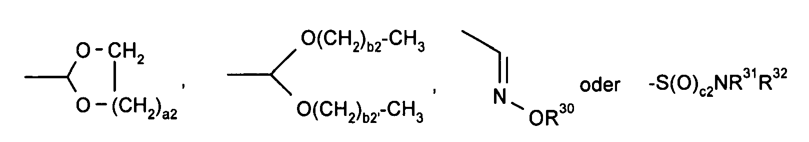 substituiert ist,
worin
- b2 und b2': gleich oder verschieden sind und eine Zahl 0, 1, 2 oder 3 bedeuten,
- a2: eine Zahl 1, 2 oder 3 bedeutet,
- R³⁰: Wasserstoff oder geradkettiges oder verzweigtes Alkyl mit bis zu 4 Kohlenstoff-atomen bedeutet,
- c2: eine Zahl 1 oder 2 bedeutet und
- R³¹ und R³²: gleich oder verschieden sind und Wasserstoff oder geradkettiges oder verzweigtes Alkyl mit bis zu 10 Kohlenstoffatomen bedeutet, das gegebenenfalls durch Cycloalkyl mit 3 bis 8 Kohlenstoffatomen oder durch Aryl mit 6 bis 10
Kohlenstoffatomen substituiert ist, das seinerseits durch Halogen substituiert sein kann oder
Aryl mit 6 bis 10 Kohlenstoffatomen bedeuten, das gegebenenfalls durch Halogen substituiert ist oder
Cycloalkyl mit 3 bis 7 Kohlenstoffatomen bedeuten oder
R³¹ und R³² gemeinsam mit dem Stickstoffatom einen 5- bis 7-gliedrigen gesättigten Heterocyclus bilden, der gegebenenfalls ein weiteres Sauerstoffatom oder einen Rest -NR³³ enthalten kann,
worin
- R³³: Wasserstoff, geradkettiges oder verzweigtes Alkyl mit zu 4 Kohlenstoff-atomen oder einen Rest der Formel 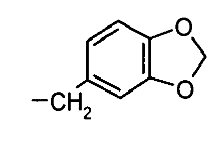
bedeutet,
oder Benzyl oder Phenyl bedeutet, wobei die Ringsysteme gegebenenfalls durch Halogen substituiert sind,
- R²¹ und R²²: unter Einbezug der Doppelbindung einen 5-gliedrigen aromatischen Heterocyclus mit einem Heteroatom aus der Reihe S, N und/oder O oder einen Phenylring bilden, die gegebenenfalls bis zu 3-fach gleich oder verschieden durch Formyl, Mercaptyl, Carboxyl, Hydroxy, Amino, geradkettiges oder verzweigtes Acyl, Alkylthio, Alkoxy oder Alkoxy-carbonyl mit jeweils bis zu 6 Kohlenstoffatomen, Nitro, Cyano, Azido, Halogen, Phenyl oder geradkettiges oder verzweigtes Alkyl mit bis zu 6 Kohlenstoffatomen substituiert sind, das seinerseits durch Hydroxy, Amino, Carboxyl, geradkettiges oder verzweigtes Acyl, Alkoxy oder Alkoxycarbonyl mit jeweils bis zu 5 Kohlenstoffatomen substituiert sein kann, oder gegebenenfalls durch eine Gruppe der Formel -S(O)_{c2'}NR^{31'}R^{32'} substituiert sind, worin c_{2'}, R^{31'} und R^{32'} die oben angegebene Bedeutung von c2, R³¹ und R³² haben und mit dieser gleich oder verschieden sind,
- A²: für Phenyl oder einen 5- bis 6-gliedrigen aromatischen oder gesättigten Heterocyclus mit bis zu 3 Heteroatomen aus der Reihe S, N und/oder O steht, der gegebenenfalls bis zu 3-fach gleich oder verschieden durch Mercaptyl, Hydroxy, Formyl, Carboxyl, geradkettiges oder
verzweigtes Acyl, Alkylthio, Alkyloxyacyl, Alkoxy oder Alkoxycarbonyl mit jeweils bis zu 6 Kohlenstoffatomen, Nitro, Cyano, Trifluormethyl, Azido, Halogen, Phenyl oder gerad-kettiges oder verzweigtes Alkyl mit bis zu 6 Kohlenstoffatomen substituiert ist, das seiner-seits durch Hydroxy, Carboxyl, geradkettiges oder verzweigtes Acyl, Alkoxy oder Alkoxy-carbonyl mit jeweils bis zu 5 Kohlenstoffatomen substituiert sein kann, und/oder durch eine Gruppe der Formel -(CO)_{d2}-NR³⁴R³⁵ substituiert ist,
worin
- d2: eine Zahl 0 oder 1 bedeutet,
- R³⁴ und R³⁵: gleich oder verschieden sind und Wasserstoff, Phenyl, Benzyl oder geradkettiges oder verzweigtes Alkyl oder Acyl mit jeweils bis zu 5 Kohlenstoffatomen bedeuten,
deren isomere Formen und Salze und deren N-Oxide.

Im Rahmen der Ausführungsform II der vorliegenden Erfindung werden physiologisch unbedenkliche Salze mit organischen oder anorganischen Basen oder Säuren bevorzugt. Physiologisch unbedenkliche Salze der 1-Heterocyclyl-methyl-substituierten Pyrazole können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall-oder Ammoniumsalze der erfindungsgemäßen Verbindungen sein, welche eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Die erfindungsgemäßen Verbindungen gemäß Ausführungsform II können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen.

Heterocyclus steht im Rahmen der Erfindung gemäß der Ausführungsform II im Fall R²⁰ für einen 6-gliedrigen aromatischen Heterocyclus im Fall R²¹/R²² für einen 5-gliedrigen aromatischen Heterocyclus mit 1 Heteroatom und im Fall A² für einen 5- bis 6-gliedrigen, aromatischen oder gesättigten Heterocyclus und im Fall der Gruppe NR²³R²⁴ für einen gesättigten oder partiell ungesättigten 3- bis 7-gliedrigen Heterocyclus. Beispielsweise seien genannt: Pyridazinyl, Chinolyl, Isochinolyl, Pyrazinyl, Pyridyl, Pyrimidyl, Thienyl, Furyl, Morpholinyl, Pyrrolyl, Thiazolyl, Oxazolyl, Imidazolyl, Tetrahydropyranyl oder Tetrahydrofuranyl.

Bevorzugt sind erfindungsgemäße Verbindungen der allgemeinen Formel (II-I),
in welcher
- R²⁰: für einen Rest der Formel 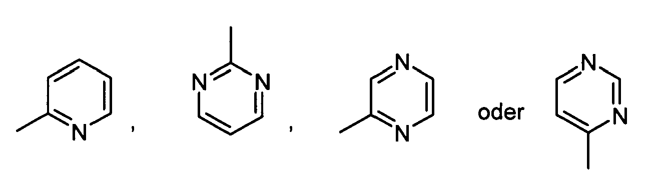
steht,
die gegebenenfalls bis zu 3-fach gleich oder verschieden durch Formyl, Carboxyl, Hydroxy, geradkettiges oder verzweigtes Acyl, Alkoxy oder Alkoxycarbonyl mit jeweils bis zu 5 Kohlenstoffatomen, Nitro, Cyano, Azido, Fluor, Chlor, Brom, Phenyl und/oder durch eine Gruppe der Formel -NR²³ R²⁴ substituiert sind,
worin
- R²³ und R²⁴: gleich oder verschieden sind und Wasserstoff oder geradkettiges oder verzweigtes Acyl mit bis zu 4 Kohlenstoffatomen oder geradkettiges oder verzweigtes Alkyl mit bis zu 4 Kohlenstoffatomen bedeuten, das gegebenenfalls durch Hydroxy, Amino oder durch geradkettiges oder verzweigtes Alkoxy mit bis zu 3 Kohlenstoffatomen substituiert ist, oder
- R²³ und R²⁴: gemeinsam mit dem Stickstoffatom einen Morpholinring oder einen Rest der Formel 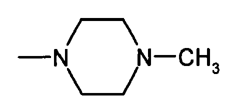
bilden
und/oder durch geradkettiges oder verzweigtes Alkyl mit bis zu 5 Kohlenstoffatomen substituiert sind, das seinerseits durch Hydroxy, Amino, Fluor, Carboxyl, geradkettiges oder verzweigtes Acyl, Alkoxy, Alkoxycarbonyl oder Acylamino mit jeweils bis zu 4 Kohlenstoffatomen oder durch einen Rest der Formel -OR²⁶ substituiert sein kann,
worin
- R²⁶: geradkettiges oder verzweigtes Acyl mit bis zu 4 Kohlenstoffatomen bedeutet,
und/oder gegebenenfalls durch einen Rest der Formel 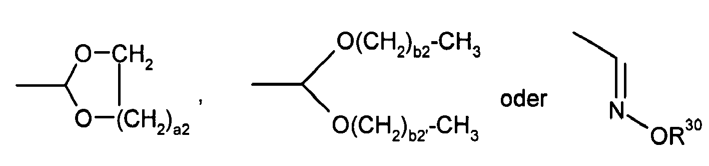
substituert sind,
worin
- b2 und b2': gleich oder verschieden sind und eine Zahl 0, 1, 2 oder 3 bedeuten,
- a2: eine Zahl 1, 2 oder 3 bedeutet,
- R³⁰: Wasserstoff oder geradkettiges oder verzweigtes Alkyl mit bis zu 4 Kohlenstoff-atomen bedeutet,
- R²¹ und R²²: unter Einbezug der Doppelbindung einen Furyl-, Thienyl- oder Phenylring bilden, die gegebenenfalls bis zu 3-fach gleich oder verschieden durch Formyl, Carboxyl, Hydroxy, Amino, geradkettiges oder verzweigtes Acyl, Alkoxy oder Alkoxycarbonyl mit jeweils bis zu 5 Kohlenstoffatomen, Nitro, Cyano, Azido, Fluor, Chlor, Brom, Phenyl oder geradkettiges oder verzweigtes Alkyl mit bis zu 5 Kohlenstoffatomen substituiert sind, das seinerseits durch Hydroxy, Amino, Carboxyl, geradkettiges oder verzweigtes Acyl, Alkoxy oder Alkoxycarbonyl mit jeweils bis zu 4 Kohlenstoffatomen substituiert sein kann,
- A²: für Phenyl oder für Tetrahydropyranyl, Furyl, Tetrahydrofuranyl, Morpholinyl, Pyrimidyl, Pyridazinyl oder Pyridyl steht, die gegebenenfalls bis zu 2-fach gleich oder verschieden durch Hydroxy, Formyl, Carboxyl, geradkettiges oder verzweigtes Acyl, Alkylthio, Alkyl-oxyacyl, Alkoxy oder Alkoxycarbonyl mit jeweils bis zu 4 Kohlenstoffatomen, Fluor, Chlor, Brom, Nitro, Cyano, Trifluormethyl oder geradkettiges oder verzweigtes Alkyl mit bis zu 4 Kohlenstoffatomen substituiert sind, das seinerseits durch Hydroxy, Carboxyl, geradkettiges oder verzweigtes Acyl, Alkoxy oder Alkoxycarbonyl mit jeweils bis zu 4 Kohlenstoffatomen substituiert sein kann,
und/oder durch eine Gruppe der Formel -(CO)_{d2}-NR³⁴R³⁵ substituiert sind,
worin

- d2: eine Zahl 0 oder 1 bedeutet,
- R³⁴ und R³⁵: gleich oder verschieden sind und Wasserstoff, Phenyl, Benzyl oder geradkettiges oder verzweigtes Alkyl oder Acyl mit jeweils bis zu 4 Kohlenstoffatomen bedeuten,
deren isomere Formen und Salze und deren N-Oxide.

Besonders bevorzugt sind erfindungsgemäße Verbindungen der allgemeinen Formel (II-I),
in welcher
- R²⁰: für einen Rest der Formel 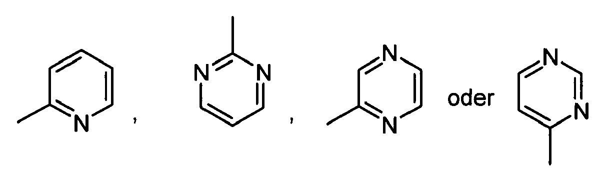
steht,
wobei die Ringsysteme, gegebenenfalls bis zu 3-fach gleich oder verschieden durch Formyl, geradkettiges oder verzweigtes Acyl, Alkoxy oder Alkoxycarbonyl mit jeweils bis zu 4 Kohlenstoffatomen, Methylamino, Amino, Fluor, Chlor, Brom, Cyano, Azido oder gerad-kettiges oder verzweigtes Alkyl mit bis zu 4 Kohlenstoffatomen substituiert sind, das seinerseits durch Hydroxy, Carboxyl, Amino, geradkettiges oder verzweigtes Acyl, Alkoxy, Alkoxycarbonyl oder Acylamino mit jeweils bis zu 3 Kohlenstoffatomen substituiert sein kann,
und/oder gegebenenfalls durch einen Rest der Formel 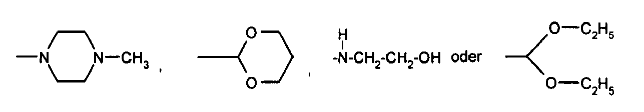 substituiert sind
- R²¹ und R²²: unter Einbezug der Doppelbindung einen Furyl-, Thienyl- oder Phenylring bilden, die gegebenenfalls bis zu 2-fach gleich oder verschieden durch Formyl, Carboxyl, Hydroxy, Amino, geradkettiges oder verzweigtes Acyl, Alkoxy oder Alkoxycarbonyl mit jeweils bis zu 4 Kohlenstoffatomen, Nitro, Cyano, Fluor, Chlor, Phenyl oder geradkettiges oder verzweigtes Alkyl mit bis zu 3 Kohlenstoffatomen substituiert sind, das seinerseits durch Hydroxy, Amino, Carboxyl, geradkettiges oder verzweigtes Acyl, Alkoxy oder Alkoxycarbonyl mit jeweils bis zu 3 Kohlenstoffatomen substituiert sein kann,

- A²: für Phenyl, Tetrahydropyranyl, Tetrahydrofuranyl, Furyl oder Pyridyl steht, die gegebenen-falls bis zu 2-fach gleich oder verschieden durch Formyl, Carboxyl, geradkettiges oder verzweigtes Acyl, Alkylthio, Alkyloxyacyl, Alkoxy oder Alkoxycarbonyl mit jeweils bis zu 3 Kohlenstoffatomen, Fluor, Chlor, Brom, Nitro, Cyano, Trifluormethyl, oder für geradkettiges oder verzweigtes Alkyl mit bis zu 3 Kohlenstoffatomen substituiert sind, das seinerseits durch Hydroxy, Carboxyl, geradkettiges oder verzweigtes Acyl, Alkoxy oder Alkoxy-carbonyl mit jeweils bis zu 3 Kohlenstoffatomen substituiert sein kann,
und/oder durch eine Gruppe der Formel -(CO)_{d2}-NR³⁴R³⁵ substituiert sind,
worin
- d2: eine Zahl 0 oder 1 bedeutet,
- R³⁴ und R³⁵: gleich oder verschieden sind und Wasserstoff oder geradkettiges oder verzweigtes Alkyl oder Acyl mit jeweils bis zu 3 Kohlenstoffatomen bedeuten,
deren isomere Formen, Salze und N-Oxide.

Ganz besonders bevorzugt sind erfindungsgemäße Verbindungen der allgemeinen Formel (II-I), in welcher
- R²⁰: für einen Rest der Formel 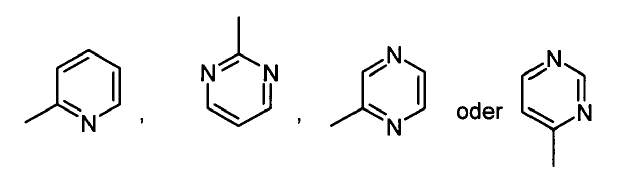
steht,
wobei die oben aufgeführten heterocyclischen Ringsysteme, gegebenenfalls bis zu 3-fach gleich oder verschieden durch Methyl, Fluor, Formyl, Amino, Cyano, Methoxy, Methoxy-carbonyl, Methylamino, Chlor oder durch einen Rest der Formel 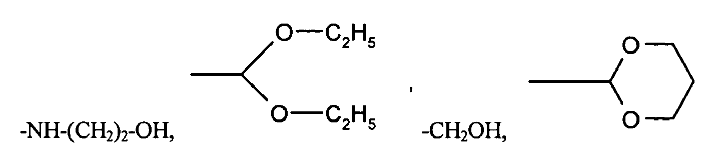 oder 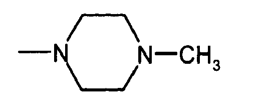 substituiert sind,
- R²¹ und R²²: unter Einbezug der Doppelbindung gemeinsam einen Phenylring bilden und
- A²: für Phenyl steht, das gegebenenfalls durch Fluor oder Cyano substituiert ist
und deren isomere Formen, Salze und N-Oxide.

Die Erfindung betrifft weiterhin Verfahren zur Herstellung von Verbindungen der allgemeinen Formel (II-I), dadurch gekennzeichnet, dass man

### [A2] Verbindungen der allgemeinen Formel (II-II)

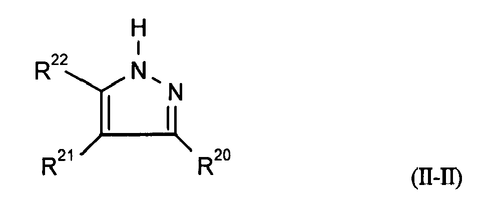

in welcher
R²⁰, R²¹ und R²² die oben angegebene Bedeutung haben,
mit Verbindungen der allgemeinen Formel (II-III)

D²-CH₂A² (II-III)

in welcher
- A²: die oben angegebene Bedeutung hat,
und
- D²: für Triflat oder Halogen, vorzugsweise für Brom steht,
in inerten Lösemitteln, gegebenenfalls in Anwesenheit einer Base umsetzt,
oder

### [B2] Verbindungen der allgemeinen Formel (II-IV)

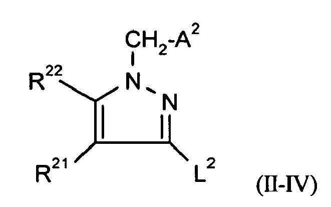

in welcher
A², R²¹ und R²² die oben angegebene Bedeutung haben,
und
- L²: für einen Rest der Formel -SnR³⁶R³⁷R³⁸, ZnR³⁹, Iod oder Triflat steht,
worin
- R³⁶, R³⁷ und R³⁸: gleich oder verschieden sind und geradkettiges oder verzweigtes Alkyl mit bis zu 4 Kohlenstoffatomen bedeuten,
und
- R³⁹: Halogen bedeutet,
mit Verbindungen der allgemeinen Formel (II-V)

R²⁰-T² (II-V)

in welcher
- R²⁰: die oben angegebene Bedeutung hat,
und
im Fall L² = SnR³⁶R³⁷R³⁸ oder ZnR³⁹
- T²: für Triflat oder für Halogen, vorzugsweise für Brom steht,
und
im Fall L²= Jod oder Triflat
- T²: für einen Rest der Formel SnR^{36'}R^{37'}R^{38'}, ZnR^{39'} oder BR⁴⁰R⁴¹steht,
worin
- R^{36'}, R^{37'}, R^{38'} und R^{39'}: die oben angegebene Bedeutung von R³⁶, R³⁷, R³⁸ und R³⁹ haben und mit dieser gleich oder verschieden sind,
- R⁴⁰ und R⁴¹: gleich oder verschieden sind und Hydroxy, Aryloxy mit 6 bis 10 Kohlen-stoffatomen oder geradkettiges oder verzweigtes Alkyl oder Alkoxy mit jeweils bis zu 5 Kohlenstoffatomen bedeuten, oder gemeinsam einen 5- oder 6-gliedrigen carbocyclischen Ring bilden,
in einer palladiumkatalysierten Reaktion in inerten Lösemitteln umsetzt,
und im Fall der Reste -S(O)_{c2}NR³¹R³² und -S(O)_{c2'}NR^{31'}R^{32'} ausgehend von den unsubstituierten Verbindungen der allgemeinen Formel (II-I) zunächst mit Thionylchlorid umsetzt und abschließend die Aminkomponente einsetzt,
und gegebenenfalls die unter R²⁰, R²¹, R²² und/oder A² aufgeführten Substituenten nach üblichen Methoden, vorzugsweise durch Reduktion, Oxidation, Abspaltung von Schutzgruppen und/oder nucleophiler Substitution variiert oder einführt.

Die erfindungsgemäßen Verfahren zur Herstellung der Verbindungen gemäß Ausführungsform II können durch folgendes Formelschema beispielhaft erläutert werden:

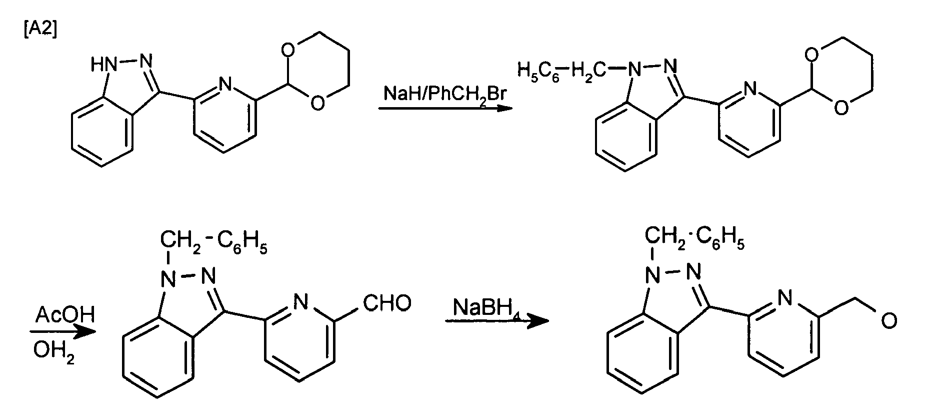

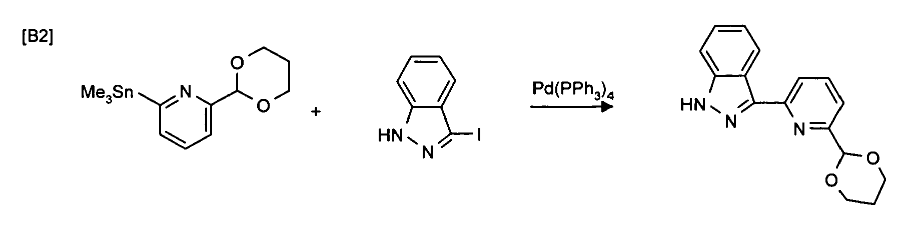

Als Lösemittel für die einzelnen Schritte des Verfahrens [A2] eignen sich hierbei inerte organische Lösemittel, die sich unter den Reaktionsbedingungen nicht verändern. Hierzu gehören Ether, wie Diethylether oder Tetrahydrofuran, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Tetrahydrofuran, Toluol oder Dimethylformamid.

Als Basen für das erfindungsgemäße Verfahren gemäß Ausführungsform II können im Allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo[5.4.0]undec-7-en (DBU), Pyridin, Diaminopyridin, Methylpiperidin oder Morpholin. Es ist auch möglich als Basen Alkalimetalle wie Natrium und deren Hydride wie Natriumhydrid einzusetzen. Bevorzugt sind Natrium- und Kaliumcarbonat, Triethylamin und Natriumhydrid.

Die Base wird in einer Menge von 1 mol bis 5 mol, bevorzugt von 1 mol bis 3 mol, bezogen auf 1 mol der Verbindung der allgemeinen Formel (II-II) eingesetzt.

Die Umsetzung wird im Allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im Allgemeinen arbeitet man bei Normaldruck.

Als Lösemittel für das Verfahren [B2] eignen sich hierbei inerte organische Lösemittel, die sich unter den Reaktionsbedingungen nicht verändern. Hierzu gehören Ether, wie Diethylether oder Tetrahydrofuran, DME, Dioxan, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Tetrahydrofuran, Dimethylformamid, Toluol, Dioxan oder Dimethoxyethan.

Die Reaktion wird im Allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im Allgemeinen arbeitet man bei Normaldruck.

Als Palladiumverbindungen im Rahmen der vorliegenden Erfindung eignen sich im Allgemeinen PdCl₂((C₆H₅)₃)₂, Palladium-bis-dibenzylidenaceton (Pd(dba)₂), [1,1'-Bis-(diphenylphosphino)ferrocen]-Palladium(II)-chlorid (Pd(dppf)Cl₂) oder Pd(P(C₆H₅)₃)₄. Bevorzugt ist Pd(P(C₆H₅)₃)₄.

Die Verbindungen der allgemeinen Formeln (II-III) und (II-V) sind bekannt oder nach üblichen Methoden herstellbar.

Die Verbindungen der allgemeinen Formel (II-II) sind teilweise bekannt und können hergestellt werden, indem man Verbindungen der allgemeinen Formel (VI) 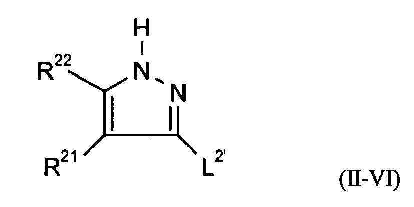 in welcher
- R²¹ und R²²: die oben angegebene Bedeutung haben
und
- L^{2'}: die oben angegebene Bedeutung von L² hat und mit dieser gleich oder verschieden ist,
mit Verbindungen der allgemeinen Formel (II-V) in Analogie zu dem oben aufgeführten Verfahren [B2] umsetzt.

Die Verbindungen der allgemeinen Formel (II-IV) sind teilweise bekannt oder im Fall der Stannyle neu und können dann beispielsweise hergestellt werden, indem man die Verbindungen der allgemeinen Formel (II-IVa) 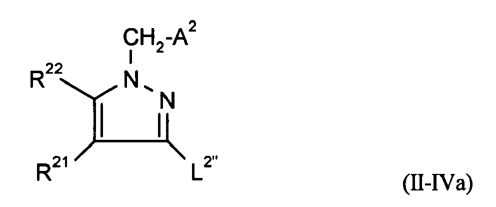 in welcher
R²¹ R²² und A² die oben angegebene Bedeutung haben,
- L^{2"}: für Triflat oder Halogen, vorzugsweise für Iod steht,
mit Verbindungen der allgemeinen Formel (II-VII)

(SnR³⁶R³⁷R³⁸)₂ (II-VII)

in welcher
R³⁶, R³⁷, R³⁸ die oben angegebene Bedeutung haben,
wie oben beschrieben palladiumkatalysiert umsetzt.

Die Verbindungen der allgemeinen Formel (II-VII) sind bekannt oder nach üblichen Methoden herstellbar.

Die Verbindungen der allgemeinen Formel (II-IVa) sind größtenteils neu und können hergestellt werden, indem man Verbindungen der allgemeinen Formel (II-VIII) 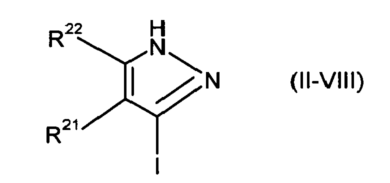 in welcher
R²¹ und R²² die oben angegebene Bedeutung haben mit den oben aufgeführten Verbindungen der allgemeinen Formel (II-V)

R²⁰-T² (II-V)

worin R²⁰ und T² die oben angegebene Bedeutung besitzen, in einem der oben aufgeführten Lösemittel, vorzugsweise Tetrahydrofuran, und in Anwesenheit von Natriumhydrid in einem Temperaturbereich von 0°C bis 40°C, vorzugsweise bei Raumtemperatur und unter Schutzgasatmosphäre umsetzt.

Die Verbindungen der allgemeinen Formel (II-VIII) sind größtenteils bekannt oder nach üblichen Methoden herstellbar.

Die Reduktionen werden im Allgemeinen mit Reduktionsmitteln, bevorzugt mit solchen, die für die Reduktion von Carbonyl zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat, Diisobutylaluminiumhydrid oder Lithiumaluminiumhydrid durchgeführt. Ganz besonders bevorzugt wird die Reduktion mit Diisobutylaluminiumhydrid und Natriumborhydrid durchgeführt.

Das Reduktionsmittel wird im Allgemeinen in einer Menge von 1 mol bis 6 mol, bevorzugt von 1 mol bis 4 mol bezogen auf 1 mol der zu reduzierenden Verbindungen, eingesetzt.

Die Reduktion verläuft im Allgemeinen in einem Temperaturbereich von -78°C bis +50°C, bevorzugt von -78°C bis 0°C, im Falle des DIBAH, 0°C, Raumtemperatur im Falle des NaBH₄, besonders bevorzugt bei -78°C, jeweils in Abhängigkeit von der Wahl des Reduktionsmittels sowie Lösemittel.

Die Reduktion verläuft im Allgemeinen bei Normaldruck, es ist aber auch möglich bei erhöhtem oder erniedrigtem Druck zu arbeiten.

Im Fall der Reste der Formel -S(O)_{c2}NR³¹R³² und -S(O)_{c2'}NR^{31'}R^{32'} substituiert ist, werden die entsprechenden unsubstituierten Verbindungen zunächst mit Thionylchlorid umgesetzt und in einem zweiten Schritt mit den Aminen in Anwesenheit von den oben aufgeführten Ethern, vorzugsweise Dioxan, versetzt und im Fall c2 = 2 eine Oxidation nach üblichen Methoden angeschlossen. Die Umsetzungen erfolgen im Allgemeinen in einem Temperaturbereich von 0°C bis 70°C und Normaldruck.

Die Abspaltung der Schutzgruppe erfolgt im Allgemeinen in einem der oben aufgeführten Alkohole und/oder THF oder Aceton, vorzugsweise Methanol / THF in Anwesenheit von Salzsäure oder Trifluoressigsäure oder Toluolsulfonsäure in einem Temperaturbereich von 0°C bis 70°C, vorzugsweise bei Raumtemperatur und Normaldruck.

Darüber hinaus umfasst die Erfindung die Kombination der erfindungsgemäßen Verbindungen der allgemeinen Formel (II-I) mit organischen Nitraten und NO-Donatoren.

Organische Nitrate und NO-Donatoren im Rahmen der Erfindung sind im Allgemeinen Substanzen, die über die Freisetzung von NO bzw. NO-Species ihre therapeutische Wirkung entfalten. Bevorzugt sind Natriumnitroperussid (SNP), Nitroglycerin, Isosorbiddinitrat, Isosorbidmononitrat, Molsidomin und SIN-1.

Außerdem umfasst die Erfindung die Kombination mit Verbindungen, die dem Abbau von cyclischen Guanosinmonophosphat (cGMP) inhibieren. Dies sind insbesondere Inhibitoren der Phosphodiesterasen 1, 2 und 5; Nomenklatur nach Beavo und Reifsnyder (1990) TIPS 11 S. 150 - 155. Durch diese Inhibitoren wird die Wirkung der erfindungsgemäßen Verbindungen potenziert und der gewünschte pharmakologische Effekt gesteigert.

Die erfindungsgemäßen Verbindungen der allgemeinen Formel (II-I) zeigen ein nicht vorhersehbares, wertvolles pharmakologisches Wirkspektrum.

Die erfindungsgemäßen Verbindungen der allgemeinen Formel (II-I) führen zu einer Gefäßrelaxation/Thrombozytenaggregationshemmung und zu einer Blutdrucksenkung sowie zu einer Steigerung des koronaren Blutflusses. Diese Wirkungen sind über eine direkte Stimulation der löslichen Guanylatzyklase und einem intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivate.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose und Krankheiten des Urogenitalsystems wie beispielsweise Prostatahypertrophie, erektile Dysfunktion und Inkontinenz eingesetzt werden.

Zur Feststellung der kardiovaskulären Wirkungen wurden folgende Untersuchungen durchgeführt: In in vitro-Untersuchungen an Zellen vaskulären Ursprungs wurde der Einfluss auf die Guanylatzyklaseabhängige cGMP-Bildung mit und ohne NO-Donor geprüft. Die antiaggregatorischen Eigenschaften wurden an mit kollagenstimulierten menschlichen Thrombozyten gezeigt. Die gefäßrelaxierende Wirkung wurde an mit Phenylephrin vorkontrahierten Kaninchenaortenringen bestimmt. Die blutdrucksenkende Wirkung wurde an narkotisierten Ratten untersucht.

### Stimulation der löslichen Guanylatzyklase in primären Endothelzellen

Primäre Endothelzellen wurden aus Schweineaorten durch Behandlung mit Kollagenase-Lsg. isoliert. Anschließend wurden die Zellen in Kulturmedium bis zum Erreichen der Konfluenz kultiviert. Für die Untersuchungen wurden die Zellen passagiert, in Zellkulturplatten ausgesät und bis zum Erreichen der Konfluenz subkultiviert. Zur Stimulation der endothelialen Guanylatzyklase wurde das Kulturmedium abgesaugt und die Zellen einmal mit Ringerlösung gewaschen und in Stimulationspuffer mit oder ohne NO-Donor (Natrium-Nitroprussid, SNP, 1 µM) inkubiert. Im Anschluss daran wurden die Testsubstanzen (Endkonzentration 1 µM) zu den Zellen pipettiert. Nach Ende der 10-minütigen Inkubationszeit wurde die Pufferlösung abgesaugt und die Zellen 16 Stunden lang bei -20°C lysiert. Anschließend wurde das intrazelluläre cGMP radioimmunologisch bestimmt.

### Tabelle A

| Beispiel-Nr. | cGMP-Steigerung % |
|---|---|
| II-36 | 225 |
| II-38 | > 1 000 |
| II-39 | 909 |
| II-40 | > 1 000 |
| II-41 | 557 |
| II-42 | 611 |
| II-43 | > 1 000 |
| II-44 | > 1 000 |
| II-45 | 326 |
| II-46 | 390 |
| II-47 | 240 |
| II-48 | > 1 000 |
| II-49 | > 1 000 |
| II-50 | 116 |
| II-52 | 397 |
| II-53 | 428 |
| II-56 | 233 |
| II-58 | 271 |
| II-59 | 268 |

### Gefäßrelaxierende Wirkung in vitro

1,5 mm breite Ringe einer isolierten Kaninchen-Aorta werden einzeln unter einer Vorspannung in 5 ml-Organbäder mit 37°C warmer, carbogenbegaster Krebs-Henseleit-Lösung gebracht. Die Kontraktionskraft wird verstärkt und digitalisiert sowie parallel auf Linienschreiber registriert. Zur Erzeugung einer Kontraktion wird Phenylephrin dem Bad kumulativ in ansteigender Konzentration zugesetzt.

Nach mehreren Kontrollzyklen wird die zu untersuchende Substanz in jedem weiteren Durchgang in jeweils steigender Dosierung untersucht und mit der Höhe der im letzten Vordurchgang erreichten Kontraktion verglichen. Daraus wird die Konzentration errechnet, die erforderlich ist, um die Höhe des Kontrollwertes um 50 % zu reduzieren (IC₅₀). Das Standardapplikationsvolumen beträgt 5 µl.

### Tabelle B

| Beispiel-Nr. | AORTA IC₅₀ (µM) |
|---|---|
| II-36 | 13 |
| II-39 | 11 |
| II-40 | 2,4 |
| II-41 | 13 |
| II-42 | 10 |
| II-38 | 11 |
| II-48 | 13 |
| II-49 | 65 |
| II-50 | >>31 |
| II-51 | >>30 |
| II-52 | 14 |
| II-53 | 18 |
| II-55 | >35 |
| II-56 | >33 |
| II-59 | >33 |
| II-60 | >30 |
| II-61 | >30 |
| II-62 | 13 |

### Blutdruckmessungen an narkotisierten Ratten

Männliche Wistar-Ratten mit einem Körpergewicht von 300 - 350 g werden mit Thiopental (100 mg/kg i.p.) anästhesiert. Nach Tracheotomie wird in die Femoralarterie ein Katheter zur Blutdruckmessung eingeführt. Die zu prüfenden Substanzen werden als Suspension in Tyloselösung mittels Schlundsonde in verschiedenen Dosen oral verabreicht.

### Thrombozytenaggregationshemmung in vitro

Zur Bestimmung der thrombozytenaggregationshemmenden Wirkung wurde Blut von gesunden Probanden beiderlei Geschlechts verwendet. Als Antikoagulans wurden einem Teil 3,8 %iger wässriger Natriumzitratlösung 9 Teile Blut zugemischt. Mittels Zentrifugation erhält man aus diesem Blut plättchenreicheres Zitratplasma (PRP).

Für diese Untersuchungen wurden 445 µl PRP und 5 µl der Wirkstofflösung bei 37°C im Wasserbad vorinkubiert. Anschließend wurde die Thrombozytenaggregation nach der turbidometrischen Methode im Aggregometer bei 37°C bestimmt. Hierzu wurde die vorinkubierte Probe mit 50 µl Kollagen, einem aggregationsauslösenden Agens, versetzt, und die Veränderung der optischen Dichte erfaßt. Zur quantitativen Ausweitung wurde der maximale Aggregationsresponse ermittelt und daraus die prozentuale Hemmung gegenüber der Kontrolle errechnet.

Die in der vorliegenden Erfindung in der Ausführungsform II beschriebenen Verbindungen stellen auch Wirkstoffe zur Bekämpfung von Krankheiten im Zentralnervensystem dar, die durch Störungen der NO/cGMP-Systems gekennzeichnet sind. Insbesondere sind sie geeignet zur Beseitigung kognitiver Defizite, zur Verbesserung von Lern- und Gedächtnisleistungen und zur Behandlung der Alzheimer'schen Krankheit. Sie eignen sich auch zur Behandlung von Erkrankungen des Zentralnervensystems wie Angst-, Spannungs- und Depressionszuständen, zentralnervös bedingten Sexualdysfunktionen und Schlafstörungen, sowie zur Regulierung krankhafter Störungen der Nahrungs-, Genuß- und Suchtmittelaufnahme.

Weiterhin eignen sich diese Wirkstoffe auch zur Regulation der cerebralen Durchblutung und stellen somit wirkungsvolle Mittel zur Bekämpfung von Migräne dar.

Auch eignen sie sich zur Prophylaxe und Bekämpfung der Folgen cerebraler Infarktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im Allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

### Abkürzungen:

- Me =: Methyl
- OMe =: Methoxy
- Et =: Ethyl
- OEt =: Ethoxy
- Ph =: Phenyl

### IV

Die vorliegende Erfindung betrifft gemäß Ausführungsform IV 1-Benzyl-3-(substituierte heteroaryl)-kondensierte Pyrazol-Derivate der allgemeinen Formel (IV-I), 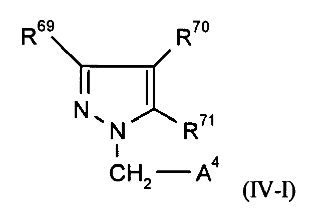 in welcher
- A⁴: für Phenyl steht, das gegebenenfalls bis zu 3-fach gleich oder verschieden durch Halogen, Hydroxy, Cyano, Carboxyl, Nitro, Trifluormethyl, Trifluormethoxy, Azido, geradkettiges oder verzweigtes Alkyl, Alkoxy oder Alkoxycarbonyl mit jeweils bis zu 6 Kohlen-stoffatomen substituiert ist,
- R⁶⁹: für einen Rest der Formel 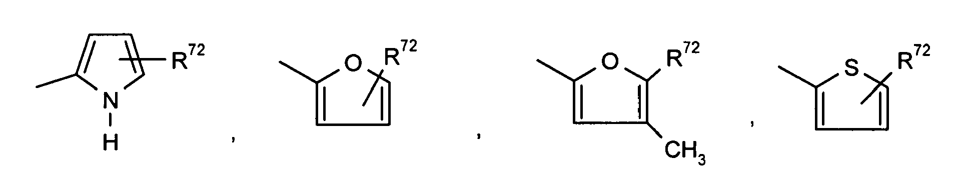
oder 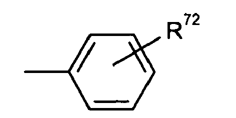 steht,
worin
- R⁷²: einen Rest der Formel -CH(OH)-CH₃ oder geradkettiges oder verzweigtes Alkyl mit 2 bis 6 Kohlenstoffatomen bedeutet, das 1- bis 2-fach durch Hydroxy oder gerad-kettiges oder verzweigtes Alkoxy mit bis zu 4 Kohlenstoffatomen substituiert ist, oder
Formyl, geradkettiges oder verzweigtes Acyl mit bis zu 6 Kohlenstoffatomen, Nitro oder geradkettiges oder verzweigtes Alkyl mit bis zu 6 Kohlenstoffatomen bedeutet, das durch Amino, Azido oder durch einen Rest der Formel -OR⁷³ substituiert ist,
worin
- R⁷³: geradkettiges oder verzweigtes Acyl mit bis zu 5 Kohlenstoffatomen oder eine Gruppe der Formel -SiR⁷⁴R⁷⁵R⁷⁶, 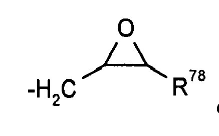
oder -CH₂-OR⁷⁹ bedeutet,
worin
- R⁷⁴, R⁷⁵ und R⁷⁶: gleich oder verschieden sind und Aryl mit 6 bis 10 Kohlenstoffatomen oder geradkettiges oder verzweigtes Alkyl mit bis zu 6 Kohlenstoffatomen bedeuten,
- R⁷⁸: Wasserstoff oder geradkettiges oder verzweigtes Alkyl mit bis zu 3 Kohlenstoff-atomen bedeutet,
und
- R⁷⁹: Wasserstoff oder geradkettiges oder verzweigtes Alkyl mit bis zu 4 Kohlen-stoffatomen bedeutet,
oder
- R⁷²: eine Gruppe der Formel 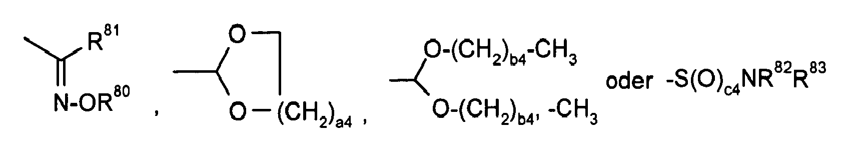
bedeutet, worin
- R⁸⁰: Wasserstoff oder geradkettiges oder verzweigtes Alkyl mit bis zu 4 Kohlenstoff-atomen bedeutet,
- R⁸¹: Wasserstoff oder geradkettiges oder verzweigtes Alkyl mit bis zu 4 Kohlenstoff-atomen bedeutet,
und
- a4: eine Zahl 1, 2 oder 3 bedeutet,
- b4 und b4': gleich oder verschieden sind, eine Zahl 0, 1, 2 oder 3 bedeutet,

- c4: eine Zahl 1 oder 2 bedeutet und
- R⁸² und R⁸³: gleich oder verschieden sind und Wasserstoff oder geradkettiges oder verzweigtes Alkyl mit bis zu 10 Kohlenstoffatomen bedeutet, das gegebenenfalls durch Cycloalkyl mit 3 bis 8 Kohlenstoffatomen oder durch Aryl mit 6 bis 10 Kohlenstoffatomen substituiert ist, das seinerseits durch Halogen substituiert sein kann oder
Aryl mit 6 bis 10 Kohlenstoffatomen bedeuten, das gegebenenfalls durch Halogen sub-stituiert ist oder
Cycloalkyl mit 3 bis 7 Kohlenstoffatomen bedeuten oder
R⁸² und R⁸³ gemeinsam mit dem Stickstoffatom einen 5- bis 7-gliedrigen gesättigten Hetero-cyclus bilden, der gegebenenfalls ein weiteres Sauerstoffatom oder einen Rest -NR⁸⁴ enthalten kann,
worin
R⁸⁴ Wasserstoff, geradkettiges oder verzweigtes Alkyl mit zu 4 Kohlenstoffatomen oder einen Rest der Formel 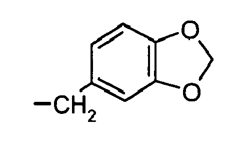 bedeutet,
oder Benzyl oder Phenyl bedeutet, wobei die Ringsysteme gegebenenfalls durch Halogen substituiert sind,
oder
- R⁷²: eine Gruppe der Formel -CH₂-OR⁸⁵ bedeutet,
worin
- R⁸⁵: Wasserstoff oder geradkettiges oder verzweigtes Alkyl mit bis zu 3 Kohlenstoff atomen bedeutet,
- R⁷⁰ und R⁷¹: gemeinsam einen Rest der Formel 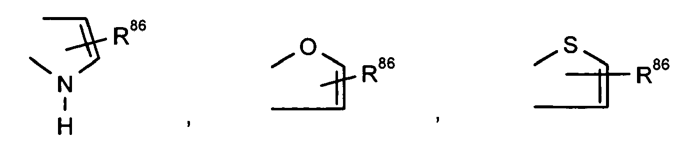
oder 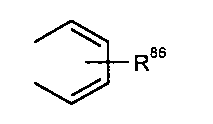 bilden,
worin
- R⁸⁶: Wasserstoff, Halogen, Hydroxy, Nitro, Amino, Trifluormethyl oder geradkettiges oder verzweigtes Alkyl oder Alkoxy mit jeweils bis zu 4 Kohlenstoffatomen oder eine Gruppe der Formel -S(O)_{c4'}NR^{82'}R^{83'} bedeutet, worin c4', R^{82'} und R^{83'} die oben angegebene Bedeutung von c4, R⁸² und R⁸³ haben und mit dieser gleich oder verschieden sind,
und deren isomere Formen und Salze,
mit der Maßgabe, dass R⁷² im Fall des Phenylringes und in der direkt zum Heteroatom benachbarten Position nur dann für die Gruppe der Formel -CH₂-OR⁸⁵ stehen darf, wenn A⁴ entweder für Phenyl steht, das durch Cyano, Nitro, Trifluormethyl, Azido, Carboxyl oder geradkettiges oder verzweigtes Alkoxycarbonyl mit bis zu 6 Kohlenstoffatomen substituiert ist oder mindestens 2-fach durch die oben aufgeführten Reste substituiert ist oder R⁸⁶ für Nitro, Amino, Trifluormethyl oder für die Gruppe der Formel -S(O)_{c4}NR^{82'}R^{83'} steht.

Die erfindungsgemäßen Verbindungen der allgemeinen Formel (IV-I) können auch in Form ihrer Salze vorliegen. Im Allgemeinen seien hier Salze mit organischen oder anorganischen Basen oder Säuren genannt.

Im Rahmen der Ausführungsform IV der vorliegenden Erfindung werden physiologisch unbedenkliche Salze bevorzugt. Physiologisch unbedenkliche Salze können Salze der erfindungsgemäßen Stoffe mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsäure, Propionsäure, Milchsäure, Weinsäure, Zitronensäure, Fumarsäure, Maleinsäure oder Benzoesäure.

Physiologisch unbedenkliche Salze können ebenso Metall- oder Ammoniumsalze der erfindungsgemäßen Verbindungen sein, wenn sie eine freie Carboxylgruppe besitzen. Besonders bevorzugt sind z.B. Natrium-, Kalium-, Magnesium- oder Calciumsalze, sowie Ammoniumsalze, die abgeleitet sind von Ammoniak, oder organischen Aminen wie beispielsweise Ethylamin, Di- bzw. Triethylamin, Di- bzw. Triethanolamin, Dicyclohexylamin, Dimethylaminoethanol, Arginin, Lysin oder Ethylendiamin.

Bevorzugt sind erfindungsgemäße Verbindungen der allgemeinen Formel (IV-I),
in welcher
- A⁴: für Phenyl steht, das gegebenenfalls bis zu 3-fach gleich oder verschieden durch Fluor, Chlor, Brom, Hydroxy, Cyano, Carboxyl, Nitro, Trifluormethyl, Trifluormethoxy, Azido, gerad-kettiges oder verzweigtes Alkyl, Alkoxy oder Alkoxycarbonyl mit jeweils bis zu 5 Kohlen-stoffatomen substituiert ist,
- R⁶⁹: für einen Rest der Formel 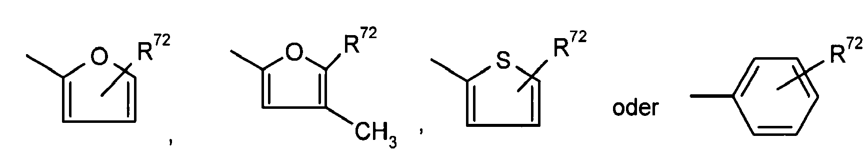
steht,
worin
- R⁷²: einen Rest der Formel -CH(OH)-CH₃ oder geradkettiges oder verzweigtes Alkyl mit 2 bis 4 Kohlenstoffatomen bedeutet, das 1- bis 2-fach durch Hydroxy oder geradkettiges oder verzweigtes Alkoxy mit bis zu 3 Kohlenstoffatomen substituiert ist, oder
Formyl, geradkettiges oder verzweigtes Acyl mit bis zu 4 Kohlenstoffatomen, Nitro oder geradkettiges oder verzweigtes Alkyl mit bis zu 4 Kohlenstoffatomen bedeutet, das durch Amino, Azido oder durch einen Rest der Formel -OR⁷³ substituiert ist,
worin

- R⁷³: geradkettiges oder verzweigtes Acyl mit bis zu 4 Kohlenstoffatomen oder eine Gruppe der Formel 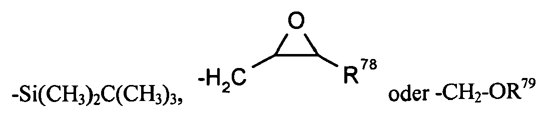
bedeutet,
worin
- R⁷⁸: Wasserstoff oder geradkettiges oder verzweigtes Alkyl mit bis zu 3 Kohlenstoffatomen bedeutet,
und
- R⁷⁹: Wasserstoff oder geradkettiges oder verzweigtes Alkyl mit bis zu 3 Kohlen-stoffatomen bedeutet,
oder
- R⁷²: eine Gruppe der Formel 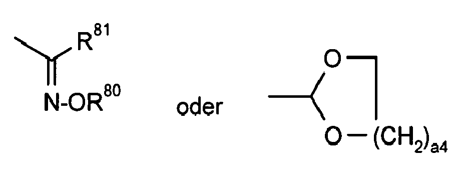
bedeutet,
worin
- R⁸⁰: Wasserstoff oder geradkettiges oder verzweigtes Alkyl mit bis zu 3 Kohlen-stoffatomen bedeutet,
- R⁸¹: Wasserstoff oder geradkettiges oder verzweigtes Alkyl mit bis zu 3 Kohlen-stoffatomen bedeutet,
und
- a4: eine Zahl 1 oder 2 bedeutet,
oder
- R⁷²: eine Gruppe der Formel -CH₂-OR⁸⁵ bedeutet,
worin
- R⁸⁵: Wasserstoff oder geradkettiges oder verzweigtes Alkyl mit bis zu 3 Kohlen-stoffatomen bedeutet,

- R⁷⁰ und R⁷¹: gemeinsam einen Rest der Formel 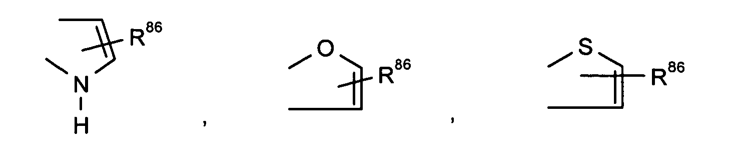
oder 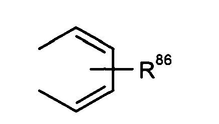 bilden,
worin
- R⁸⁶: Wasserstoff, Fluor, Chlor, Brom, Hydroxy, Nitro, Amino, Trifluormethyl oder gerad-kettiges oder verzweigtes Alkyl oder Alkoxy mit jeweils bis zu 3 Kohlenstoffatomen bedeutet,
und deren isomere Formen und Salze, mit der Maßgabe, dass R⁷² im Fall des Phenylringes und in der direkt zum Heteroatom benachbarten Position nur dann für die Gruppe der Formel -CH₂-OR⁸⁵ stehen darf, wenn A⁴ entweder für Phenyl steht, das durch Cyano, Nitro, Trifluormethyl, Azido, Carboxyl oder geradkettiges oder verzweigtes Alkoxycarbonyl mit bis zu 6 Kohlenstoffatomen substituiert ist oder mindestens 2-fach durch die oben aufgeführten Reste substituiert ist, oder
- R⁸⁶: für Nitro, Amino oder Trifluormethyl steht.
Besonders bevorzugt sind erfindungsgemäße Verbindungen der allgemeinen Formel (IV-I),
in welcher
- A⁴: für Phenyl steht, das gegebenenfalls bis zu 3-fach gleich oder verschieden durch Fluor, Chlor, Brom, Trifluormethyl, Trifluormethoxy, Cyano, Nitro, Carboxyl, Azido, geradkettiges oder verzweigtes Alkyl, Alkoxy der Alkoxycarbonyl mit jeweils bis zu 3 Kohlenstoffatomen substituiert ist,
- R⁶⁹: für einen Rest der Formel 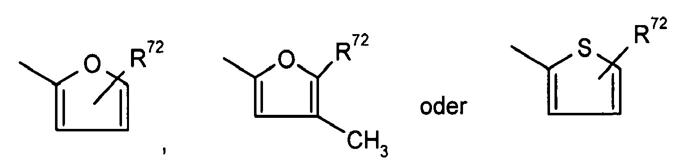
steht,
worin
- R⁷²: einen Rest der Formel -CH(OH)-CH₃ oder geradkettiges oder verzweigtes Alkyl mit 2 bis 4 Kohlenstoffatomen bedeuten, das 1- bis 2-fach durch Hydroxy, Methyl oder Methoxy substituiert ist, oder
Formyl, geradkettiges oder verzweigtes Acyl mit bis zu 3 Kohlenstoffatomen, Nitro oder geradkettiges oder verzweigtes Alkyl mit bis zu 3 Kohlenstoffatomen bedeuten, das durch Amino, Azido oder durch einen Rest der Formel -OR⁷³ substituiert ist,
worin

- R⁷³: geradkettiges oder verzweigtes Acyl mit bis zu 3 Kohlenstoffatomen oder eine Gruppe der Formel 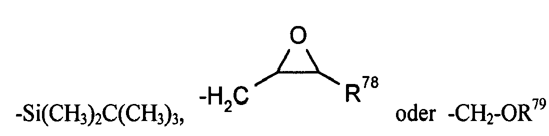
bedeutet,
worin
- R⁷⁸: Wasserstoff oder Methyl bedeutet,
und
- R⁷⁹: Wasserstoff oder geradkettiges oder verzweigtes Alkyl mit bis zu 3 Kohlenstoff-atomen bedeutet,
oder
- R⁷²: eine Gruppe der Formel 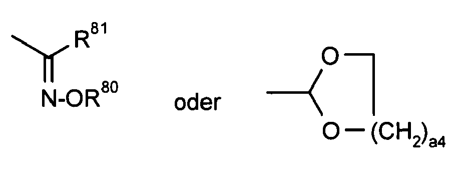
bedeutet,
worin
- R⁸⁰: Wasserstoff oder geradkettiges oder verzweigtes Alkyl mit bis zu 3 Kohlen-stoffatomen bedeutet,
- R⁸¹: Wasserstoff oder Methyl bedeutet,
und
- a4: eine Zahl 1 oder 2 bedeutet,
oder
- R⁷²: gleich oder verschieden ist und die Gruppe der Formel -CH₂-OR⁸⁵ bedeutet,
worin
- R⁸⁵: Wasserstoff oder Methyl bedeutet,
R⁷⁰ und R⁷¹ gemeinsam einen Rest der Formel 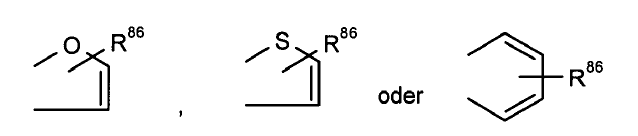 bilden,
worin
- R⁸⁶: Wasserstoff, Fluor, Chlor, Brom, Nitro, Trifluormethyl, Amino, Hydroxy oder geradkettiges oder verzweigtes Alkyl oder Alkoxy mit jeweils bis zu 3 Kohlenstoff-atomen bedeutet,
und deren isomere Formen und Salze,
mit der Maßgabe, dass R⁷² im Fall des Phenylringes und in der direkt zum Heteroatom benachbarten Position nur dann für die Gruppe der Formel -CH₂-OR⁸⁵ stehen darf, wenn A entweder für Phenyl steht, das durch Cyano, Nitro, Trifluormethyl, Azido, Carboxyl oder geradkettiges oder verzweigtes Alkoxycarbonyl mit bis zu 6 Kohlenstoffatomen substituiert ist oder mindestens 2-fach durch die oben aufgeführten Reste substituiert ist oder R⁸⁶ für Nitro, Amino oder Trifluormethyl steht.

Ganz besonders bevorzugt sind erfindungsgemäße Verbindungen der allgemeinen Formel (IV-I), in welcher
- A⁴: für Phenyl steht, das gegebenenfalls bis zu 2-fach gleich oder verschieden durch Fluor, Chlor, Methyl, Methoxy, Cyano, Nitro, Trifluormethyl oder Trifluormethoxy substituiert ist.
- R⁷⁰ und R⁷¹: gemeinsam unter Einbezug der Doppelbindung einen Phenylring bilden, der gegebe-nenfalls durch Nitro, Fluor, Amino oder Methoxy substituiert ist,
mit der Maßgabe, dass R⁷² im Fall des Phenylringes und in der direkt zum Heteroatom benachbarten Position nur dann für die Gruppe der Formel -CH₂OR⁸⁵ stehen darf, wenn A⁴ entweder für Phenyl steht, das durch Cyano, Nitro, Trifluormethyl, Azido, Carboxyl oder geradkettiges oder verzweigtes Alkoxycarbonyl mit bis zu 6 Kohlenstoffatomen substituiert ist oder mindestens 2-fach durch die oben aufgeführten Reste substituiert ist,
oder
- R⁸⁶: für Nitro, Amino oder Trifluormethyl steht.

Die Erfindung betrifft weiterhin Verfahren zur Herstellung der erfindungsgemäßen Verbindungen der allgemeinen Formel (IV-I), dadurch gekennzeichnet, dass man

### [A4] Verbindungen der allgemeinen Formel (IV-II)

H₂N-NH-CH₂-A⁴ (IV-II)

in welcher
- A⁴: die oben angegebene Bedeutung hat,
durch Umsetzung mit Verbindungen der allgemeinen Formel (IV-III) 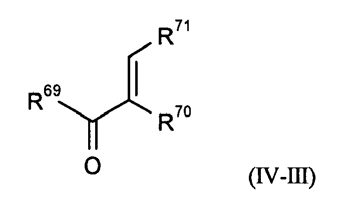 in welcher
R⁶⁹, R⁷⁰ und R⁷¹ die oben angegebene Bedeutung haben,
in die Verbindungen der allgemeinen Formel (IV-IV) 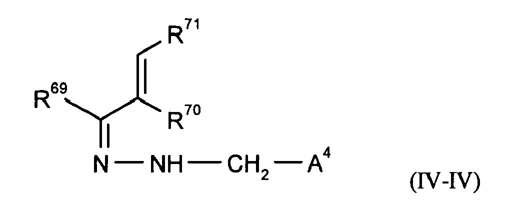 in welcher
A⁴ R⁶⁹, R⁷⁰ und R⁷¹ die oben angegebene Bedeutung haben,
in inerten Lösemitteln, gegebenenfalls in Anwesenheit einer Säure überführt,
und abschließend mit Bleitetraacetat / BF₃ x Ether oxidiert und cyclisiert,
oder

### [B4] Verbindungen der allgemeinen Formel (IV-V)

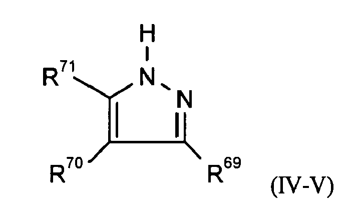

in welcher
R⁶⁹, R⁷⁰ und R⁷¹ die oben angegebene Bedeutung haben,
mit Verbindungen der allgemeinen Formel (IV-VI)

D⁴-CH₂-A⁴ (IV-VI)

in welcher
- A⁴: die oben angegebene Bedeutung hat
und
- D⁴: für Triflat oder Halogen, vorzugsweise für Brom steht,
in inerten Lösemitteln, gegebenenfalls in Anwesenheit einer Base umsetzt,
oder

### [C4] Verbindungen der allgemeinen Formel (IV-VII)

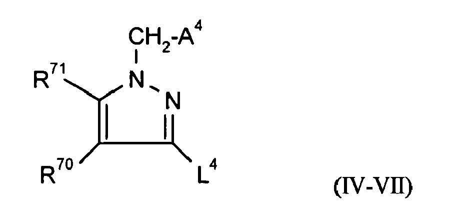

in welcher
A⁴, R⁷⁰ und R⁷¹ die oben angegebene Bedeutung haben,
und
- L⁴: für einen Rest der Formel -SnR⁸⁷R⁸⁸R⁸⁹, ZnR⁹⁰ , Iod oder Triflat steht,
worin
R⁸⁷, R⁸⁸ und R⁸⁹ gleich oder verschieden sind und
geradkettiges oder verzweigtes Alkyl mit bis zu 4 Kohlenstoffatomen bedeuten,
und
- R⁹⁰: Halogen bedeutet,
mit Verbindungen der allgemeinen Formel (IV-VIII)

R⁶⁹-T⁴ (IV-VIII)

in welcher
- R⁶⁹: die oben angegebene Bedeutung hat
und
im Fall L⁴ = SnR⁸⁷R⁸⁸R⁸⁹ oder ZnR⁹⁰
- T⁴: für Triflat oder für Halogen, vorzugsweise für Brom steht,
und
im Fall L⁴ = Jod oder Triflat
- T⁴: für einen Rest der Formel SnR^{87'}R^{88'}R^{89'}, ZnR^{90'} oder BR⁹¹R⁹²steht,
worin
- R^{87'} , R^{88'}, R^{89'} und R^{90'}: die oben angegebene Bedeutung von R⁸⁷, R⁸⁸, R⁸⁹ und R⁹⁰ haben und mit dieser gleich oder verschieden sind,
R⁹¹ und R⁹² gleich oder verschieden sind und
Hydroxy, Aryloxy mit 6 bis 10 Kohlenstoffatomen oder geradkettiges oder ver-zweigtes Alkyl oder Alkoxy mit jeweils bis zu 5 Kohlenstoffatomen bedeuten, oder gemeinsam einen 5- oder 6-gliedrigen carbocyclischen Ring bilden,
in einer palladiumkatalysierten Reaktion in inerten Lösemitteln umsetzt,
oder

### [D4] im Fall, dass R⁷² für einen Alkyl mit 2 bis 6 Kohlenstoffatomen steht, das 2-fach durch Hydroxy substituiert ist

Verbindungen der allgemeinen Formel (IV-Ia) 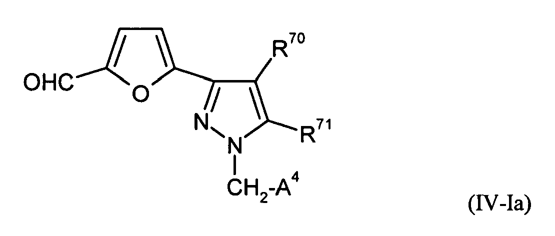 in welcher
A⁴, R⁷⁰ und R⁷¹ die oben angegebene Bedeutung haben,
zunächst durch eine Wittig-Reaktion im System (C₆H₅)₃P^{⊕}-CH₂- in die Verbindungen der allgemeinen Formel (IV-IX) 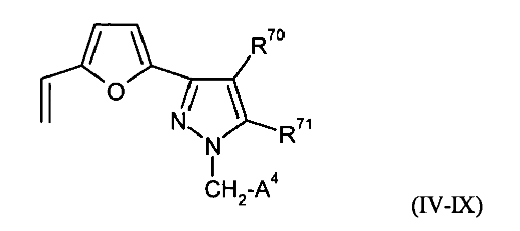 in welcher
R⁷⁰, R⁷¹ und A⁴ die oben angegebene Bedeutung haben,
überführt,
und abschließend mit Osmiumtetroxid die Hydroxyfunktionen einführt
und gegebenenfalls die unter R⁶⁹, R⁷⁰, R⁷¹ und/oder A⁴ aufgeführten Substituenten nach üblichen Methoden, vorzugsweise durch Reduktion, Oxidation, Abspaltung von Schutzgruppen und/oder nucleophiler Substitution variiert oder einführt.

Die erfindungsgemäßen Verfahren können durch folgende Formelschemata beispielhaft erläutert werden:

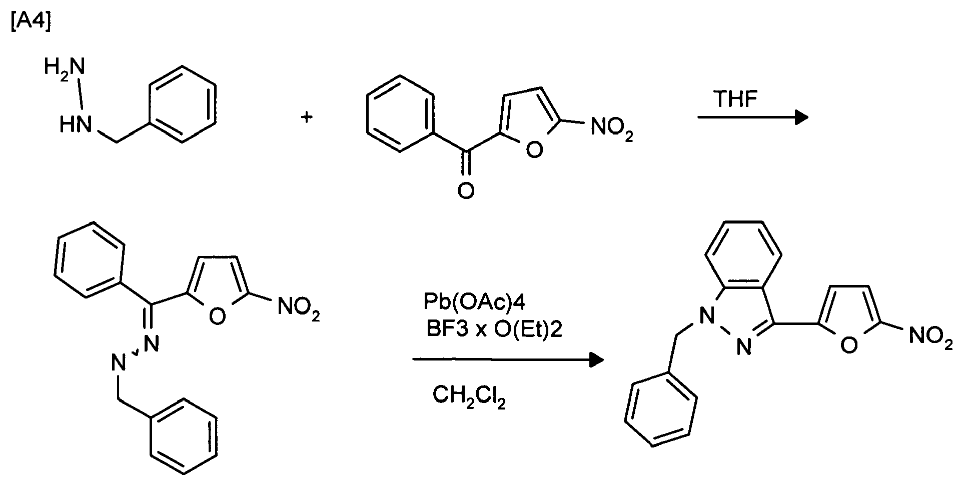

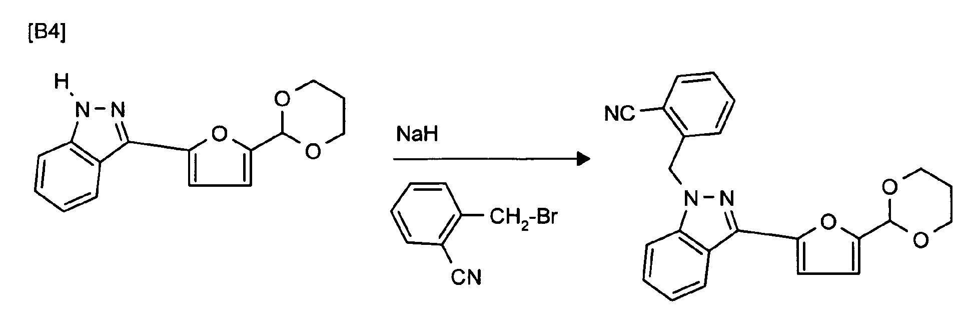

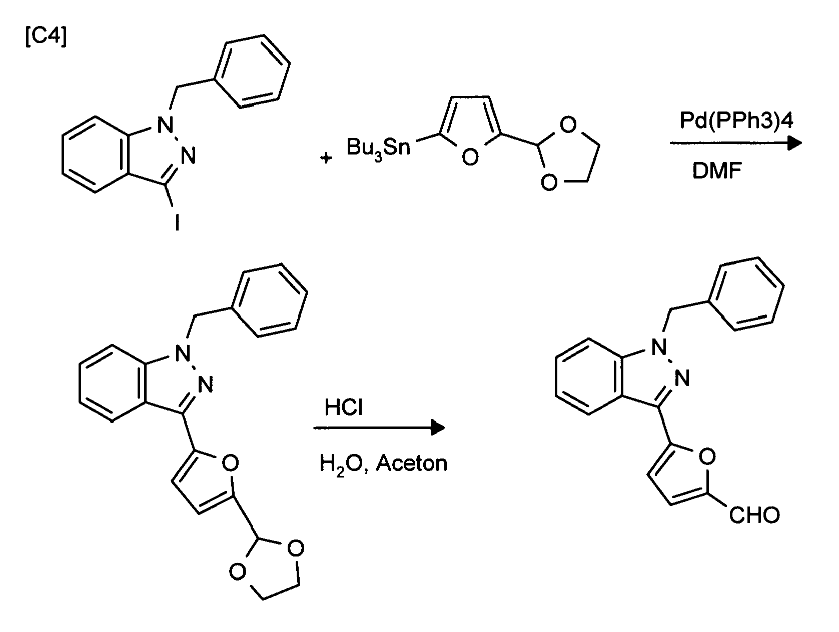

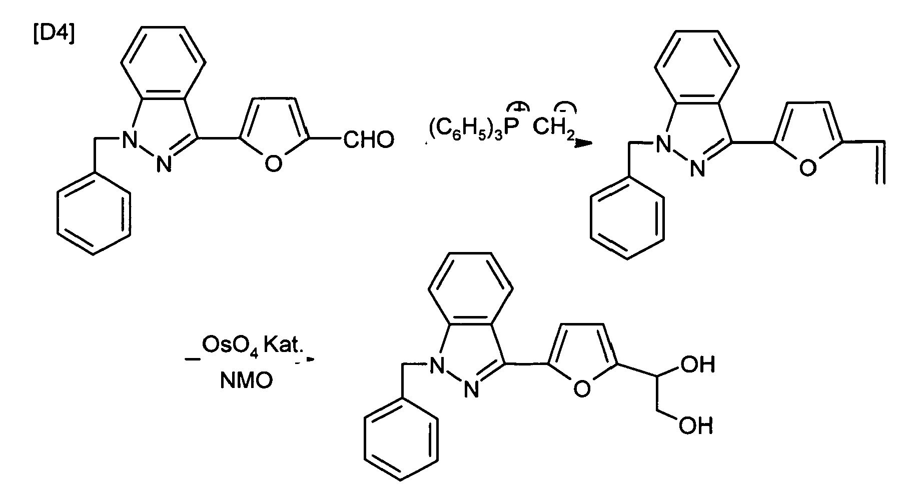

Als Lösemittel für die einzelnen Schritte des Verfahrens [A4] eignen sich im Allgemeinen inerte organische Lösemittel, die sich unter den Reaktionsbedingungen nicht verändern. Hierzu gehören Ether, wie Diethylether, Dimethoxyethan oder Tetrahydrofuran, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Alkohole wie Methanol, Ethanol oder Propanol, Kohlenwasserstoffe wie Benzol, Xylol,Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Bevorzugt für den ersten Schritt des Verfahrens [A4] sind Ethanol und THF; für die Cyclisierung Dichlormethan.

Die Reaktion wird im Allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im Allgemeinen arbeitet man bei Normaldruck.

Als Säuren eignen sich im Allgemeinen Carbonsäuren wie beispielsweise Essigsäure, Toluolsulfonsäure, Schwefelsäure oder Chlorwasserstoff. Bevorzugt ist Essigsäure.

Als Lösemittel für die einzelnen Schritte des Verfahrens [B4] eignen sich hierbei inerte organische Lösemittel, die sich unter den Reaktionsbedingungen nicht verändern. Hierzu gehören Ether, wie Diethylether oder Tetrahydrofuran, Kohlenwasserstoffe wie Benzol, Xylol, Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Tetrahydrofuran, Toluol oder Dimethylformamid.

Als Basen für das erfindungsgemäße Verfahren können im Allgemeinen anorganische oder organische Basen eingesetzt werden. Hierzu gehören vorzugsweise Alkalihydroxide wie zum Beispiel Natriumhydroxid oder Kaliumhydroxid, Erdalkalihydroxide wie zum Beispiel Bariumhydroxid, Alkalicarbonate wie Natriumcarbonat oder Kaliumcarbonat, Erdalkalicarbonate wie Calciumcarbonat, oder Alkali- oder Erdalkalialkoholate wie Natrium- oder Kaliummethanolat, Natrium- oder Kaliumethanolat oder Kalium-tert.butylat, oder organische Amine (Trialkyl(C₁-C₆)amine) wie Triethylamin, oder Heterocyclen wie 1,4-Diazabicyclo[2.2.2]octan (DABCO), 1,8-Diazabicyclo-[5.4.0]undec-7-en (DBU), Pyridin, Diaminopyridin, Methylpiperidin oder Morpholin. Es ist auch möglich als Basen Alkalimetalle wie Natrium und deren Hydride wie Natriumhydrid einzusetzen. Bevorzugt sind Natrium- und Kaliumcarbonat, Triethylamin und Natriumhydrid.

Die Base wird in einer Menge von 1 mol bis 5 mol, bevorzugt von 1 mol bis 3 mol, bezogen auf 1 mol der Verbindung der allgemeinen Formel (II) eingesetzt.

Die Umsetzung wird im Allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgeführt werden (z.B. 0,5 bis 5 bar). Im Allgemeinen arbeitet man bei Normaldruck.

Als Lösemittel für die Verfahren [C4] und [D4] eignen sich hierbei inerte organische Lösemittel, die sich unter den Reaktionsbedingungen nicht verändern. Hierzu gehören Ether, wie Diethylether oder Tetrahydrofuran, DME, Dioxan, Halogenkohlenwasserstoffe wie Dichlormethan, Trichlormethan, Tetrachlormethan, 1,2-Dichlorethan, Trichlorethan, Tetrachlorethan, 1,2-Dichlorethan oder Trichlorethylen, Kohlenwasserstoffe wie Benzol, Xylol,Toluol, Hexan, Cyclohexan, oder Erdölfraktionen, Nitromethan, Dimethylformamid, Aceton, Acetonitril oder Hexamethylphosphorsäuretriamid. Ebenso ist es möglich, Gemische der Lösemittel einzusetzen. Besonders bevorzugt sind Tetrahydrofuran, Dimethylformamid, Toluol, Dioxan oder Dimethoxyethan.

Die Reaktion wird im Allgemeinen in einem Temperaturbereich von 0°C bis 150°C, bevorzugt von +20°C bis +110°C durchgeführt.

Die Umsetzung kann bei normalen, erhöhtem oder bei erniedrigtem Druck durchgerührt werden (z.B. 0,5 bis 5 bar). Im Allgemeinen arbeitet man bei Normaldruck.

Als Palladiumverbindungen im Rahmen der vorliegenden Erfidung eignen sich im Allgemeinen PdCl₂((C₆H₅)₃)₂, Palladium-bis-dibenzylidenaceton (Pd(dba)₂), [1,1'-Bis-(diphenylphosphino)ferrocen]-Palladium(II)-chlorid (Pd(dppf)Cl₂) oder Pd(P(C₆H₅)₃)₄. Bevorzugt ist Pd(P(C₆H₅)₃)₄.

Die Verbindungen der allgemeinen Formeln (IV-II), (IV-III), (IV-VI) und (IV-VIII) sind an sich bekannt oder nach üblichen Methoden herstellbar.

Die Verbindungen der allgemeinen Formel (IV-IV) sind teilweise bekannt oder können wie oben beschrieben hergestellt werden.

Die Verbindungen der allgemeinen Formel (IV-V) sind teilweise bekannt und können hergestellt werden, indem man Verbindungen der allgemeinen Formel (IX) 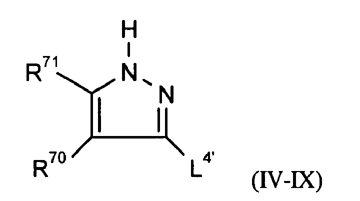 in welcher
R⁷⁰ und R⁷¹ die oben angegebene Bedeutung haben
und
- L^{4'}: die oben angegebene Bedeutung von L⁴ hat und mit dieser gleich oder verschieden ist,
mit Verbindungen der allgemeinen Formel (IV-VIII) in Analogie zu dem oben aufgeführten Verfahren [C4] umsetzt.

Die Verbindungen der allgemeinen Formel (IV-VII) sind teilweise bekannt oder im Fall der Stannyle neu und können dann beispielsweise hergestellt werden, indem man die Verbindungen der allgemeinen Formel (IV-VIIa) 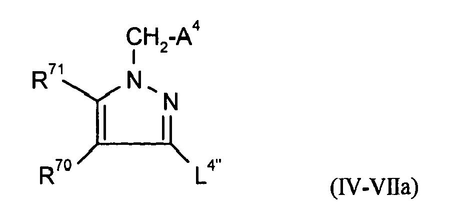 in welcher
R⁷⁰, R⁷¹ und A die oben angegebene Bedeutung haben,
- L^{4"}: für Triflat oder Halogen, vorzugsweise für Iod steht,
mit Verbindungen der allgemeinen Formel (IV-X)

(SnR⁸⁷R⁸⁸R⁸⁹)2 (IV-X)

in welcher
R⁸⁷, R⁸⁸, R⁸⁹ die oben angegebene Bedeutung haben
wie oben beschrieben unter Palladiumkatalyse umsetzt.

Die Verbindungen der allgemeinen Formeln (IV-VIIa), (IV-IX) und (IV-X) sind bekannt oder nach üblichen Methoden herstellbar.

Die Verbindungen der allgemeinen Formel (IV-IX) sind neu und können wie oben beschrieben hergestellt werden.

Die Reduktionen werden im Allgemeinen mit Reduktionsmitteln, bevorzugt mit solchen, die für die Reduktion von Carbonyl zu Hydroxyverbindungen geeignet sind, durchgeführt werden. Besonders geeignet ist hierbei die Reduktion mit Metallhydriden oder komplexen Metallhydriden in inerten Lösemitteln, gegebenenfalls in Anwesenheit eines Trialkylborans. Bevorzugt wird die Reduktion mit komplexen Metallhydriden wie beispielsweise Lithiumboranat, Natriumboranat, Kaliumboranat, Zinkboranat, Lithium-trialkylhydrido-boranat, Diisobutylaluminiumhydrid oder Lithiumaluminiumhydrid durchgeführt. Ganz besonders bevorzugt wird die Reduktion mit Diisobutylaluminiumhydrid und Natriumborhydrid durchgeführt.

Das Reduktionsmittel wird im Allgemeinen in einer Menge von 1 mol bis 6 mol, bevorzugt von 1 mol bis 4 mol bezogen auf 1 mol der zu reduzierenden Verbindungen, eingesetzt.

Die Reduktion verläuft im Allgemeinen in einem Temperaturbereich von -78°C bis +50°C, bevorzugt von -78°C bis 0°C, im Falle des DIBAH, 0°C, Raumtemperatur im Falle des NaBH₄, besonders bevorzugt bei -78°C, jeweils in Abhängigkeit von der Wahl des Reduktionsmittels sowie Lösemittel.

Die Reduktion verläuft im Allgemeinen bei Normaldruck, es ist aber auch möglich bei erhöhtem oder erniedrigtem Druck zu arbeiten.

Im Fall der Reste -S(O)_{c4}R⁸¹R⁸² und -S(O)_{c4'}R^{81'}R^{82'} werden die entsprechenden unsubstituierten Verbindungen der allgemeinen Formel (IV-I) zunächst mit Thionylchlorid umgesetzt. In einem weiteren Schritt erfolgt die Umsetzung mit den Aminen in einem der oben aufgeführten Ether, vorzugsweise Dioxan. Im Fall c4 = 2 wird anschließend eine Oxidation nach üblichen Methoden durchgeführt. Die Umsetzungen erfolgen in einem Temperaturbereich von 0°C bis 70°C und Normaldruck.

Die Abspaltung der Schutzgruppe erfolgt im Allgemeinen in einem der oben aufgeführten Alkohole und/oder THF oder Aceton, vorzugsweise Methanol / THF in Anwesenheit von Salzsäure oder Trifluoressigsäure oder Toluolsulfonsäure in einem Temperaturbereich von 0°C bis 70°C, vorzugsweise bei Raumtemperatur und Normaldruck.

Die Verbindungen der allgemeinen Formel (IV-Ic) sind neu und können wie unter den Verfahren [A4] bis [C4] beschrieben hergestellt werden.

Darüber hinaus umfasst die Erfindung die Kombination der erfindungsgemäßen Verbindungen der allgemeinen Formeln (IV-I) und (IV-Ia) mit organischen Nitraten und NO-Donatoren.

Organische Nitrate und NO-Donatoren im Rahmen der Erfindung sind im Allgemeinen Substanzen, die über die Freisetzung von NO bzw. NO-Species ihre therapeutische Wirkung entfalten. Bevorzugt sind Natriumnitroperussid, Nitroglycerin, Isosorbiddinitrat, Isosorbidmononitrat, Molsidomin und SIN-1.

Außerdem umfasst die Erfindung die Kombination mit Verbindungen, die den Abbau von cyclischen Guanosinmonophosphat (cGMP) inhibieren. Dies sind insbesondere Inhibitoren der Phosphodiesterasen 1, 2 und 5; Nomenklatur nach Beavo und Reifsnyder (1990) TIPS 11 S. 150 - 155. Durch diese Inhibitoren wird die Wirkung der erfindungsgemäßen Verbindungen potenziert und der gewünschte pharmakologische Effekt gesteigert.

Die erfindungsgemäßen Verbindungen der allgemeinen Formeln (IV-I) und (IV-Ia) zeigen ein nicht vorhersehbares, wertvolles pharmakologisches Wirkspektrum.

Die erfindungsgemäßen Verbindungen der allgemeinen Formeln (IV-I) und (IV-Ia) führen zu einer Gefäßrelaxation/Thrombozytenaggregationshemmung und zu einer Blutdrucksenkung sowie zu einer Steigerung des koronaren Blutflusses. Diese Wirkungen sind über eine direkte Stimulation der löslichen Guanylatzyklase und einem intrazellulären cGMP-Anstieg vermittelt. Außerdem verstärken die erfindungsgemäßen Verbindungen die Wirkung von Substanzen, die den cGMP-Spiegel steigern, wie beispielsweise EDRF (Endothelium derived relaxing factor), NO-Donatoren, Protoporphyrin IX, Arachidonsäure oder Phenylhydrazinderivate.

Sie können daher in Arzneimitteln zur Behandlung von kardiovaskulären Erkrankungen wie beispielsweise zur Behandlung des Bluthochdrucks und der Herzinsuffizienz, stabiler und instabiler Angina pectoris, peripheren und kardialen Gefäßerkrankungen, von Arrhythmien, zur Behandlung von thromboembolischen Erkrankungen und Ischämien wie Myokardinfarkt, Hirnschlag, transistorisch und ischämische Attacken, periphere Durchblutungsstörungen, Verhinderung von Restenosen wie nach Thrombolysetherapien, percutan transluminalen Angioplastien (PTA), percutan transluminalen Koronarangioplastien (PTCA), Bypass sowie zur Behandlung von Arteriosklerose und Krankheiten des Urogenitalsystems wie beispielsweise Prostatahypertrophie, erektile Dysfunktion und Inkontinenz eingesetzt werden.

Zur Feststellung der kardiovaskulären Wirkungen wurden folgende Untersuchungen durchgeführt: In in vitro-Untersuchungen an Zellen vaskulären Ursprungs wurde der Einfluss auf die Guanylatzyklaseabhängige cGMP-Bildung mit und ohne NO-Donor geprüft. Die antiaggregatorischen Eigenschaften wurden an mit kollagenstimulierten menschlichen Thrombozyten gezeigt. Die gefäßrelaxierende Wirkung wurde an mit Phenylephrin vorkontrahierten Kaninchenaortenringen bestimmt. Die blutdrucksenkende Wirkung wurde an narkotisierten Ratten untersucht.

### Stimulation der löslichen Guanylatzyklase in primären Endothelzellen

Primäre Endothelzellen wurden aus Schweineaorten durch Behandlung mit Kollagenase-Lsg isoliert. Anschließend wurden die Zellen in Kulturmedium bis zum Erreichen der Konfluenz kultiviert. Für die Untersuchungen wurden die Zellen passagiert, in Zellkulturplatten ausgesät und bis zum Erreichen der Konfluenz subkultiviert. Zur Stimulation der endothelialen Guanylatzyklase wurde das Kulturmedium abgesaugt und die Zellen einmal mit Ringerlösung gewaschen und in Stimulationspuffer mit oder ohne NO-Donor (Natrium-Nitroprussid, SNP, 1 µM) inkubiert. Im Anschluss daran wurden die Testsubstanzen (Endkonzentration 1 µM) zu den Zellen pipettiert. Nach Ende der 10-minütigen Inkubationszeit wurde die Pufferlösung abgesaugt und die Zellen 16 Stunden lang bei -20°C lysiert. Anschließend wurde das intrazelluläre cGMP radioimmunologisch bestimmt.

### Tabelle A:

| Bsp.-Nr. | cGMP-Steigerung % |
|---|---|
| IV-136 | >1000 |
| IV-138 | 324 |
| IV-139 | 723 |
| IV-140 | 619 |
| IV-143 | >1000 |
| IV-153 | 341 |
| IV-148 | 978 |
| IV-164 | 289 |
| IV-165 | 256 |
| IV-171 | 926 |
| IV-175 | 473 |
| IV-179 | 921 |

### Gefäßrelaxierende Wirkung in vitro

1,5 mm breite Ringe einer isolierten Kaninchen-Aorta werden einzeln unter einer Vorspannung in 5 ml-Organbäder mit 37°C warmer, carbogenbegaster Krebs-Henseleit-Lösung gebracht. Die Kontraktionskraft wird verstärkt und digitalisiert sowie parallel auf Linienschreiber registriert. Zur Erzeugung einer Kontraktion wird Phenylephrin dem Bad kumulativ in ansteigender Konzentration zugesetzt.

Nach mehreren Kontrollzyklen wird die zu untersuchende Substanz in jedem weiteren Durchgang in jeweils steigender Dosierung untersucht und mit der Höhe der im letzten Vordurchgang erreichten Kontraktion verglichen. Daraus wird die Konzentration errechnet, die erforderlich ist, um die Höhe des Kontrollwertes um 50 % zu reduzieren (IC₅₀). Das Standardapplikationsvolumen beträgt 5 µl.

### Tabelle B

| Bsp.-Nr. | AORTA IC₅₀ (µM) |
|---|---|
| IV-136 | 7,2 |
| IV-139 | 12 |
| IV-140 | 12-17 |
| IV-153 | 9,1 |
| IV-148 | 6,7 |
| IV-164 | 12 |
| IV-165 | 29 |
| IV-166 | 18 |
| IV-171 | 8,7 |
| IV-175 | 11 |
| IV-179 | 11 |

### Blutdruckmessungen an narkotisierten Ratten

Männliche Wistar-Ratten mit einem Körpergewicht von 300 - 350 g werden mit Thiopental (100 mg/kg i.p.) anästhesiert. Nach Tracheotomie wird in die Femoralarterie ein Katheter zur Blutdruckmessung eingeführt. Die zu prüfenden Substanzen werden als Suspension in Tyloselösung mittels Schlundsonde in verschiedenen Dosen oral verabreicht.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

### Thrombozytenaggregationshemmung in vitro

Zur Bestimmung der thrombozytenaggregationshemmenden Wirkung wurde Blut von gesunden Probanden beiderlei Geschlechts verwendet. Als Antikoagulans wurden einem Teil 3,8%iger wäßriger Natriumzitratlösung 9 Teile Blut zugemischt. Mittels Zentrifugation erhält man aus diesem Blut plättchenreicheres Zitratplasma (PRP).

Für diese Untersuchungen wurden 445 µl PRP und 5 µl der Wirkstofflösung bei 37°C im Wasserbad vorinkubiert. Anschließend wurde die Thrombozytenaggregation nach der turbidometrischen Methode im Aggregometer bei 37°C bestimmt. Hierzu wurde die vorinkubierte Probe mit 50 µl Kollagen, einem aggregationsauslösenden Agens, versetzt, und die Veränderung der optischen Dichte erfaßt. Zur quantitativen Ausweitung wurde der maximale Aggregationsresponse ermittelt und daraus die prozentuale Hemmung gegenüber der Kontrolle errechnet.

### Tabelle C

| Bsp.-Nr. | IC₅₀ (ug/ml) |
|---|---|
| IV-136 | 30 |

Die in der vorliegenden Erfindung in der Ausführungsform IV beschriebenen Verbindungen stellen auch Wirkstoffe zur Bekämpfung von Krankheiten in Zentralnervensystem dar, die durch Störungen des NO/cGMP-Systems gekennzeichnet sind. Insbesondere sind sie geeignet zur Beseitigung kognitiver Defizite, zur Verbesserung von Lern- und Gedächtnisleistungen und zur Behandlung der Alzheimer'schen Krankheit. Sie eignen sich auch zur Behandlung von Erkrankungen des Zentralnervensystems wie Angst-, Spannungs- und Depresssionszuständen, zentralnervös krankhafter Störungen der Nahrungs-, Genuss- und Suchtmittelaufnahme.

Weiterhin eignen sich diese Wirkstoffe auch zur Regulation der cerebralen Durchblutung und stellen somit wirkungsvolle Mittel zur Bekämpfung von Migräne dar.

Auch eignen sich zur Prophylaxe und Bekämpfung der Folgen cerebraler Infaktgeschehen (Apoplexia cerebri) wie Schlaganfall, cerebraler Ischämien und des Schädel-Hirn-Traumas. Ebenso können die erfindungsgemäßen Verbindungen zur Bekämpfung von Schmerzzuständen eingesetzt werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im Allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30 mg/kg Körpergewicht.

### Ausgangsverbindungen

### Ausführungsform I der Erfindung

### Beispiel I/1 A

### 5-(1,3-Dioxan-2-yl)-2-tributylstannyl-furan

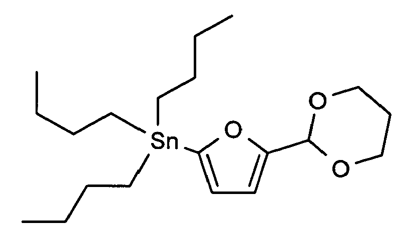

200 ml sec-Butyllithium (Lösung 1.3 M in Cyclohexan, 260 mmol) wurden zu einer Lösung von 34.4 g 2-(2-Furyl)-1,3-dioxan (224 mmol, erhältlich aus Furfural und Propan-1,3-diol) in 320 ml THF bei -70°C in 20 min zugetropft. Die Lösung wurde 30 min bei -20°C erwärmt, dann wieder auf-78°C gekühlt. Anschließend wurde eine Lösung von 60.8 ml Tributylstannylchlorid in 160 ml THF innerhalb 30 min zugetropft, wonach man die Mischung auf RT erwärmen ließ. Nach 2.5 h wurde Wasser zugegeben, und die Mischung wurde mit Essigester extrahiert. Die organische Phase wurde mit Magnesiumsulfat getrocknet, eingeengt, und der Rückstand destilliert (Kp_{0.8} 180°C). Man erhält 93 g.

### Beispiel 1/2 A

### 3-(5-(1,3-Dioxan-2-yl)furan-2-yl)indazol

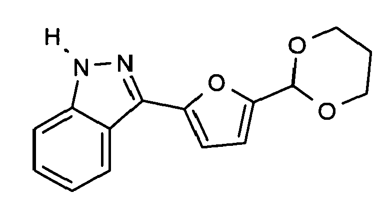

10 g (41 mmol) 3-Iodindazol (U. Wrzeciono et al., Pharmazie 1979, 34, 20) werden unter Argon in 125 ml DMF gelöst, mit 0.7 g Pd(PPh3)4 versetzt und 15 min gerührt. Man gibt 19.4 g (43.9 mmol) 2-(5-Tributylstannyl-2-furanyl)-1,3-dioxan hinzu und rührt 2 Stunden bei 100°C. Man verdampft das Lösungsmittel im Vakuum und chromatographiert auf Kieselgel mit Toluol und Toluol/Essigester-Gemischen als Eluens. Man erhält 10 g (90.3 % d. Th.) 3-(2-(5-(1,3-Dioxolan-2-yl)furyl)indazol.
Rf(SiO₂, Toluol/Essigester = 4:1): 0.1
MS (ESI/POS): 271 (82, M+H), 213 (100), 157 (10)

### Ausführungsform II der Erfindung

### Beispiel II/3 A

### 2-(1,3-Dioxan-2-yl)-6-trimethylstannylpyridin

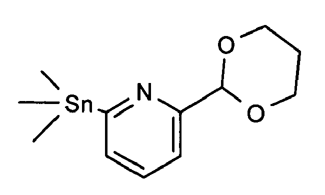

2 g (8.19 mmol) 2-(1,3-Dioxan-2-yl)-6-brompyridin (R_{f} (SiO₂, Essigester): 0.67), erhältlich aus 6-Brom-2-pyridincarboxaldehyd (Inorg. Chem. 1971, 10, 2474) und 1,3-Propandiol, werden in 50 ml Ether vorgelegt und bei -80°C mit 3.6 ml einer 2.5 N Lösung von n-BuLi in Hexan versetzt. Man rührt 30 min bei -80°C und gibt 1.8 g Trimethylzinnchlorid in 5 ml Ether hinzu. Man rührt erst bei -80°C und lässt dann auf -30°C kommen. Man gibt den Ansatz in Wasser, extrahiert mit Essigester, trocknet die organische Phase mit Natriumsulfat und dampft im Vakuum das Lösungsmittel ein. Das Produkt (1.1 g) kann ohne weitere Aufreinigung für die nächste Stufe eingesetzt werden.
R_{f} (SiO₂, Essigester): 0.2
MS (CI): 330 (80, M+H), 166 (100).

### Beispiel II/4 A

### 3-(6-(1,3-Dioxan-2-yl)-2-pyridyl)indazol

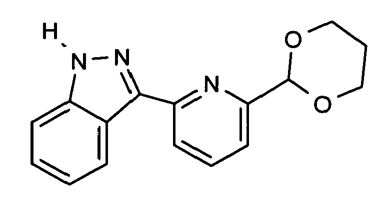

0.82 g (3.35 mmol) 3-Iodindazol werden in 10 ml DMF bei Raumtemperatur unter Argon mit 60 mg Pd(PPh₃)₄ versetzt und 15 min gerührt. Man gibt 1.1 g (3.35 mmol) 2-(1,3-Dioxan-2-yl)-6-trimethylstannylpyridin hinzu und rührt 4 Stunden bei 100°C. Anschließend wird der Ansatz im Vakuum eingedampft und der Rückstand über Kieselgel chromatographiert. Man erhält 300 mg (32% d. Th.) eines Öls.
MS (Cl/NH₃): 283(100, M+H).

### Beispiel II/5 A

### 3-Iodindazol

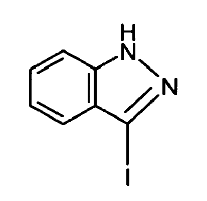

58,1 g Iod (229 mmol) wurden innerhalb einer Stunde portionsweise in eine Suspension von 25,6 g Indazol (217 mmol) in 625 ml Methanol und 625 ml 2N Natriumhydroxid-Lösung eingetragen. Die Mischung wurde 3 Tage bei Raumtemperatur gerührt und anschließend unter Eiskühlung mit 75 ml konz. Salzsäure versetzt, mit 2 N Salzsäure sauer gestellt und soviel 20 %ige Natriumthiosulfat-Pentahydrat-Lösung zugegeben, bis die Iod-Farbe verschwand. Der ausgefallene Niederschlag wurde abgesaugt, mit Wasser neutral gewaschen und im Vakuumtrockenschrank bei 50°C getrocknet. Zur Reinigung wurde der Feststoff in Methanol aufgenommen. Nach Abfiltrieren ungelöster Bestandteile wurde das Filtrat am Rotationsverdampfer zur Trockne eingeengt, wobei das Produkt als nahezu weißer Feststoff anfiel.
- Ausbeute:: 52,6 g (quant.)
- R_{f}-Wert:: 0,63 (Kieselgel; Cyclohexan/Ethylacetat 1:1)
- Schmelzpunkt:: 137°C

### Beispiel II/6 A

### 1-Benzyl-3-iodindazol

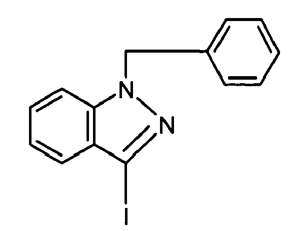

Zu einer Lösung von 12,0 g (49,2 mmol) 3-Iodindazol in 100 ml wasserfreiem Tetrahydrofuran wurden unter Argon portionsweise 1,49 g 95 %iges Natiumhydrid (59,0 mmol) gegeben. Nach 45 minütigem Rühren bei Raumtemperatur wurden 7,02 ml (59,0 mmol) Benzylbromid zugetropft. Die Mischung wurde über Nacht bei Raumtemperatur gerührt und anschließend mit Diethylether und Wasser versetzt. Die organische Phase wurde mit gesättigter Natriumchlorid-Lösung gewaschen, über Magnesiumsulfat getrocknet und am Rotationsverdampfer zur Trockne eingeengt. Der Überschuss an Benzylbromid wurde durch eine Kugelrohrdestillation abgetrennt. Der Destillationsrückstand lieferte das Produkt in Form eines Öls, das allmählich kristallisierte.
- Ausbeute:: 15,4 g (94 % d.Th.)
- R_{f}-Wert:: 0,78 (Kieselgel; Cyclohexan/Ethylacetat 1:1)
- Schmelzpunkt:: 54°C

### Beispiel II/7 A

### 1-Benzyl-3-trimethylstannylindazol

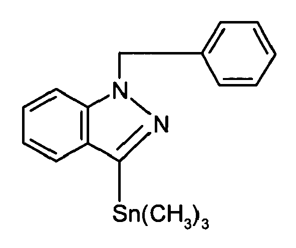

In einer Argonatmosphäre wurden 800 g 1-Benzyl-3-iodindazol (24,0 mmol), 23,7 g Hexamethyldizinn (72,0 mmol) und 2,00 g Pd(PPh₃)₄ (7,2 Mol-%) in 240 ml wasserfreiem 1,4-Dioxan über Nacht unter Rückfluss erhitzt. Die auf Raumtemperatur abgekühlte Mischung wurde mit 72 ml 1M Kaliumfluorid-Lösung und 200 ml Ethylacetat versetzt und 30 Minuten gerührt. Nach Abfiltrieren des Niederschlags über Celite wurde die organische Phase des Filtrats mit gesättigter Natriumchlorid-Lösung gewaschen, über Magnesiumsulfat getrocknet und am Rotationsverdampfer vom Lösungsmittel befreit. Der Rückstand wurde in n-Pentan verrührt, der Niederschlag abgesaugt und bei 50°C im Hochvakuum getrocknet. wodurch das Produkt in Form eines weißen Feststoffs erhalten wurde.
- Ausbeute:: 6,05 g (68 %; Reinheit: 88 % lt. GC)
- R_{f}-Wert:: 0,47 (Kieselgel; Cyclohexan/Ethylacetat 10:1)
- Schmelzpunkt:: 122°C
MS-EI:372 (Sn, M⁺, 23), 357 (Sn, 56), 207 (100), 165 (Sn, 61), 91 (68).

### Abkürzungen:

- Ph =: Phenyl
- Et =: Ethyl
- Me =: Methyl
- EE =: Essigester
- H =: Hexan
- PE =: Petrolether
- MeOH =: Methanol
- E =: Ether
- DMF =: Dimethylformamid
- Ac =: Acetyl
- KOH =: Kaliumhydroxid
- NMP =: N-Methylpyrrolidon

### Ausführungsform IV der Erfindung

### Beispiel IV/11 A

### 5-(1,3-Dioxan-2-yl)-2-tributylstannyl-furan

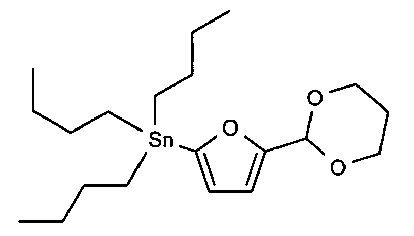

200 ml sec-Butyllithium (Lösung 1.3 M in Cyclohexan, 260 mmol) wurden zu einer Lösung von 34.4 g 2-(2-Furyl)-1,3-dioxan (224 mmol, erhältlich aus Furfural und Propan-1,3-diol) in 320 ml THF bei -70°C in 20 min zugetropft. Die Lösung wurde 30 min bei -20°C erwärmt, dann wieder auf -78°C gekühlt. Anschließend wurde eine Lösung von 60.8 ml Tributylstannylchlorid in 160 ml THF in 30 min zugetropft, wonach man die Mischung auf RT erwärmen ließ. Nach 2.5 h wurde Wasser zugegeben, und die Mischung wurde mit Essigester extrahiert. Die organische Phase wurde mit Magnesiumsulfat getrocknet, eingeengt, und der Rückstand destilliert (Kp_{0.8} 180°C). Man erhält 93 g.

### Beispiel IV/12 A

### 3-(5-(1,3-Dioxolan-2-yl)furan-2-yl)indazol

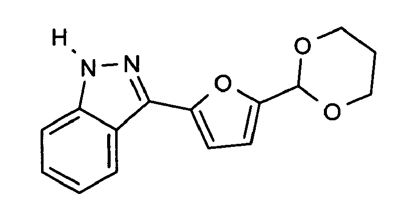

10 g (41 mmol) 3-Iodindazol (U. Wrzeciono et al., Pharmazie 1979, 34, 20) werden unter Argon in 125 ml DMF gelöst, mit 0.7 g Pd(PPh3)4 versetzt und 15 min gerührt. Man gibt 19.4 g (43.9 mmol) 2-(5-Tributylstannyl-2-furanyl)-1,3-dioxolan hinzu und rührt 2 Stunden bei 100°C. Man verdampft das Lösungsmittel im Vakuum und chromatographiert auf Kieselgel mit Toluol und Toluol/Essigester-Gemischen als Eluens. Man erhält 10 g (90.3 % d. Th.) 3-(2-(5-(1,3-Dioxolan-2-yl)furyl)-indazol.
Rf(SiO₂, Toluol/Essigester = 4:1): 0.1

### Herstellungsbeispiele

### Ausführungsform I der Erfindung

### Beispiel I/1

### 3-(5-(1,3-Dioxan-2-yl)furan-2-yl))-1-(4-picolyl)indazol

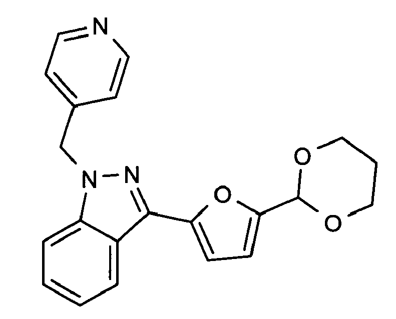

Man gibt zu einer Suspension von 355 mg NaH (60-proz. in Paraffin) in 10 ml DMF unter Argon eine Lösung von 2 g (7.41 mmol) 3-(5-(1,3-Dioxan-2-yl)furan-2-yl)indazol in 10 ml DMF und rührt eine Stunde bei Raumtemperatur. Anschließend gibt man 1.46 g 4-Picolylchlorid Hydrochlorid hinzu, gefolgt von 355 mg NaH (60-proz. in Paraffin). Man rührt 1 h bei Raumtemperatur und dann 1 h bei 100°C, gibt in Wasser, extrahiert mit Essigester, trocknet die organische Phase mit Natriumsulfat, verdampft im Vakuum und chromatographiert den Rückstand auf Kieselgel mit Toluol/Essigestergemischen als Eluens.
Man erhält 1 g (37 % d. Th.) eines Öls.
Rf (SiO₂, Essigester): 0.25

### Beispiel I/2

### 3-(5-Formyl-2-furyl)-1-(4-picolyl)indazol

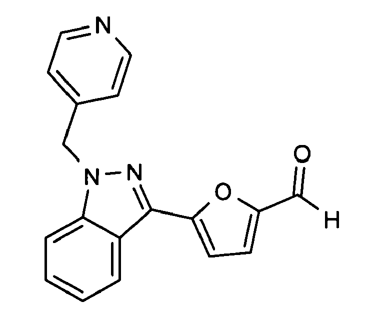

Man löst 1 g (2.77 mmol) 3-(5-(1,3-Dioxan-2-yl)furan-2-yl)-1-(4-picolyl)indazol in 10 ml Aceton und gibt 20 ml 50-proz. Essigsäure hinzu. Man kocht eine Stunde, gibt in Wasser, extrahiert mit Essigester, trocknet die organische Phase mit Natriumsulfat, verdampft im Vakuum und erhält 0.8 g (95.3 % d. Th.) eines Öls.
Rf (SiO₂, Essigester): 0.25

### Beispiel I/3

### 3-(2-(5-Hydroxymethylfuryl))-1-(4-picolyl)indazol

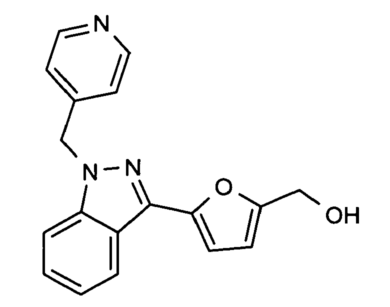

Man suspendiert 0.4 g (1.3 mmol) 3-(5-Formyl-2-furanyl)-1-(4-picolyl)indazol in 20 ml Propanol und gibt bei 0°C langsam 0.4 g NaBH4 hinzu. Nach 1 Stunde Rühren bei Raumtemperatur gibt man die klare Lösung in Wasser, extrahiert mit Essigester, trocknet die organische Phase mit Natriumsulfat, verdampft im Vakuum und chromatographiert den Rückstand auf Kieselgel mit Toluol/Essigestergemischen als Eluens.

Man erhält 200 mg (50 % d. Th.) Kristalle.
Mp.183°C
Rf (SiO₂, Essigester): 0.14
In Analogie zu den Vorschriften der Beispiele I/1, I/2 und I/3 werden die in den Tabellen I/1, I/2 und I/3 aufgeführten Verbindungen hergestellt:

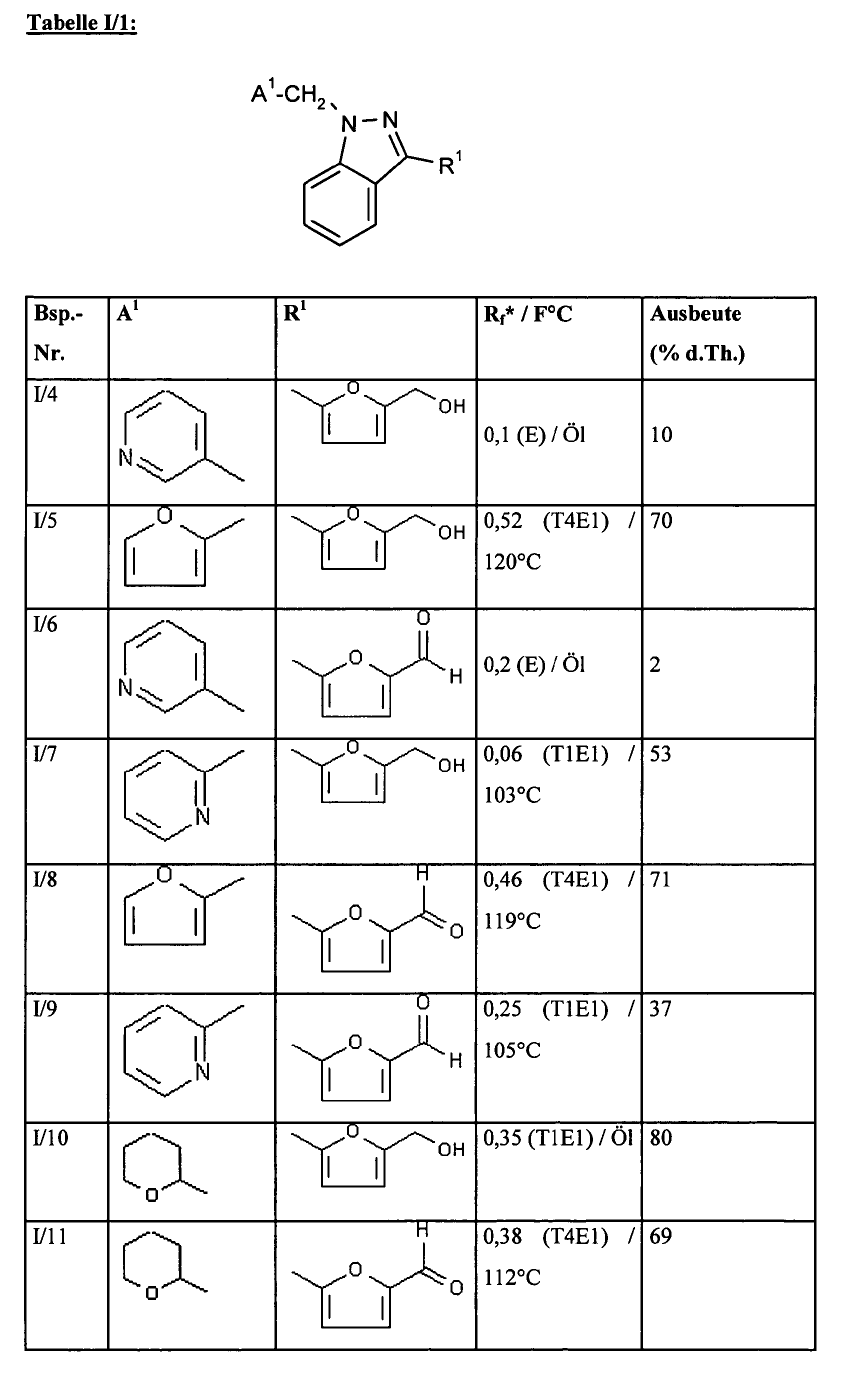

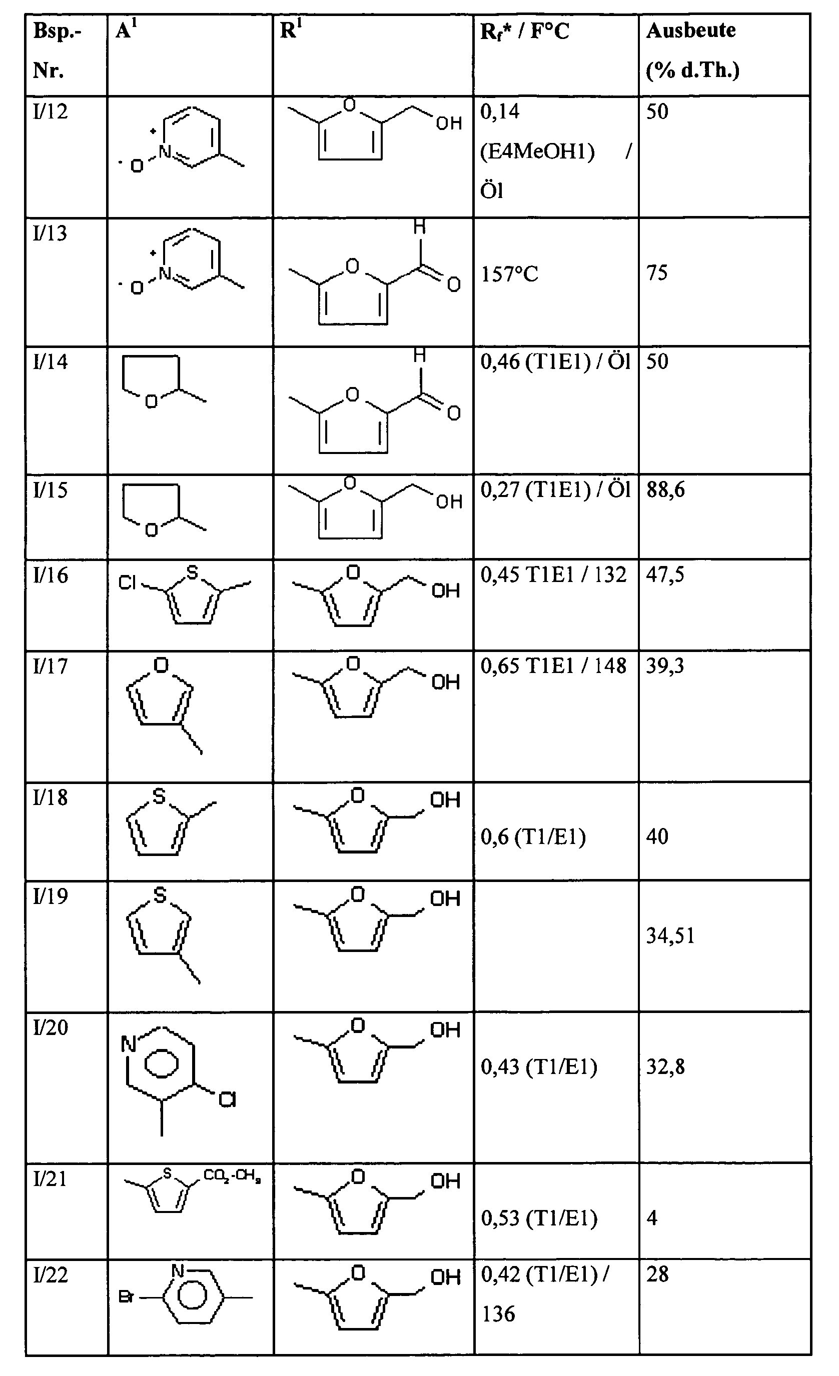

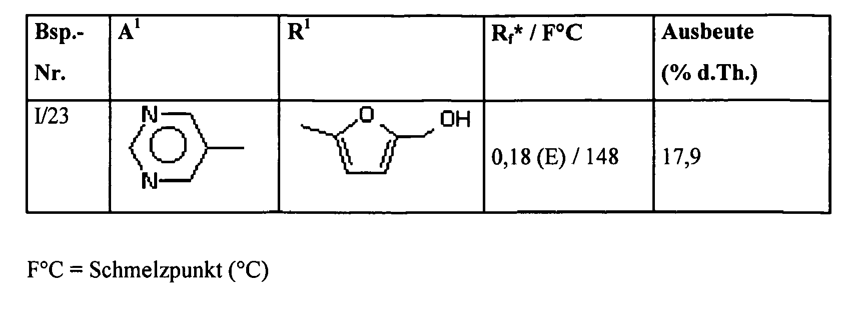

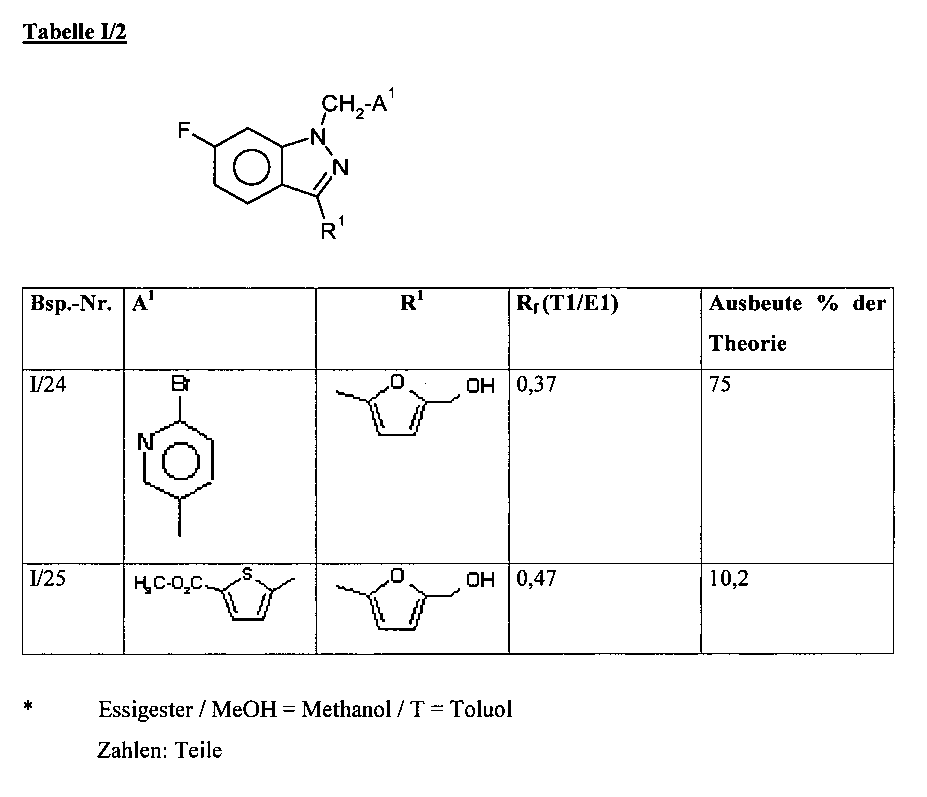

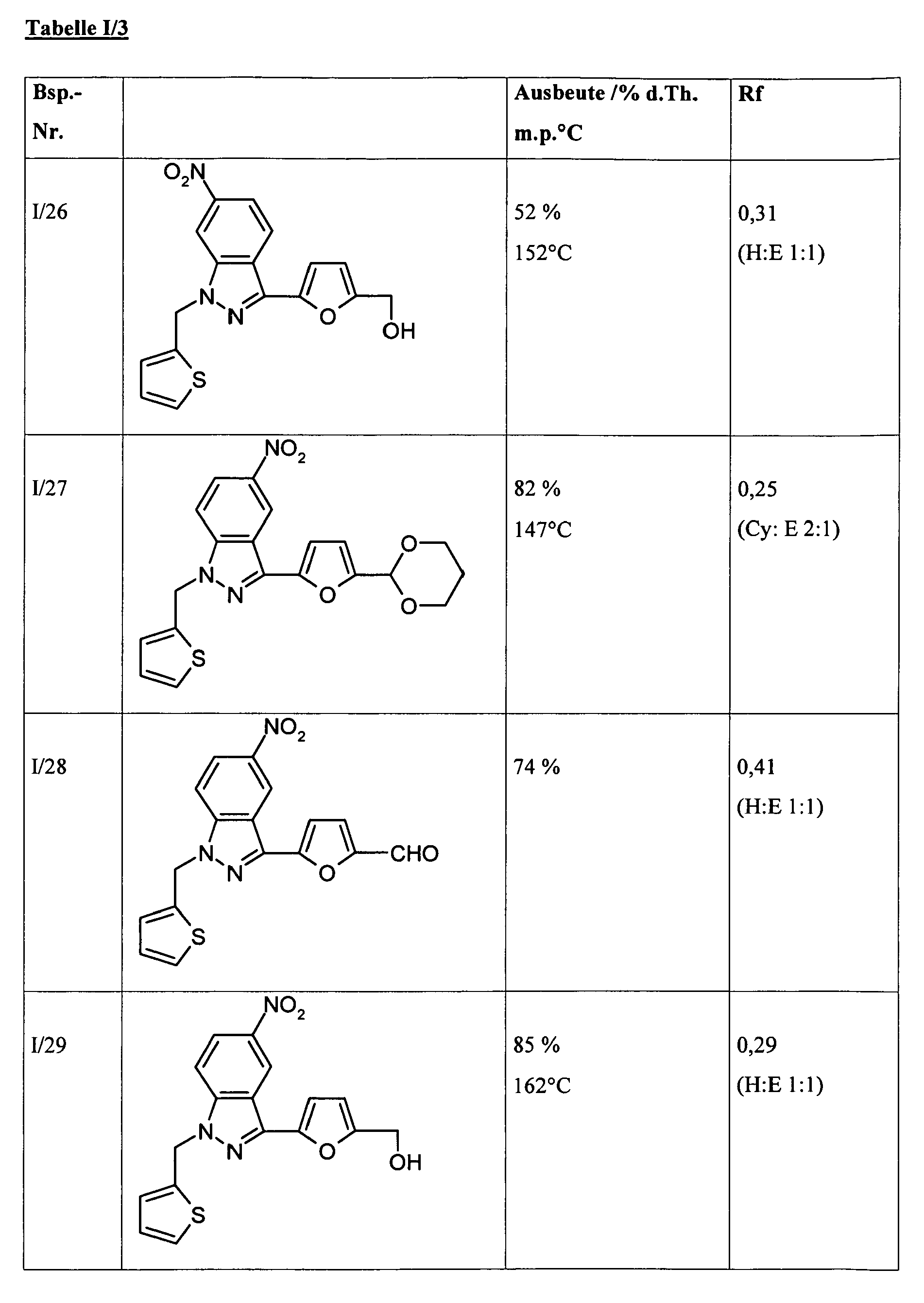

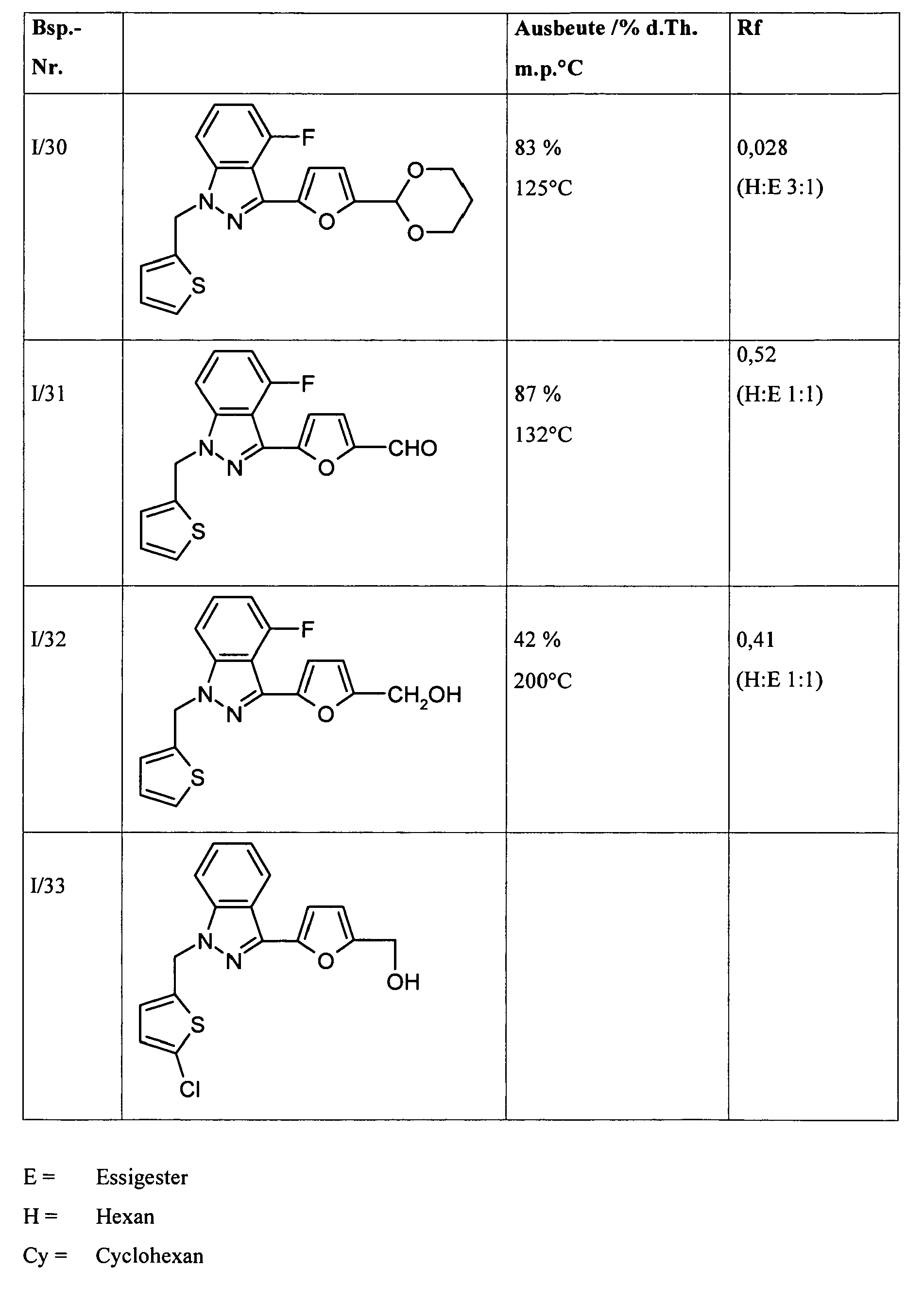

### Ausführungsform II der Erfindung

### Beispiel II/34

### 1-Benzyl-3-(6-(1,3-dioxan-2-yl)-2-pyridyl)indazol

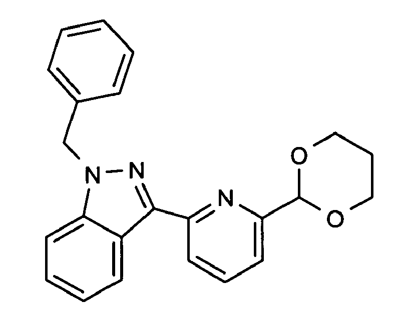

3.7 g (13.1 mmol) 3-(6-(1,3-Dioxan-2-yl)-2-pyridyl)indazol werden in THF unter Argon langsam mit 580 mg NaH (60-proz. in Paraffin) versetzt. Nachdem man 30 min gerührt hat, gibt man 1.71 ml Benzylbromid hinzu und rührt eine Stunde bei Raumtemperatur. Anschließend wird der Ansatz in Wasser gegeben, mit Essigester extrahiert, die organische Phase mit Magnesiumsulfat getrocknet, im Vakuum eingedampft, auf Kieselgel chromatographiert und mit Essigester/Toluol-Gemischen eluiert. Man erhält 1.52 g (31 % d. Th.) eines Öls.
Rf (SiO2, Essigester): 0.3
MS 372 (100, M+1)

### Beispiel II/35

### 1-Benzyl-3-(6-formyl-2-pyridyl)indazol

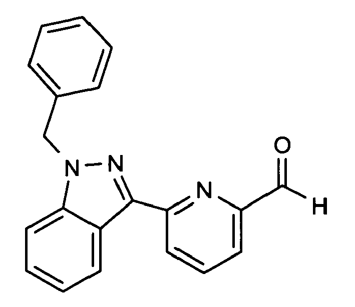

Man löst 1.52 g (4.1 mmol) 1-Benzyl-3-(6-(1,3-dioxan-2-yl)-2-pyridyl)indazol in 10 ml Aceton und gibt 20 ml 50-proz. Essigsäure hinzu. Man rührt drei Stunden bei 50°C, gibt in Wasser, extrahiert mit Essigester, trocknet die organische Phase mit Natriumsulfat, verdampft im Vakuum, chromatographiert auf Kieselgel mit Toluol/Essigester-Gemischen und erhält 180 mg (14% d. Th.) eines Öls.
Rf (SiO2, Toluol/ Essigester): 0.7
MS (CI/NH3): 314 (100, M+H).

### Beispiel II/36

### 1-Benzyl-3-(6-hydroxymethyl-2-pyridyl)indazol

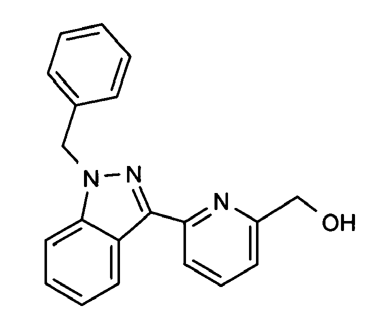

Man suspendiert 180 mg (0.57 mmol) 1-Benzyl-3-(6-formyl-2-pyridyl)indazol in 20 ml Propanol und gibt langsam 180 mg NaBH4 hinzu. Nach 30-minütigem Rühren bei Raumtemperatur gibt man die klare Lösung in Wasser, extrahiert mit Essigester, trocknet die organische Phase mit Natriumsulfat, verdampft im Vakuum und chromatographiert den Rückstand auf Kieselgel mit Toluol/Essigestergemischen als Eluens.
Man erhält 120 mg (66% d. Th.) Kristalle.
Mp. 75°C
Rf (SiO2, Essigester): 0.15
MS (CI, NH3): 316 (100, M+H).

### Beispiel II/37

### 1-Benzyl-3-(2-pyrimidyl)indazol

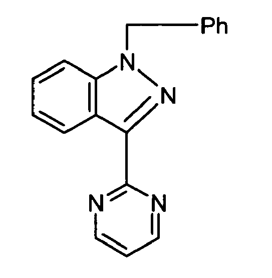

In einer Argon-Atmosphäre wurden 200 mg 1-Benzyl-3-trimethylstannylindazol (Rohprodukt, 70 % lt. GC), 35 mg 2-Chlorpyrimidin (0,30 mmol) und 29 mg (0,025 mmol) Pd(PPh3)4 in 2,5 ml Toluol über Nacht unter Rückfluss erhitzt. Die auf Raumtemperatur abgekühlte Mischung wurde mit gesättigter Ammoniumchlorid-Lösung versetzt und mit Ethylacetat extrahiert. Die organische Phase wurde über Magnesiumsulfat getrocknet und am Rotationsverdampfer vom Lösungsmittel befreit. Die Reinigung erfolgte durch Chromatographie an Aluminiumoxid mit Cyclohexan/Ethylacetat als Eluens (Gradient von 10:1 bis 1:1)
- Ausbeute:: 80 m g (93 %)
- Rf-Wert:: 0,67 (Aluminiumoxid; Cyclohexan/Ethylacetat 10:1)
- Schmelzpunkt:: 154°C
MS-EI:286 (M+, 100), 285 (64), 209 (40), 91 (71)

### Beispiel II/38

### 1-Benzyl-3-(4,5-dimethyl-2-pyrimidyl)indazol

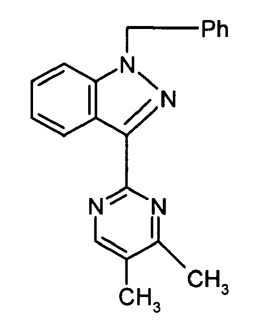

In einer Argon-Atmosphäre wurden 640 mg 1-Benzyl-3-trimethylstannylindazol (1,72 mmol), 212 mg 2-Chlor-4,5-dimethylpyrimidin^{*} (1,49 mmol) und 72 mg (0,10 mmol) Pd(PPh3)2C12 (5,8 Mol-%) in 20 ml Toluol über Nacht unter Rückfluss erhitzt. Die auf Raumtemperatur abgekühlte Mischung wurde mit gesättigter Ammoniumchlorid-Lösung versetzt und mit Ethylacetat extrahiert. Die organische Phase wurde über Magnesiumsulfat getrocknet und am Rotationsverdampfer vom Lösungsmittel befreit. Die Reinigung erfolgte durch Chromatographie an Kieselgel mit Cyclohexan/Ethylacetat als Eluens (Gradient von 10: 1 bis 1: 1)
- Ausbeute:: 239 mg (51 % d.Th.)
- Rf-Wert:: 0,33 (Kieselgel; Cyclohexan/Ethylacetat 1:1)
- Schmelzpunkt:: 119°C
*Sugasawa et al., Yakugaku Zasshi, 71, 1951, 1345, 1348, Chem. Abstr., 1952, 8034.

In Analogie zu den Vorschriften der Beispiele II/34-38 wurden die in den Tabellen II/1, II/2 und II/3 aufgeführten Beispiele hergestellt:

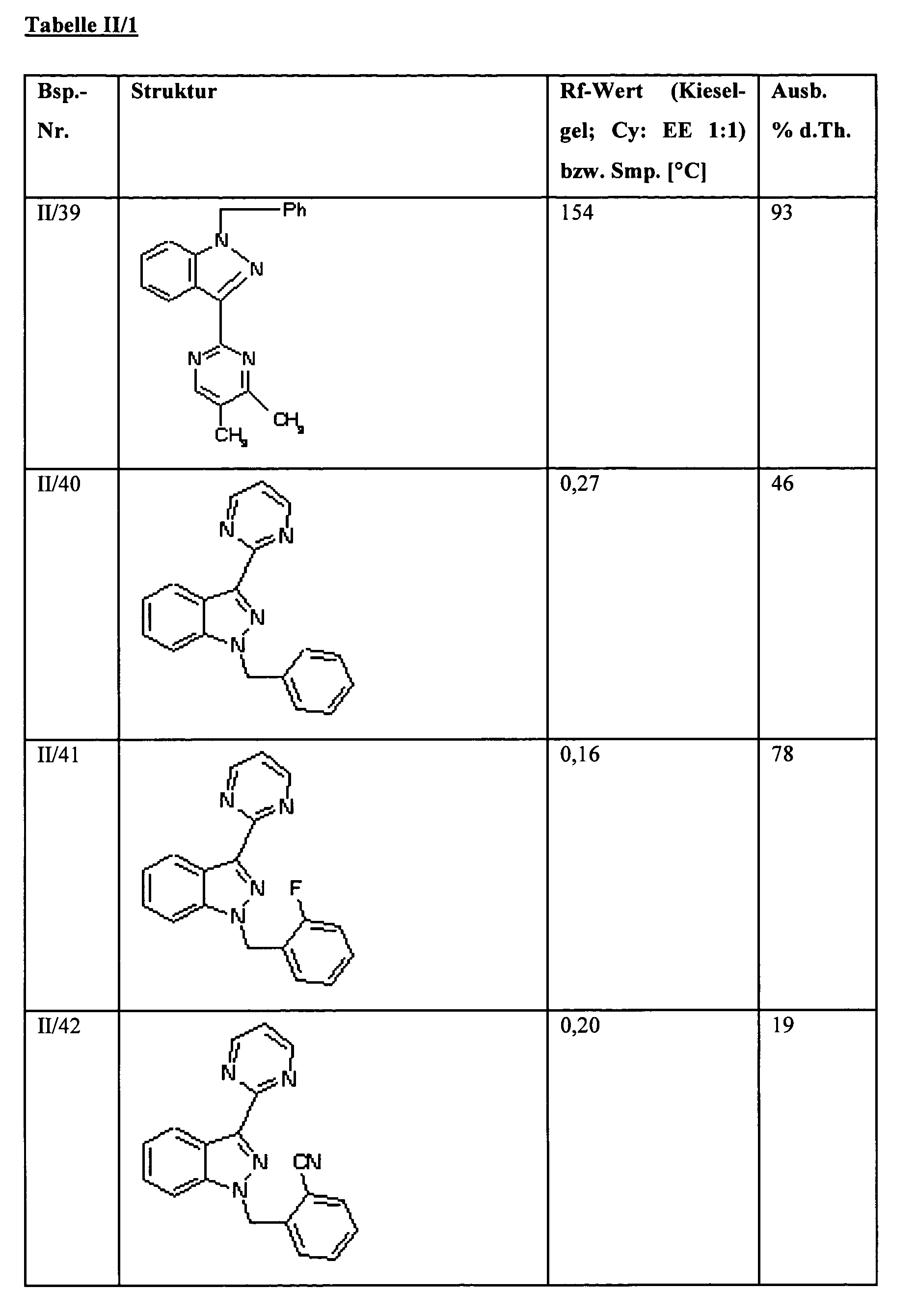

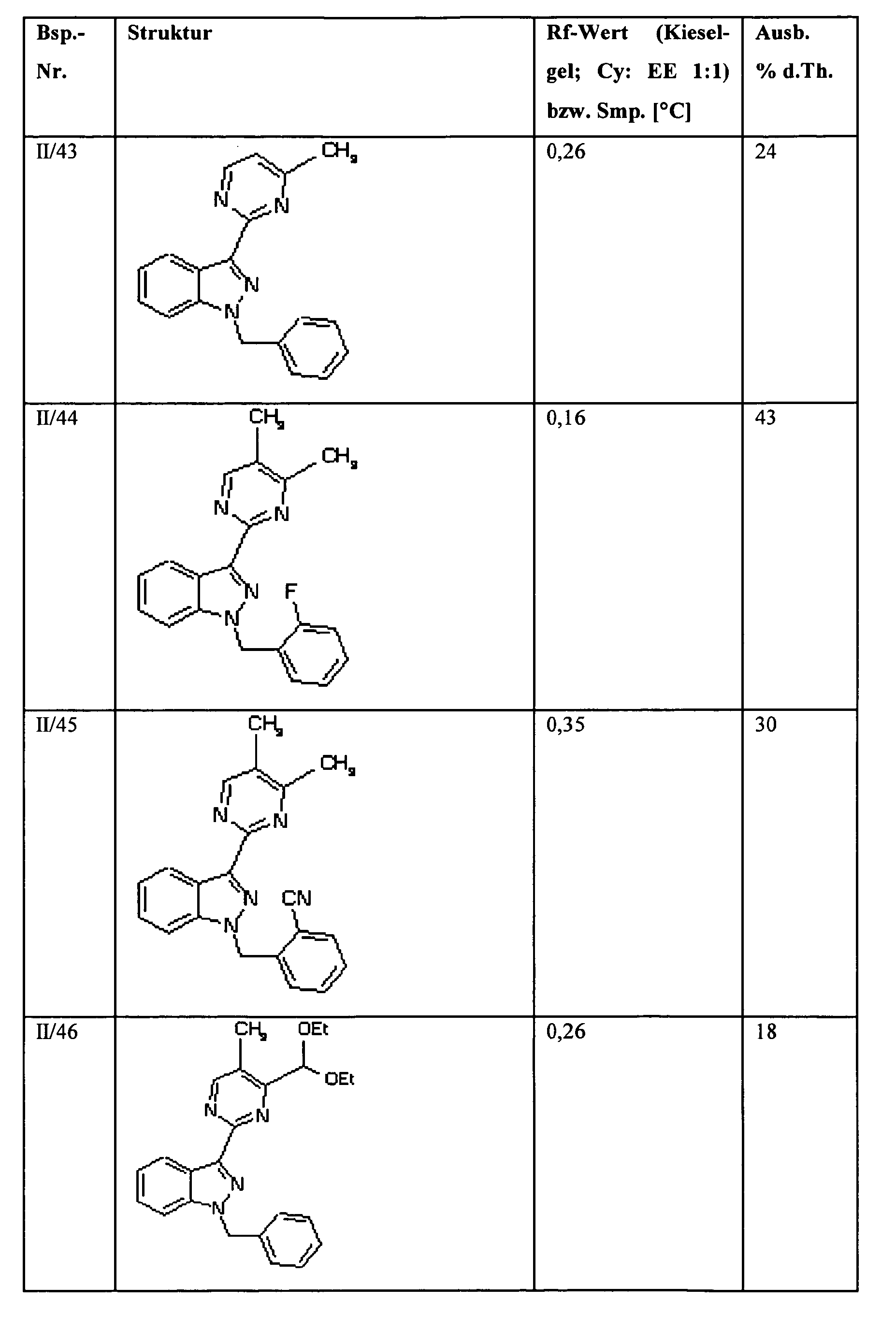

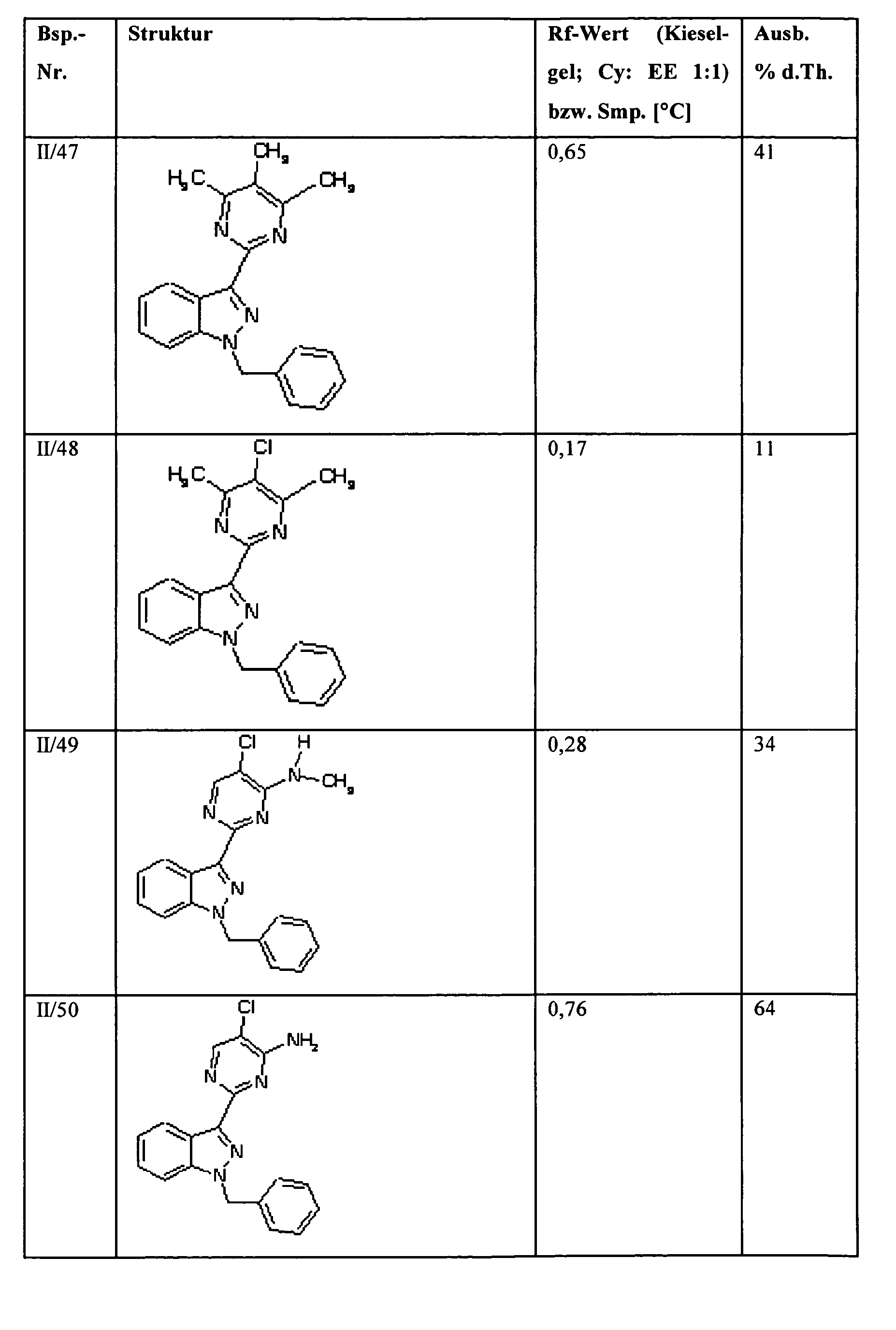

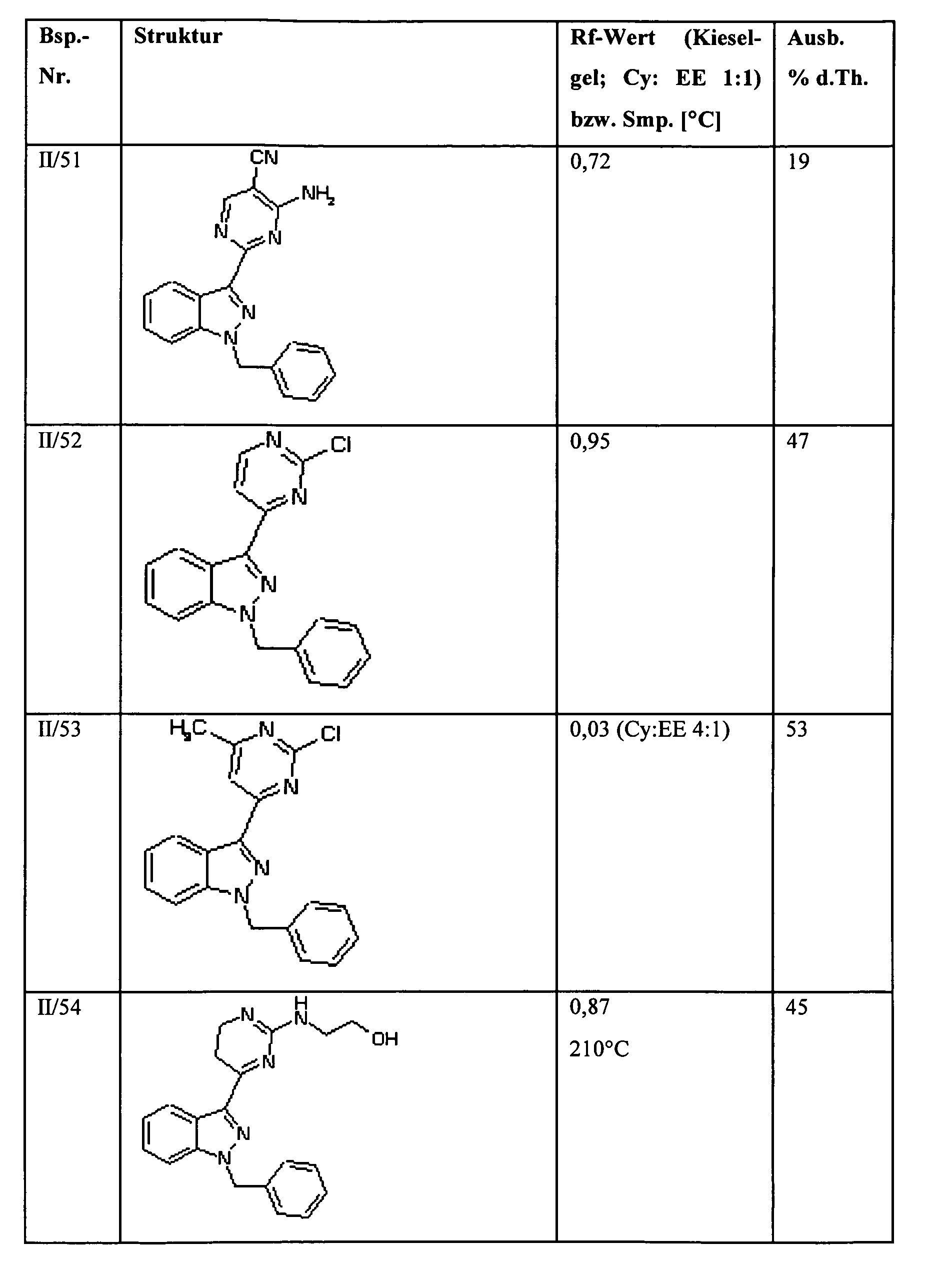

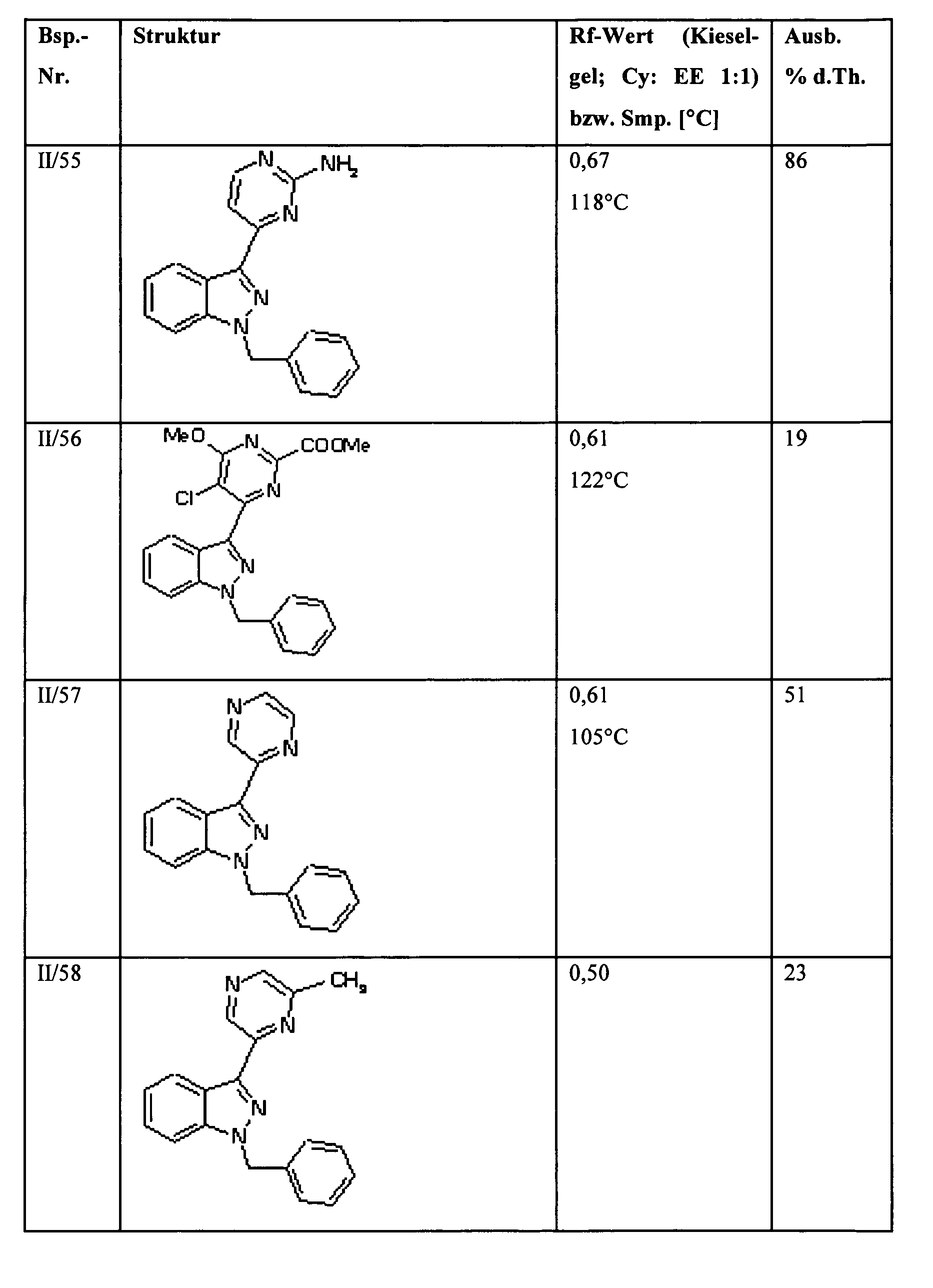

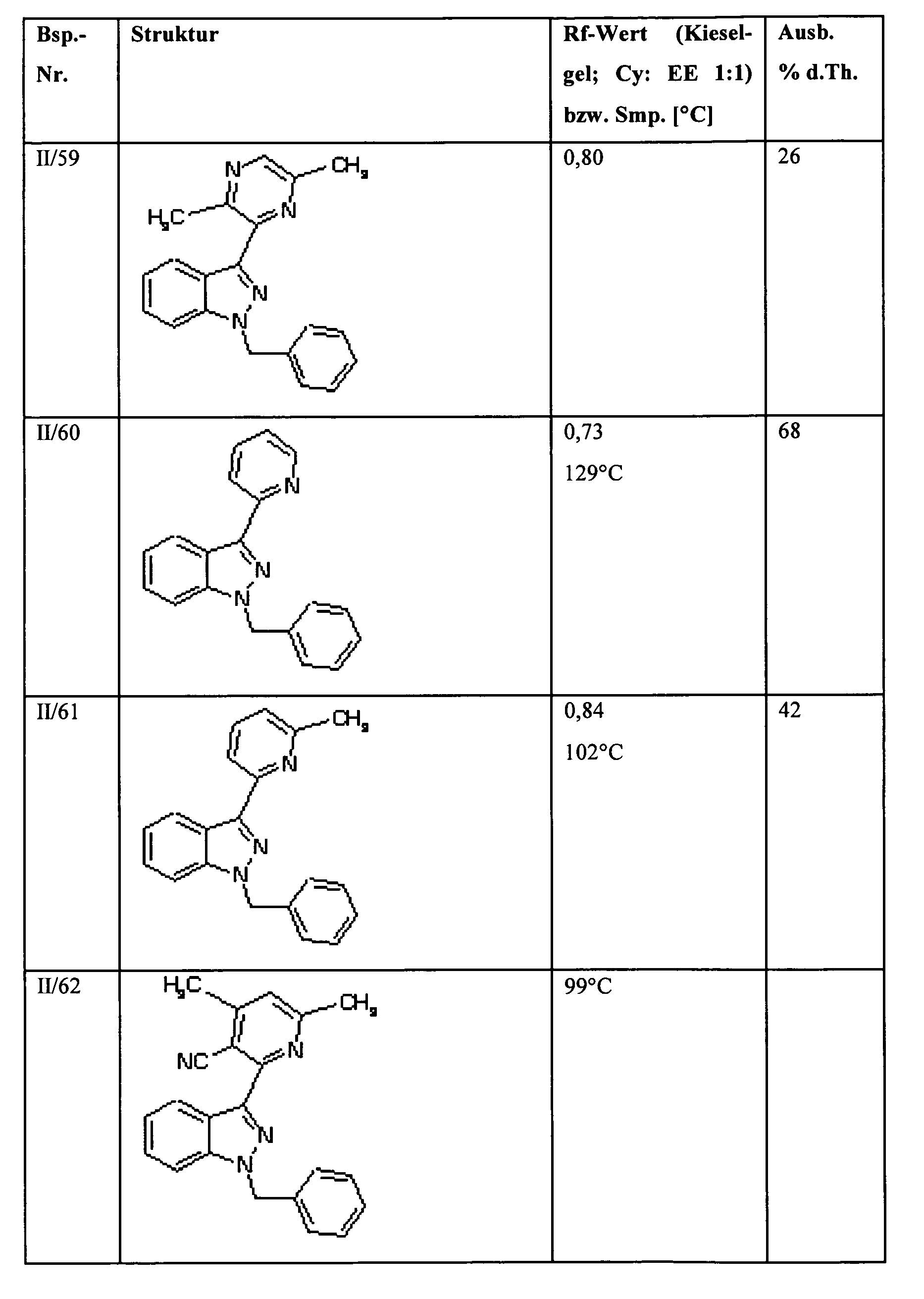

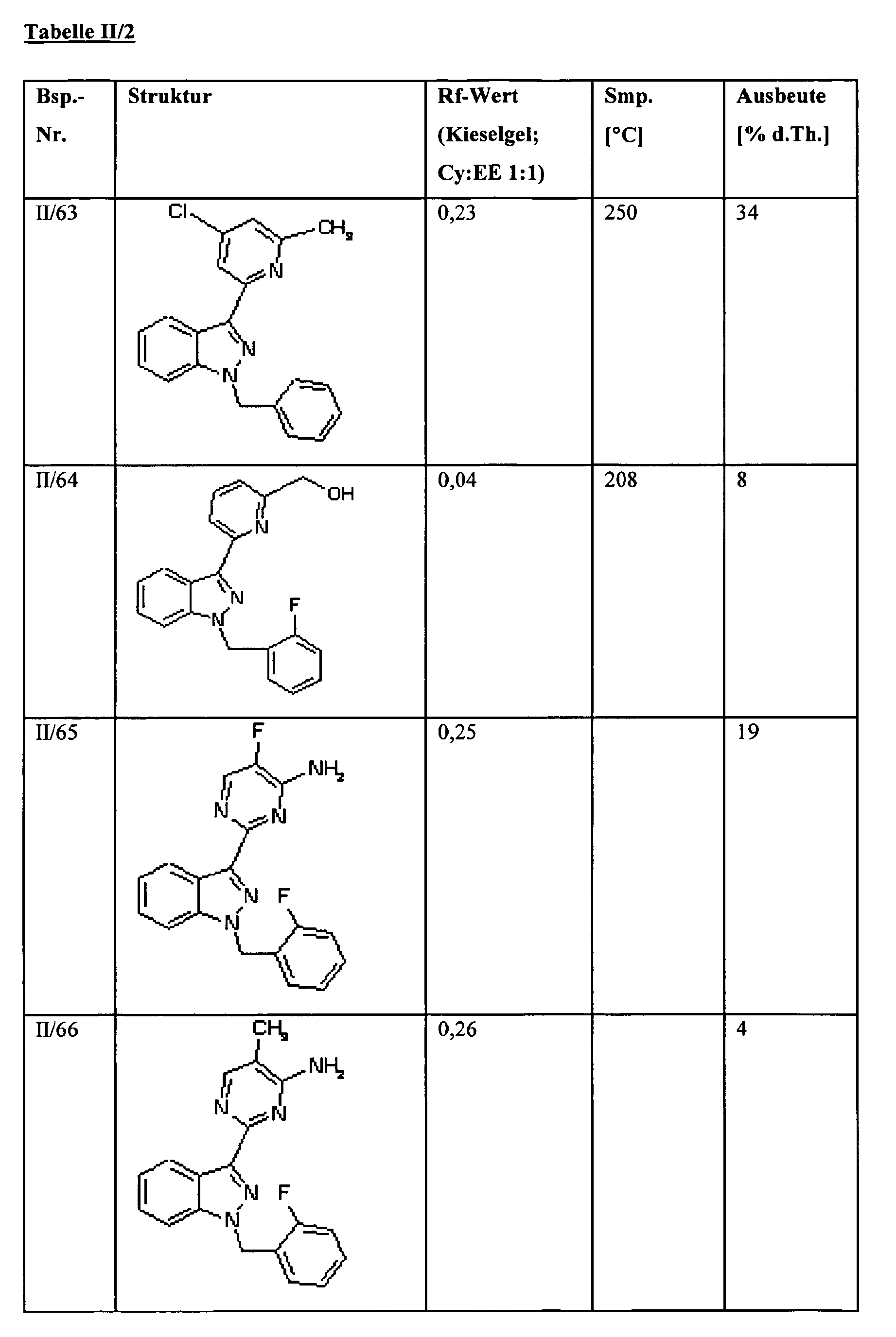

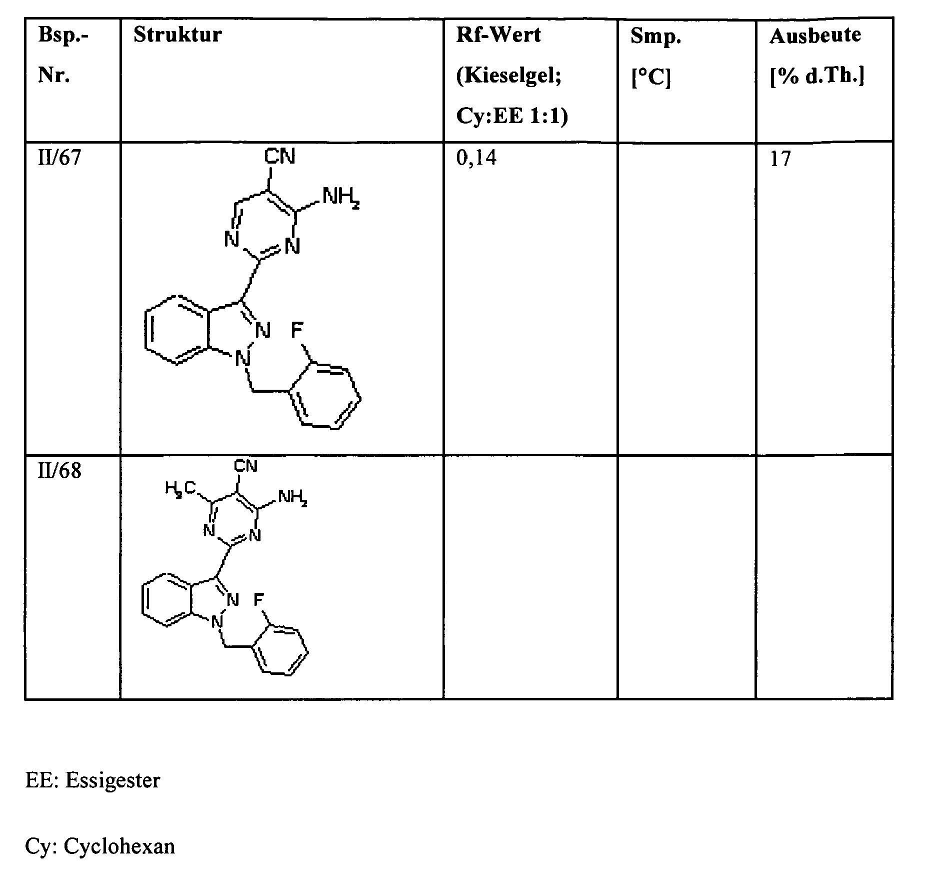

### Beispiele des erfindungsgemäßen Verfahrens zur Herstellung der Oxazolylverbindungen der allgemeinen Formel III-XXIX sowie Verbindungen, in denen R⁴² einen Rest der Formel III-XXVIII darstellt:

In den folgenden experimentellen Beschreibungen sind die Retentionsfaktoren R_{f} auf Kieselgel-DC gemessen, wenn nicht anders angegeben. H = Hexan, EE = Essigester.

### Verfahrensbeispiel 1

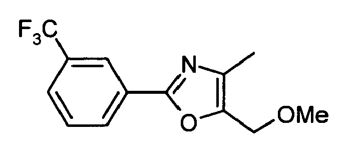

Eine Mischung von 10 g 3-(m-Trifluormethylbenzoylamido)-1,1-dichlor-but-1-en, 5,1 g Natriummethylat und 35 ml Dimethylacetamid wird über Nacht bei 25°C gerührt, dann mit 50 ml Wasser versetzt und mehrfach mit Dichlormethan extrahiert. Die Dichlormethanphase wird abgetrennt, mit Natriumsulfat getrocknet, filtriert und das Lösungsmittel im Vakuumrotationsverdampfer entfernt. Die Destillation des erhaltenen Rohprodukts ergibt 7,3 g 4-Methyl-5-methoxymethyl-2-(m-trifluorphenyl)-oxazol (Siedebereich 96-100°C / 0,2 mbar).

### Herstellung des eingesetzten 1,1-Dichlor-3-(m-trifluormethylbenzoylamido)-but-1-en:

### 1. Stufe

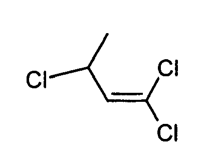

Eine Mischung aus 615 g 1,1,1,3-Tetrachlorbutan, erhalten durch radikalisch initiierte Addition von Tetrachlorkohlenstoff an Propen, 13,3 g Tetrabutylammoniumbromid und einer Lösung von 138,2 g Natriumhydroxid in 390 ml Wasser wird bei Raumtemperatur 24 h durch Rühren gut vermischt. Dann werden 21 Wasser zugegeben, die Phasen getrennt und die organische Phase mit Natriumsulfat getrocknet. Durch Destillation des Rohproduktes erhält man ein Gemisch von 1,1,3-Trichlor-but-1-en und 1,1,1-Trichlor-but-2-en, (492 g vom Siedebereich 45-50°C / 20 mbar).

### 2. Stufe

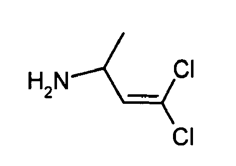

210 g eines Gemisches von 1,1,3-Trichlor-but-1-en und 1,1,1-Trichlor-but-2-en und 52 ml wasserfreie Blausäure werden gleichzeitig innerhalb 1 Stunde zu einer auf 40°C erwärmten Lösung von 38 g H₂O in 568 g konzentrierter Schwefelsäure unter Rühren zudosiert. Dann werden innerhalb von 2 Stunden weitere 78 ml Blausäure hinzugegeben. Nach weiteren 2 Stunden Reaktionszeit wird die überschüssige Blausäure abdestilliert. Mit 20%iger Natronlauge wird das Reaktionsgemisch alkalisch gestellt und das Rohprodukt (195 g) durch Extraktion mit Dichlormethan abgetrennt.

Dieses Rohprodukt wird unter Rühren mit 950 ml halbkonzentrierter Salzsäure vermischt, wobei unter Rückflusskühlung zum Sieden erhitzt wird. Nach 24 Stunden wird abgekühlt, eine geringe Menge an Nebenprodukten durch Extraktion mit Dichlormethan entfernt. Die wässrige Phase wird im Rotationsverdampfer zur Trockene eingeengt. Dann wird mit halbkonzentrierter Natronlauge versetzt und verrührt, wobei der pH-Wert auf 9 gestellt wird. Das sich abscheidende Amin wird isoliert und destilliert. Man erhält 156 g 3-Amino-1,1-dichlor-but-1-en, Kp 45-50°C / 18 mbar.

### 3. Stufe

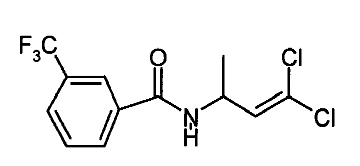

Zu einer Mischung von 21,0 g 3-Amino-1,1-dichlor-but-1-en, 35 ml Dichlormethan und einer Lösung von 15,9 g Natriumcarbonat in 45 ml Wasser wird unter Eiskühlung und kräftigem Rühren eine Lösung von 26,9 g m-Trifluormethylbenzoylchlorid in 25 ml Dichlormethan innerhalb von 30 Minuten zugetropft. Nach einer weiteren Stunde Reaktionszeit werden die Phasen getrennt. Durch Einengen der organischen Phase erhält man 39,0 g 2,2-Dichlor-3-(m-trifluormethylbenzoylamido)-but-1-en.

### Verfahrensbeispiel 2

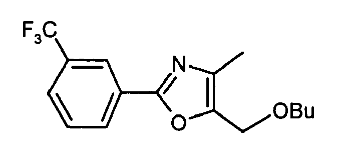

Eine Mischung von 1,1-Dichlor-4-(m-trifluormethylbenzoylamido)-but-1-en, 4,2 g Natriumbutylat und 35 ml Dimethylacetamid wird unter Rühren 7 h auf 100°C erwärmt. Nach der Umsetzung wird analog Beispiel 1 aufgearbeitet. Die Vakuumdestillation des erhaltenen Rohproduktes ergibt 6,4 g 5-Butoxymethyl-4-methyl-2-(m-trifluorphenyl)-oxazol (Siedebereich 106-110°C / 0,2 mbar).

### Verfahrensbeispiel 3

Eine Lösung von 1,8 g 3-Acetamido-1,1-dichlor-but-1-en und 1,0 g Natriummethylat in 20 ml Methanol wird 24 Stunden unter Rückfluss zum Sieden erhitzt. Darauf wird das Methanol abdestilliert, der Rückstand mit 5 ml Wasser und 30 ml Dichlormethan aufgenommen, die organische Phase abgetrennt und im Vakuum destilliert. Man erhält 0,9 g 2,4-Dimethyl-5-methoxymethyloxazol, Kp 54°C / 20 mbar.

### Verfahrensbeispiel 4

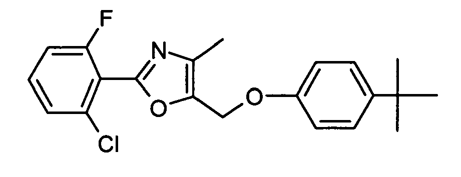

Eine Mischung von 7 g 3-(2-Chlor-6-fluor-benzoylamido)-1,1-dichlor-but-1-en, 11,7 g Natrium-4-tert.butyl-phenolat und 100 ml N-Methylpyrrolidon wird 8 Stunden unter Rühren auf 100°C erwärmt. Anschließend wird das Lösungsmittel durch Vakuumdestillation abgetrennt, der Rückstand mit Wasser und Dichlormethan aufgenommen, die organische Phase abgetrennt, mit Natriumsulfat getrocknet und im Vakuumrotationsverdampfer vom Lösungsmittel befreit. Das Rohprodukt wird chromatographisch gereinigt. Man erhält 5,1 g 2-(2-Chlor-6-fluor-phenyl)-4-methyl-5-(4-tert.butylphenoxymethyl)-oxazol.

### Verfahrensbeispiel 5

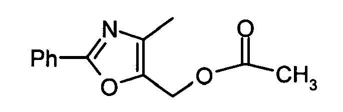

Eine Mischung von 5 g 3-Benzoylamido-1,1-dichlor-but-1-en, 36 g Natriumacetat und 500 ml N-Methylpyrrolidon wird 36 Stunden unter Rühren auf 150°C erwärmt. Anschließend wird analog wie in Beispiel 4 angegeben das Rohprodukt isoliert. Durch Vakuumdestillation erhält man 35 g 5-Acetoxymethyl-4-methyl-2-phenyl-oxazol (Siedebereich 88-91 °C / 0,1 mbar).

### Verfahrensbeispiel 6

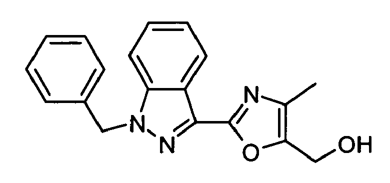

15 mg 3-(1-Benzylindazol-3-carboxamido)-1,1-dichlor-but-1-en (40 µmol) in 20 µl N-Methypyrrolidon unter Argon wurden mit 80 µl NaOH 1M versetzt und die Mischung 1 h auf 55°C erhitzt. Die Mischung wurde abgekühlt und mit 0,8 ml Wasser und 8 ml Essigester versetzt. Die organische Phase wurde eingeengt und der Rückstand durch präparative DC (SiO₂) gereinigt. 9,9 mg (77 %) 2-(1-Benzylindazol-3-yl)-5-hydroxymethyl-4-methyl-oxazol wurden erhalten (mp. 127-129°C).
MS (DCl/NH₃): 320 (100, MH⁺) R_{f}: 0,17 (H:EE 1:1).

### Verfahrensbeispiel 7

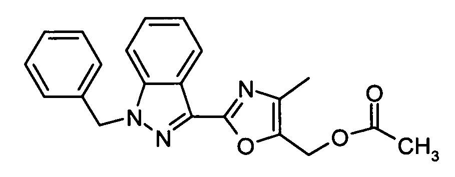

(Für dieses Beispiel wurde die Amid-Zwischenstufe in situ aus der Formel 3a und einem Säurechlorid hergestellt.)

500 mg 1-Benzylindazol-3-carboxylchlorid (1,847 mmol), 260 mg 3-Amino-1,1-dichlor-but-1-en (1,847 mmol) und 318 mg Natriumacetat (3,88 mmol) wurden in 4 ml N-Methylpyrrolidon unter Argon 5 Tage bei 150°C gerührt. Die Rohmischung wurde abgekühlt, mit Wasser versetzt und mehrmals mit Essigester extrahiert. Die organischen Phasen wurden getrocknet (auf Na₂SO₄ und eingeengt, und der Rückstand chromatographiert (SiO₂ Petrolether-Essigester 3:1). Zwei Produkte wurden isoliert: 1,1-Dichlor-3-(1-benzylindazol-3-carboxamido)-but-1-en (410 mg, 59 %) R_{f}: 0,26 (H:EE 3:1) und 5-Acetoxymethyl-2-(1-benzylindazol-3-yl)-4-methyloxazol (160 mg, 24 %).

MS (DCl/NH₃): 362 (100, MH⁺). R_{f}: 0,17 (H:EE 3:1).

### Verfahrensbeispiel 8

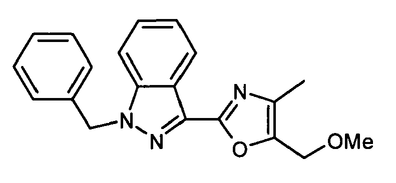

100 mg 3-(1-Benzylindazol-3-carboxamido)-1,1-dichlor-but-1-en (0,267 mmol) und 29 mg Natriummethylat wurden in 0,5 ml N-Methylpyrrolidon unter Argon über Nacht bei 100°C gerührt. Die Rohmischung wurde abgekühlt und direkt durch Säulenchromatographie gereinigt (SiO₂, Cyclohexan:Essigester 3:1). Isoliert wurden 35,9 mg (40 %) 2-(1-Benzylindazol-3-yl)-5-methoxymethyl-4-methyl-oxazol als gelbliches Öl.

MS (DCl/NH₃): 334 (100, MH⁺) R_{f}: 0,67 (CH₂Cl₂:MeOH 100:5).

### Verfahrensbeispiel 9

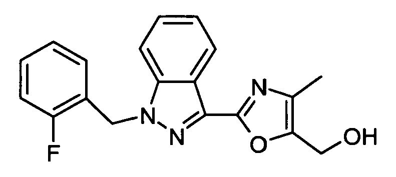

730 mg 1,1-Dichlor-3-[1-(2-fluorbenzyl)indazol-3-carboxamido]-but-1-en (1,86 mmol) und 3,75 ml NaOH 1N (3,75 mmol) wurden in 7,4 ml N-Methylpyrrolidon unter Argen über Nacht bei 50°C gerührt. Nach dem Abkühlen wurde die Mischung auf Eiswasser gegossen und das ausgefallene Produkt abfiltriert und getrocknet. Schließlich wurde es durch Säulenchromatographie (SiO₂ Cyclohexan:Essigester 2:1) gereinigt. Erhalten wurden 375 mg (60 %) 2-[1-(2-Fluorbenzyl)indazol-3-yl]-5-hydroxymethyl4-methyl-oxazol als weiße Kristalle.
Mp. 144°C.
MS (ESI-POSITIV): 338 (100, MH⁺). R_{f}: 0,20 (H:EE 1:1).

Das eingesetzte 1,1-Dichlor-3-[1-(2-fluorbenzyl)indazol-3-carboxamido]-but-1-en wurde folgenderweise hergestellt: 400 mg 1-(2-Fluorobenzyl)-indazol-3-carboxylchlorid (1,385 mmol) und 200 mg 3-Amino-1,1-dichlor-but-1-en in 1,5 ml THF wurden mit 120 µl Pyridin versetzt und 3 h bei RT gerührt. Anschließend wurde mit Essigester und Wasser versetzt. Die organische Phase wurde auf Natriumsulfat getrocknet und eingeengt. Das Rohprodukt 1,1-Dichlor-3-[1-(2-fluorbenzyl)indazol-3-carboxamido]-but-1-en ist laut DC sauber genug, um direkt weiter umgesetzt zu werden.
R_{f}: 0,32 (H:EE 3:1).

### Verfahrensbeispiel 10

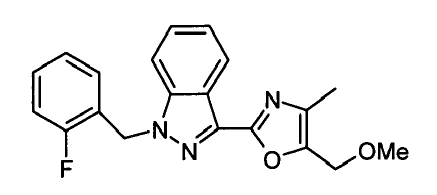

136 mg 1,1-Dichlor-3-[1-(2-fluorbenzyl)indazol-3-carboxamido]-but-1-en (0,267 mmol) und 47 mg Natriummethylat wurden in 0,6 ml N-Methylpyrrolidon unter Argon über Nacht bei 100°C gerührt. Die Rohmischung wurde abgekühlt und direkt durch Säulenchromatographie gereinigt (SiO₂). Isoliert wurden 24 mg (20 %) 2-[1-(2-Fluorobenzyl)-indazol-3-yl]-5-methoxymethyl-4-methyl-oxazol als gelbliche Kristalle.

Mp. 86-88°C. MS (ESI-POSITIV): 374 (65, M+Na⁺), 352 (100, MH⁺). R_{f}: 0,18 (CH₂Cl₂:MeOH 100:1).

### Verfahrensbeispiel 11

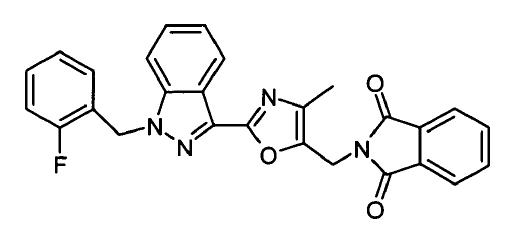

136 mg 1,1-Dichlor-3-[1-(2-fluorbenzyl)indazol-3-carboxamido]-but-1-en (0,267 mmol) und 164 mg Kaliumphthalimid wurden in 0,6 ml N-Methylpyrrolidon unter Argon über Nacht bei 150°C gerührt. Anschließend wurde das N-Methylpyrrolidon im Hochvakuum bei 60°C abgezogen und der Rückstand chromatographiert (SiO₂ Cyclohexan/Essigester 2:1), um 48,5mg (36 %) 2-[1-(2-Fluorobenzyl)-indazol-3-yl]-4-methyl-5-(N-phthalimidomethyl)-oxazol zu erhalten. Gelbliche Kristalle.

Mp. 175-177°C. MS (ESI-POSITIV): 467 (100, MH⁺). R_{f}: 0,64 (CH₂Cl₂:MeOH 100:1).

### Verfahrensbeispiel 12

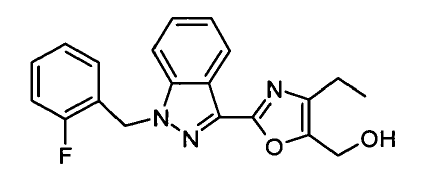

1,05 g 1,1-Dichlor-3-[1-(2-fluorbenzyl)indazol-3-carboxamido]-pent-1-en (2,58 mmol) und 5,20 ml NaOH 1N (5,20 mmol) wurden in 15 ml N-Methylpyrrolidon unter Argon 2 Tage bei 50°C gerührt. Nach dem Abkühlen wurde die Mischung auf Eiswasser gegossen und die Mischung mehrmals mit Essigester extrahiert. Die organische Phase wurde getrocknet (Natriumsulfat) und eingeengt (N-Methylpyrrolidon im Hochvakuum abgezogen). Der Rückstand wurde durch Säulenchromatographie (Aluminiumoxid, Cyclohexan/Essigester 2:1) gereinigt. Erhalten wurden 470 mg (52 %) 5-Ethyl-2-[1-(2-fluorobenzyl)-indazol-3-yl]-5-hydroxymethyl-oxazol als weiße Kristalle.

Mp. 137-139°C. MS (ESI-POSITIV): 352 (100, MH⁺). R_{f}: 0,08 (Aluminiumoxid, Cyclohexan:EE 2:1).

### Herstellungsbeispiele zur Ausführungsform IV der Erfindung

### Beispiel IV/134

### 1-(2-Cyanobenzyl)-3-(5-(1,3-dioxolan-2-yl)furan-2-yl)-indazol

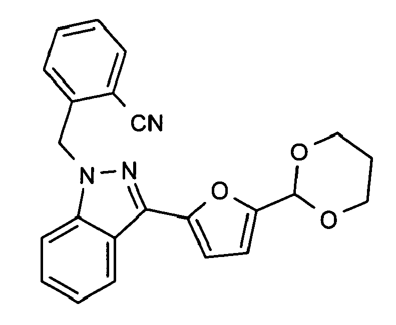

Man gibt zu einer Suspension von 355 mg NaH (60-proz. in Paraffin) in 10 ml DMF unter Argon eine Lösung von 2 g (7.41 mmol) 3-(5-(1,3-Dioxolan-2-yl)-furan-2-yl)indazol in 10 ml DMF und rührt eine Stunde bei Raumtemperatur. Anschließend gibt man 1,5 g 2-Cyanobenzylbromid hinzu. Man rührt 30 Minuten bei 100°C, gibt in Wasser, extrahiert mit Essigester, trocknet die organische Phase mit Natriumsulfat, verdampft im Vakuum und chromatographiert den Rückstand auf Kieselgel mit Toluol/Essigestergemischen als Eluens.
Man erhält 2,1 g ( 73,5% d. Th.) eines Öls.
R_{f}(SiO2, Toluol/Essigester 1:1): 0.63

### Beispiel IV/135

### 1-(2-Cyanobenzyl)-3-(5-formyl-2-furanyl)-indazol

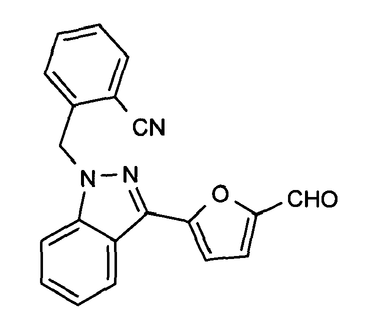

Man löst 2,1 g (5,5 mmol) von 1-(2-Cyanobenzyl)-3-(5-(1,3-dioxolan-2-yl)furan-2-yl)-indazol in 20 ml Aceton und gibt 40 ml 50-proz. Essigsäure hinzu. Man kocht eine Stunde, gibt in Wasser, extrahiert mit Essigester, wäscht die organische Phase mit NaHCO₃-Lösung, trocknet sie mit Natriumsulfat, verdampft im Vakuum und erhält 1,61 g (89 % d. Th.) eines Feststoffs.
Mp: 137°C
R_{f}(SiO2, Toluol/Essigester 4:1): 0.4

### Beispiel IV/136

### 1-(2-Cyanobenzyl)-3-(5-hydroxymethylfuran-2-yl)-indazol

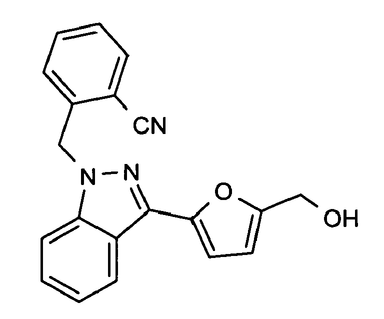

Man suspendiert 0,8 g (2.44 mmol) 1-(2-Cyanobenzyl)-3-(5-formyl-2-furanyl)-indazol in 50 ml Propanol und gibt bei 0°C langsam 0,8 g NaBH4 hinzu. Nach 1 Stunde Rühren bei Raumtemperatur gibt man die klare Lösung in Wasser, extrahiert mit Essigester, trocknet die organische Phase mit Natriumsulfat, verdampft im Vakuum und chromatographiert den Rückstand auf Kieselgel mit Toluol/Essigestergemischen als Eluens.
Man erhält 600 mg (75% d. Th.) Kristalle.
Mp. 147°C
R_{f}(SiO2, Toluol/Essigester 1:1): 0.52

### Beispiel IV/137

### 1-Benzyl-3-[5-(1,3-dioxolan-2-yl)-furan-2-yl]-indazol

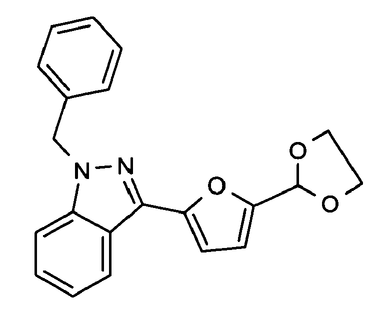

661 mg 5-(1,3-Dioxolan-2-yl)-2-tributylstannyl-furan (1,54 mmol)(M.Yamamoto, H. Izukawa, M. Saiki, K. Yamada, J. Chem. Soc. Chem. Comm. 1988, 560), 431,5 mg 1-Benzyl-3-iodindazol (1,29 mmol) und 90 mg Tetrakistriphenylphosphinpalladium (0.078 mmol) in 2,5 ml DMF unter Argon wurden 10 h bei 80°C erhitzt. Nach Abkühlung wurde die Mischung mit Wasser versetzt und mit Essigester extrahiert. Die organischen Phasen wurden mit Natriumsulfat getrocknet und eingeengt. Nach Chromatographie (SiO₂; Petrolether: Essigester 9:1) erhält man 381,3 mg eines viskösen Öls.
R_{f}: 0.16 (Hexan/Essigester 3:1)

### Beispiel IV/138

### 1-Benzyl-3-(5-formylfuran-2-yl)-indazol

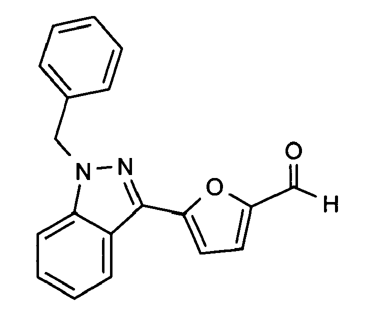

336 mg 1-Benzyl-3-[5-(1,3-dioxolan-2-yl)-furan-2-yl]-indazol (0,97 mmol) wurden mit ein paar Kristalle p-Toluolsulfonsäure in 5 ml Aceton und 0,3 ml Wasser 20 h auf Rückfluss erhitzt. Danach wurde die Reaktionsmischung mit 40 ml Ether versetzt. Die organische Phase wurde mit wenig Natriumchlorid-Lösung gewaschen, auf Natriumsulfat getrocknet und eingeengt. Der Rückstand kristallisiert langsam. Die Kristalle wurden mit wenig Ether gewaschen und in vacuo getrocknet: 210,4 mg.
R_{f}: 0.24 (Hexan/Essigester 3:1)
Mp: 97-99°C

### Beispiel IV/139

### 1-[ 5-(1-Benzylindazol-3-yl)-furan-2-yl]-ethanol

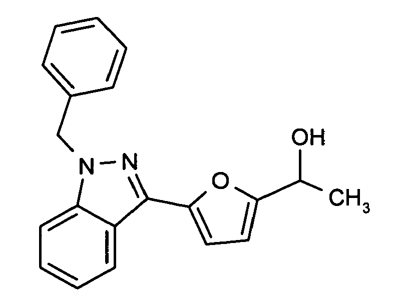

300 l einer MeLi-Lösung (1,6M in Ether; 0,480 mmol) wurden zu einer auf -78°C abgekühlten Lösung von 1-Benzyl-3-(5-formylfuran-2-yl)-indazol (134,1 mg; 0,44 mmol) in 3 ml THF zugetropft. Nach 10 mn wurde die Mischung mit NH₄Claq versetzt und mit Ether extrahiert. Die organische Phase wurde mit einer Natriumchlorid-Lösung gewaschen, auf Natriumsulfat getrocknet und eingeengt. Nach Chromatographie (SiO₂; Petrolether: Essigester 2:1) erhält man 133 mg eines viskösen Öls.
R_{f}: 0,11 (Hexan/Essigester 3: 1)
MS (CI, NH3): 319 (M+H⁺, 100).

### Beispiel IV/140

### 3-(5-Acetylfuran-2-yl)-1-benzyl-indazol

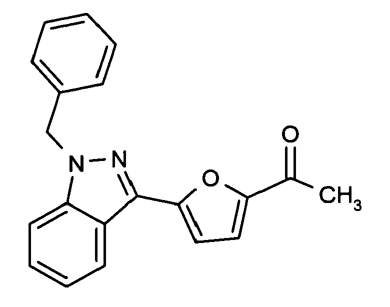

Eine Mischung von 1-[ 5-(1-Benzylindazol-3-yl)-furan-2-yl]-ethanol (51,2 mg; 0,160 mmol) und 250 mg MnO₂ (2,9 mmol) in 2 ml CHCl₃ wurde auf Rückfluß erhitzt. Nach 12 h wurden erneut 250 mg MnO₂ zugegeben. Nach weiteren 12 h wurde die Mischung durch Celite filtriert, das Filtrat eingeengt und der Rückstand chromatographiert (SiO₂; Petrolether: Essigester 3:1). Man erhält 29,8 mg eines hell-gelben Feststoffs.
R_{f}: 0.21 (Hexan/Essigester 3:1)
Mp: 99-100°C

### Beispiel IV/141

### 3-(5-Azidomethylfuran-2-yl)-1-benzyl-indazol

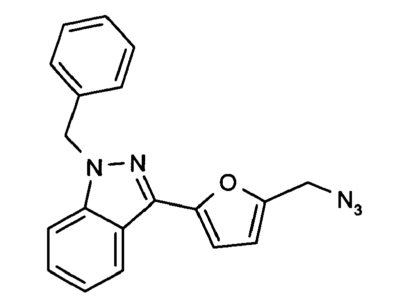

195,1 mg 1-Benzyl-3-(5-hydroxymethylfuran-2-yl)-indazol (0,64 mmol)(Kuo S.-C., Yu Lee F., Teng C.-M. EP 0 667 345 A1) , 252 mg Triphenylphosphin (0,96 mmol) und 60,3 mg Natriumazid (0,93 mmol) wurden in 2,5 ml DMF gelöst. 308,4 mg Tetrabrommethan wurden zugegeben und die resultierende Mischung 20h bei RT rühren lassen. Nach Zugabe von 5 ml Wasser wurde sie mit Essigester extrahiert. Die organische Phase wurde mit einer Natriumchlorid-Lösung gewaschen, auf Natriumsulfat getrocknet und eingeengt. Nach Chromatographie (SiO₂; Cyclohexan: Essigester 2:1) erhält man 206,7 mg eines viskösen Öls, die langsam fest wird.
R_{f}: 0.63 (Hexan/Essigester 1:1)
Mp. 51-52°C.

### Beispiel IV/142

### 3-(5-Aminomethylfuran-2-yl)-1-benzyl-indazol

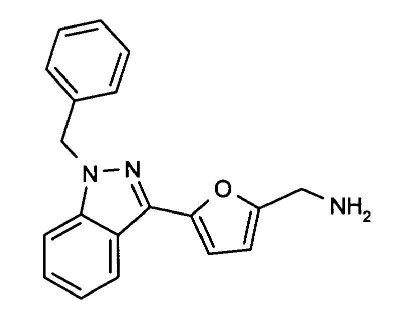

121,5 mg 3-(5-Azidomethylfuran-2-yl)-1-benzyl-indazol (0,369 mmol) und 102 mg Triphenylphosphin (0,389 mmol) wurden in 1 ml THF 3,5 h rühren lassen. 10 µl Wasser wurden dann zugegeben. Nach 24 h wurde die Reaktion mit Ether und HClaq 0.3M versetzt. Der abfiltrierten Feststoff und die wässrige Phase wurden vereinigt und mit NaOH 2M alkalisch gestellt und mit Ether extrahiert. Die organische Phase wurde mit wenig Wasser dann mit einer Natriumchlorid-Lösung gewaschen, auf Natriumsulfat getrocknet und eingeengt. Man erhält 79,9 mg eines gelben Öls.

### Beispiel IV/143

### 1-Benzyl-3-(5-nitrofuran-2-yl)-indazol

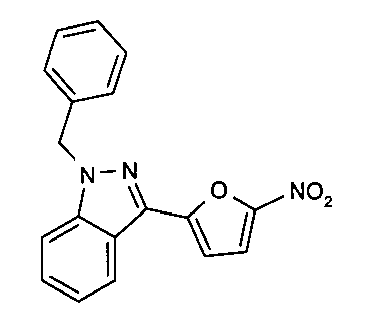

800 mg 5-Nitrofuran-2-yl-phenylketon-benzylhydrazone (Gemisch E+Z, 2,49 mmol) wurden in 35 ml Dichlormethan gelöst. 1,99 g Bleitetraacetat (4,49 mmol) und 15,6 ml BF₃-Etherat (50 % in Ether, 62 mmol) wurden zugegeben und die Mischung 1 h auf Rückfluss erhitzt. Nach dem Abkühlen wurde die Reaktionsmischung auf Eis gegossen, die organische Phase abgetrennt und mit 0.2M NaOH, dann Wasser, gewaschen, über Natriumsulfat getrocknet und eingeengt. Nach Chromatographie erhält man 140 mg gelbe Kristalle.
R_{f}: 0,20 (Petrolether/ Essigester 10:1)
Mp: 148-151°C (Zersetzung)
In Analogie zur Vorschrift der oben aufgeführten Beispiele wurden die in den Tabellen IV/1, IV/2, IV/3, IV/4 und IV/5 erfassten Verbindungen hergestellt.

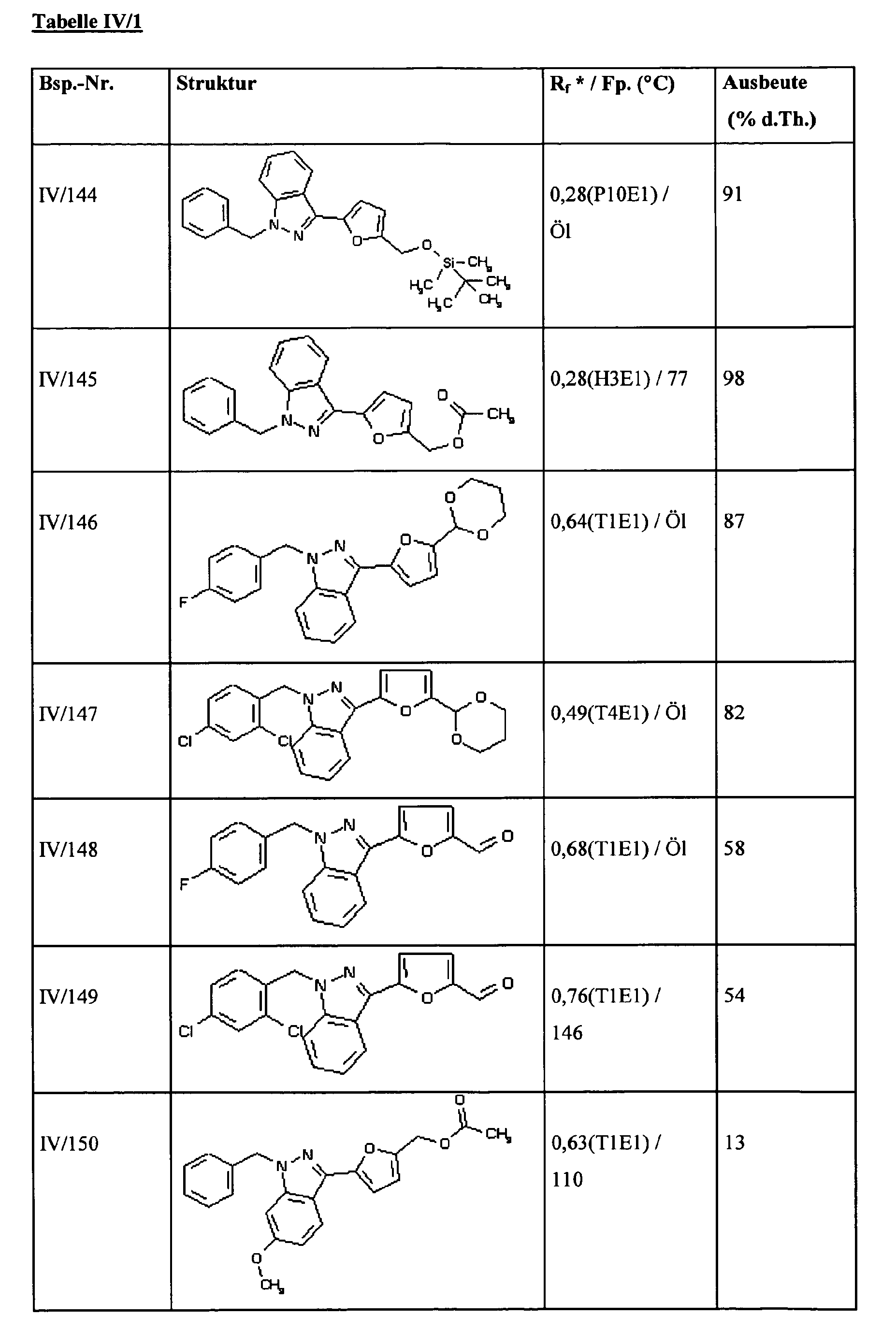

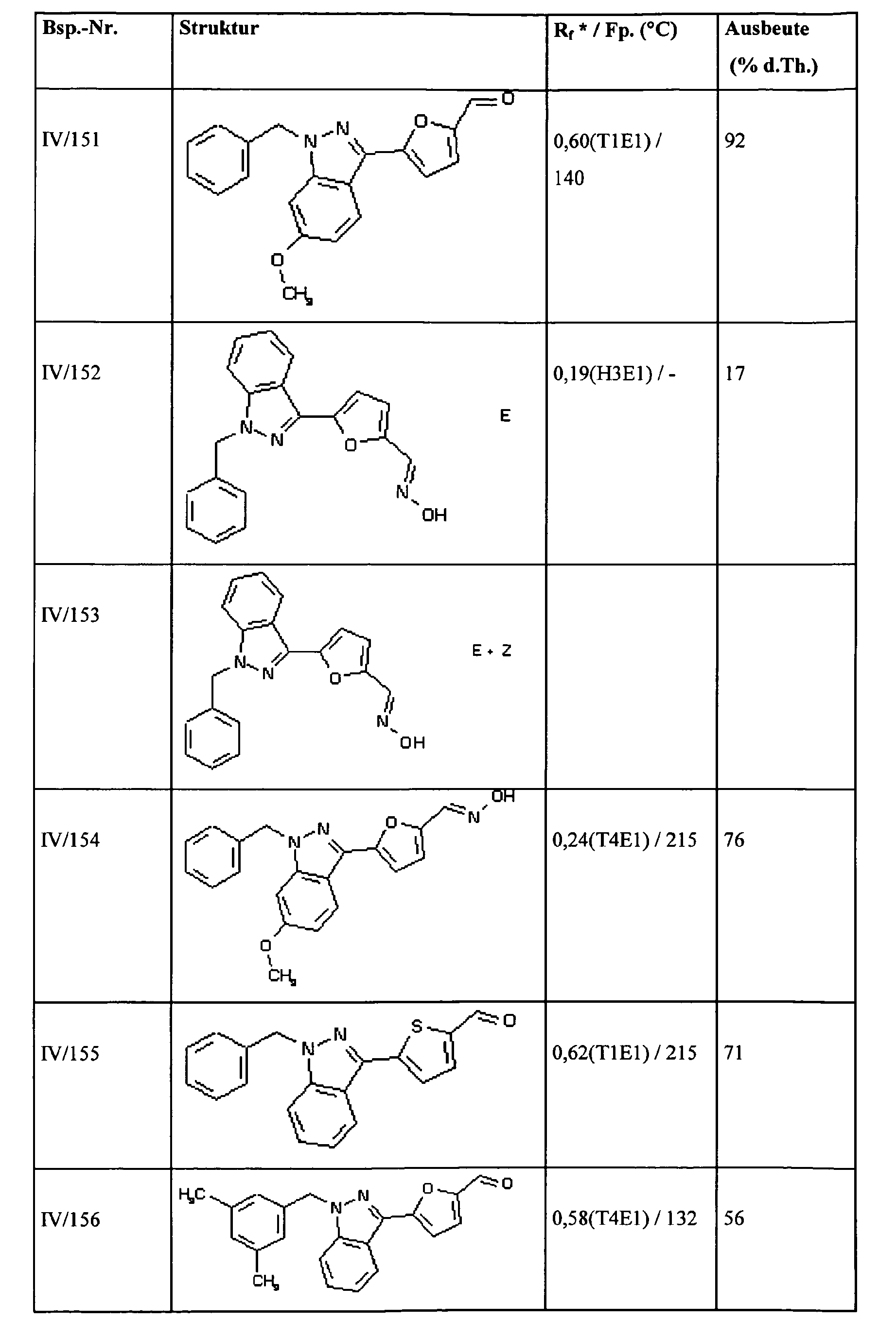

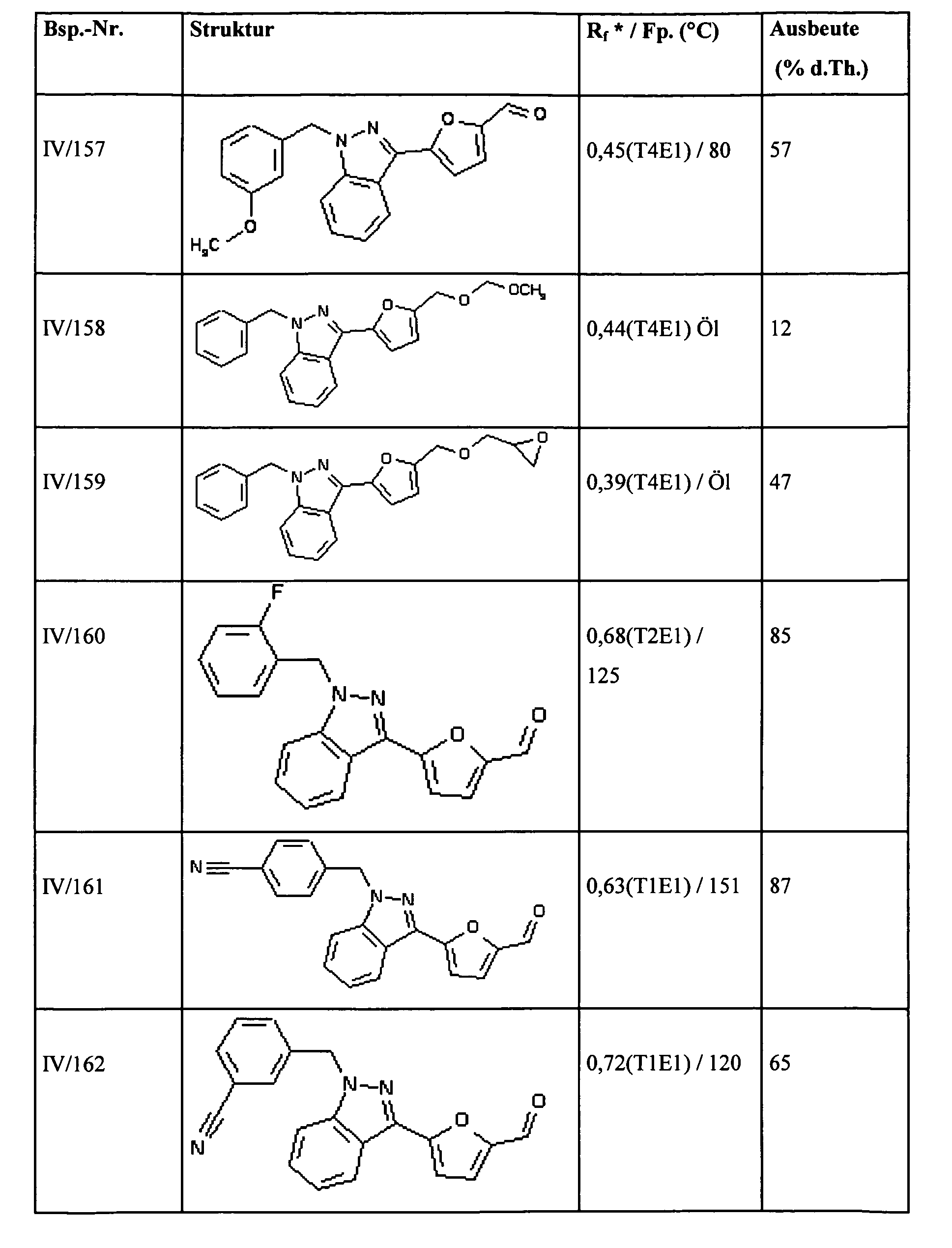

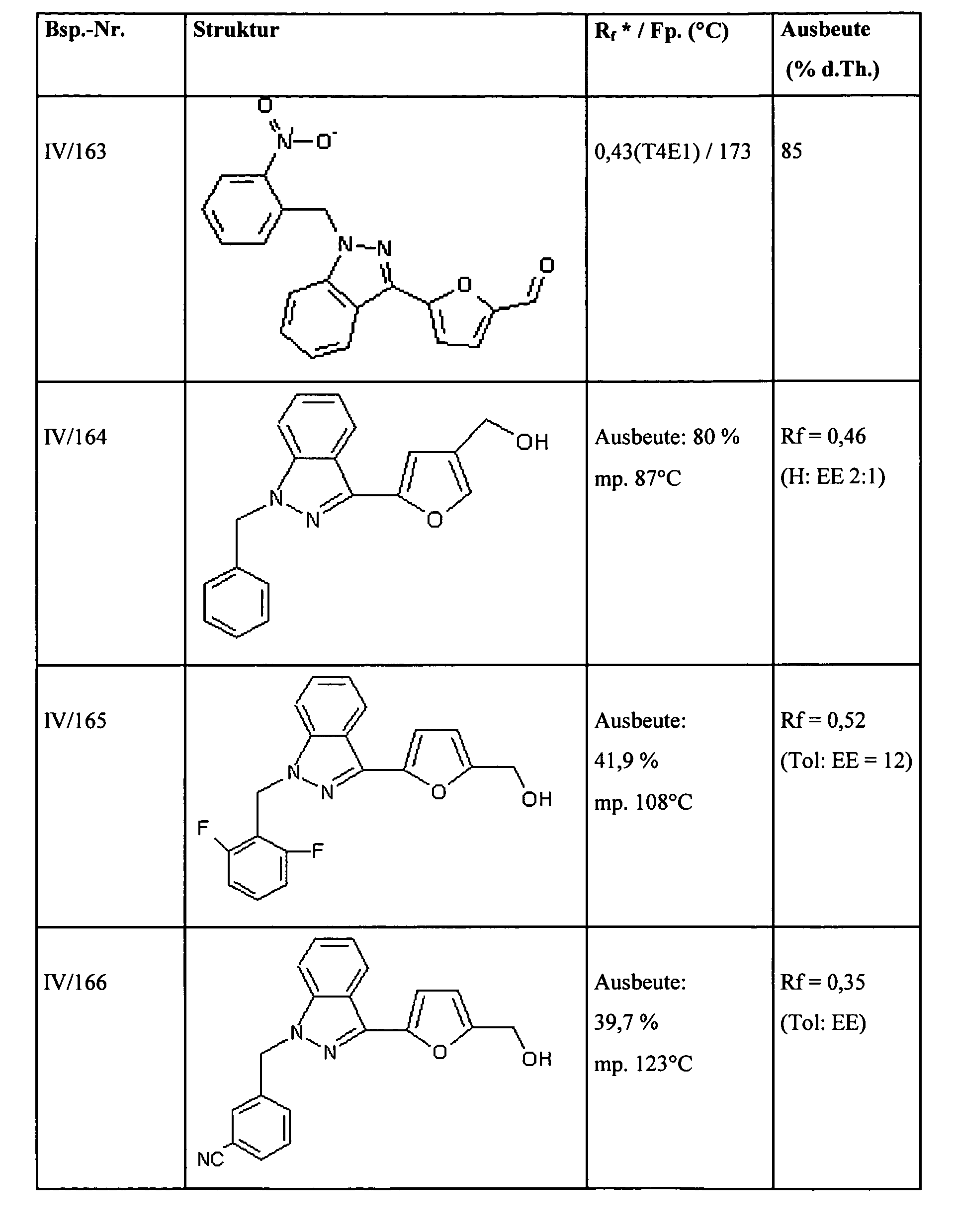

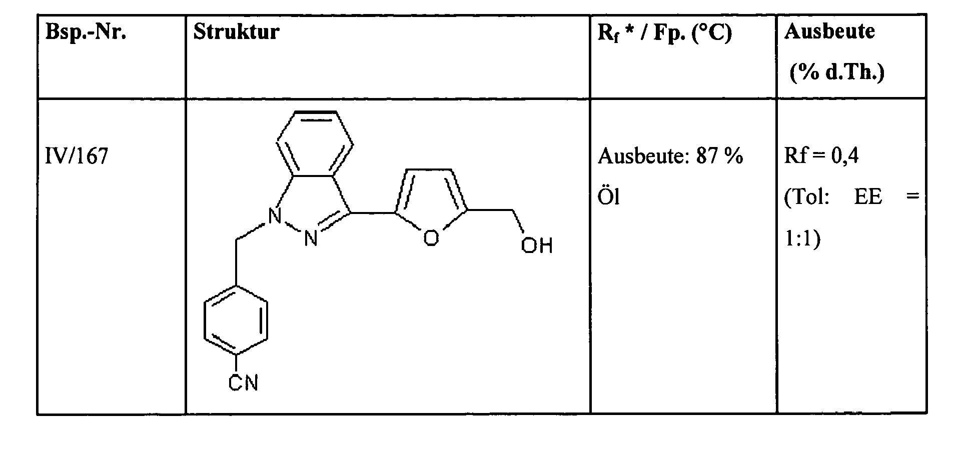

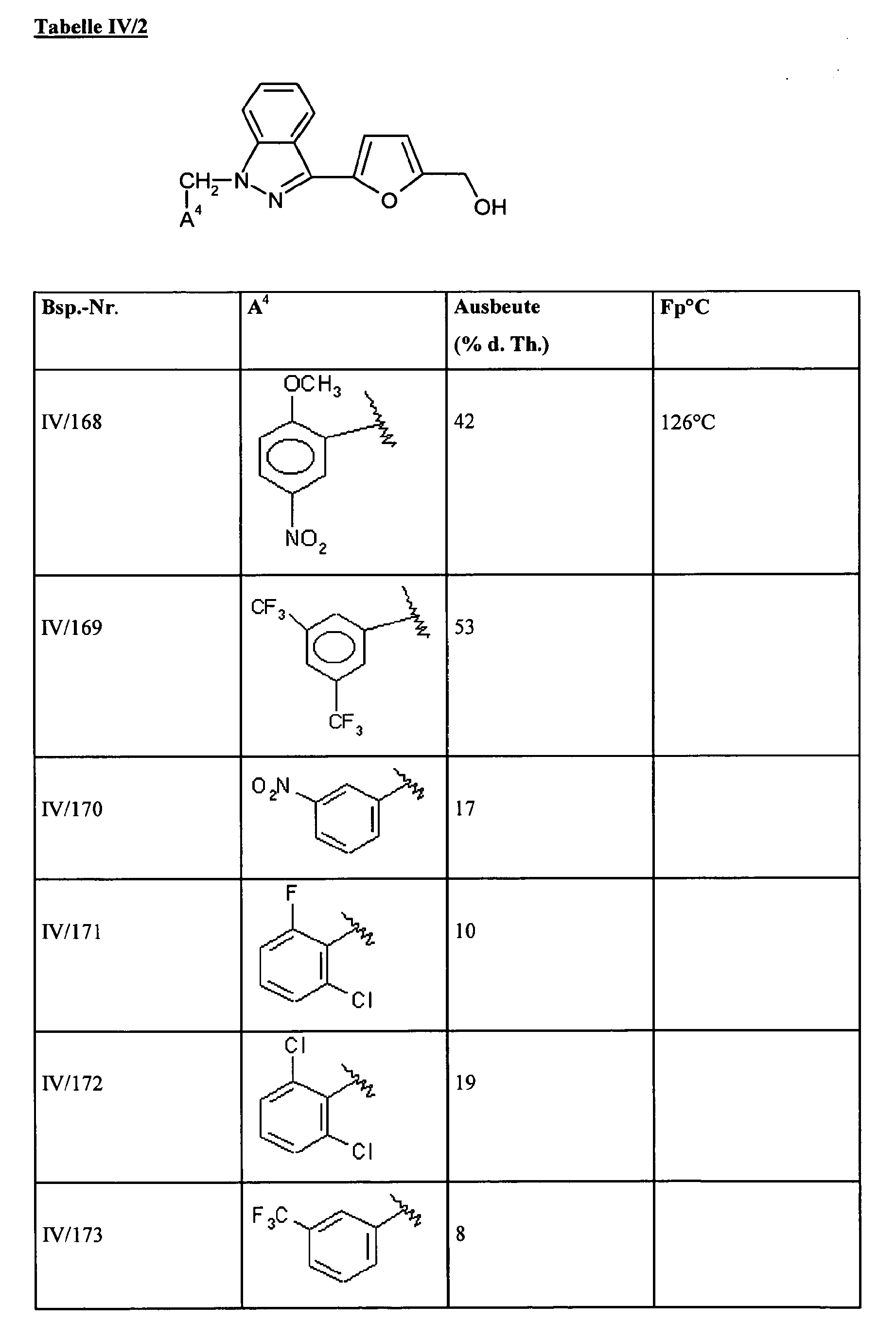

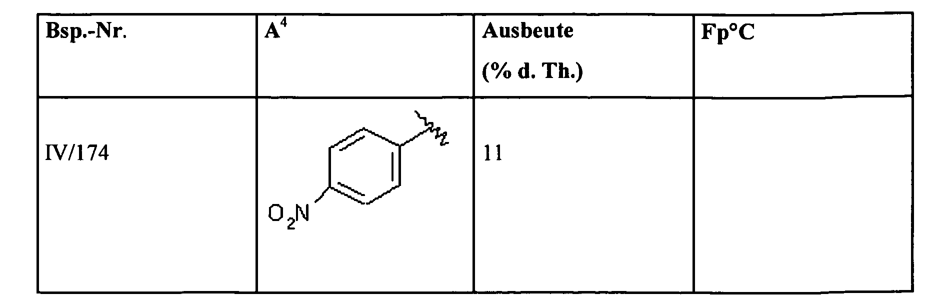

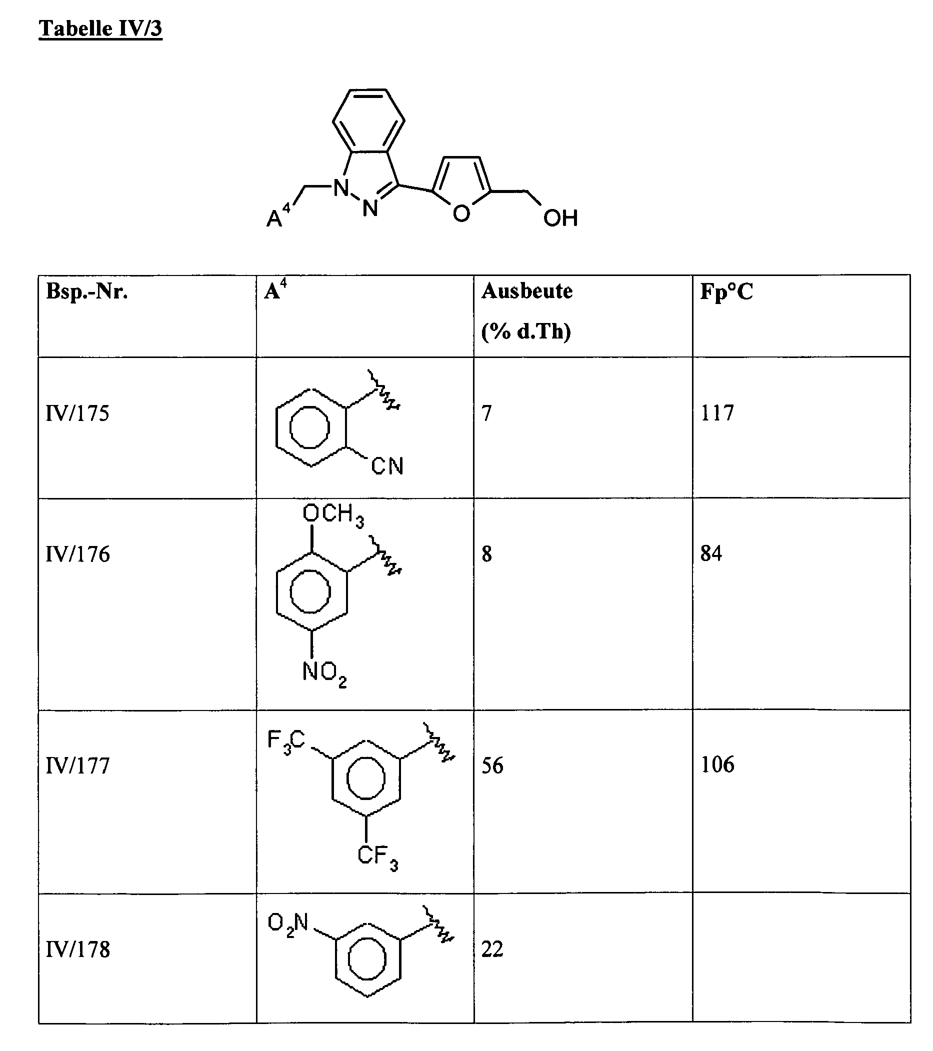

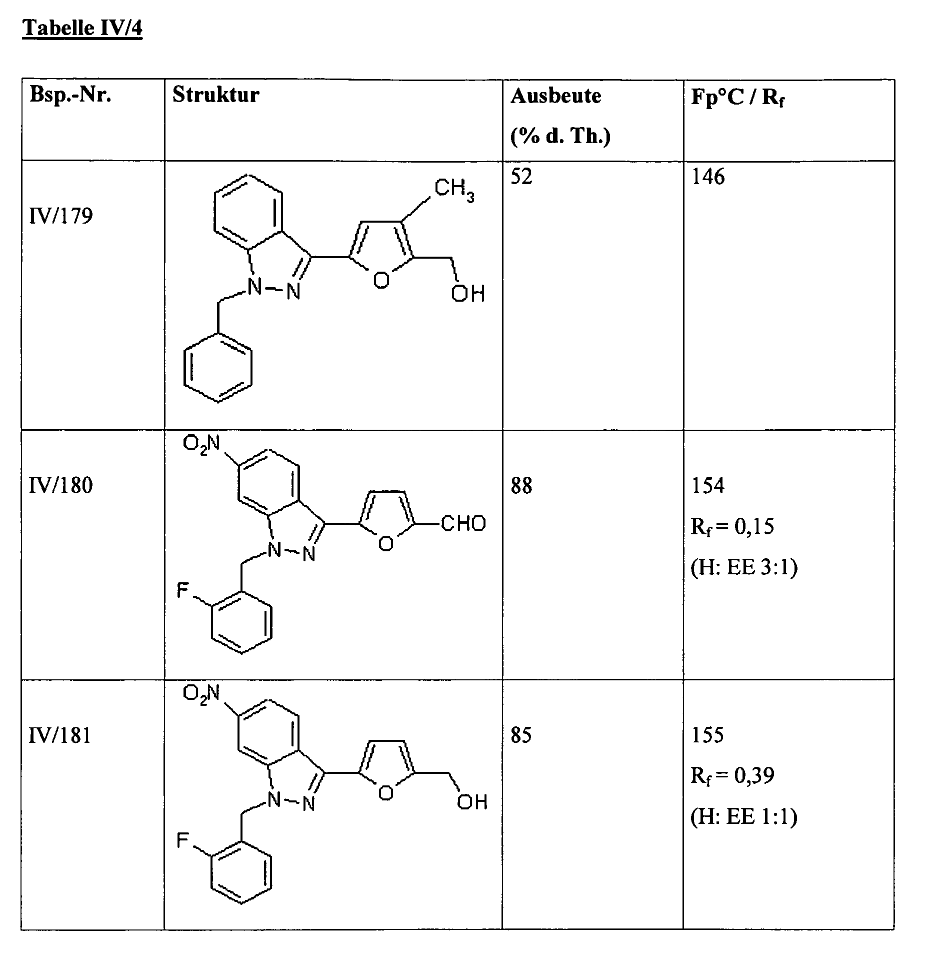

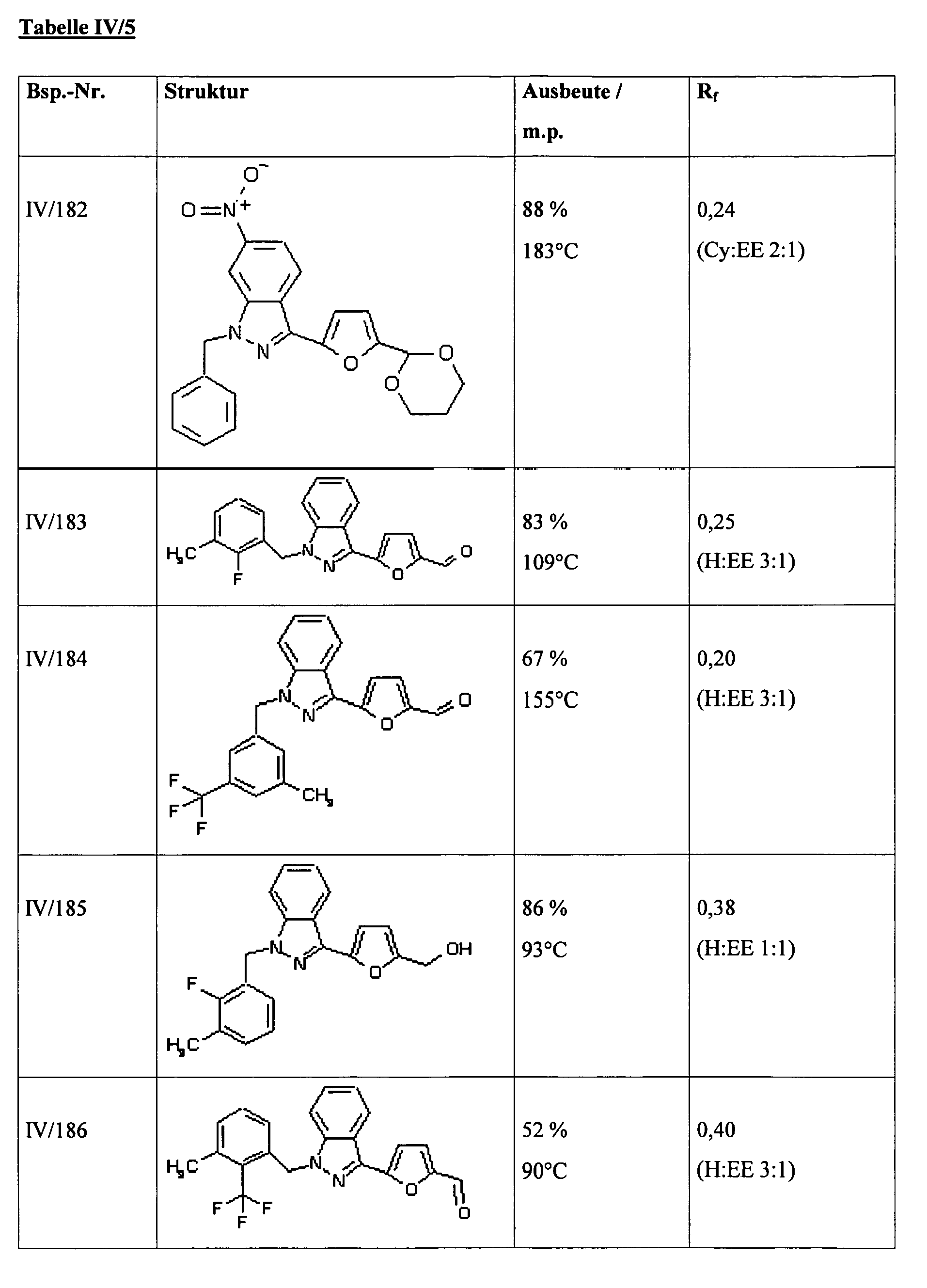

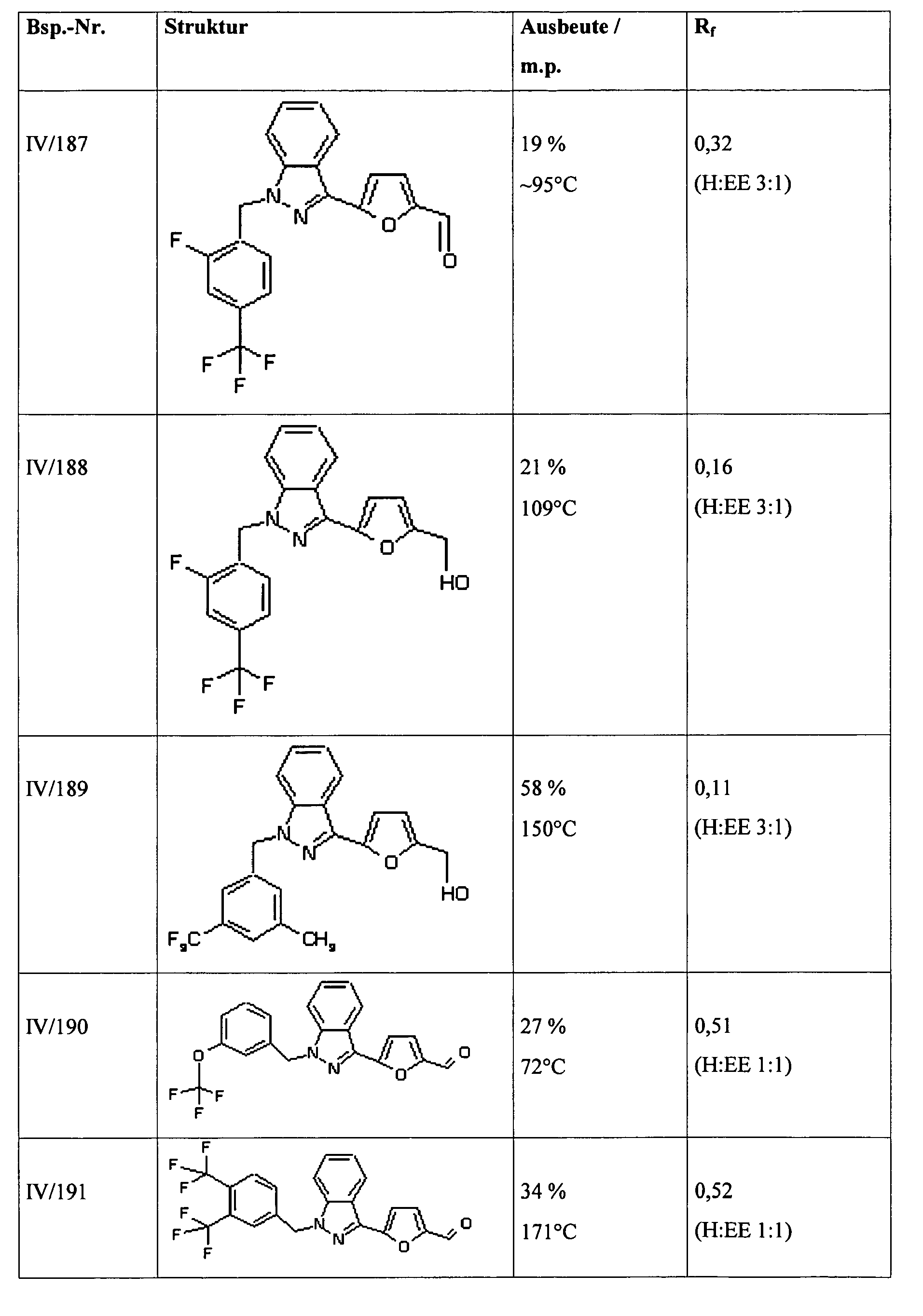

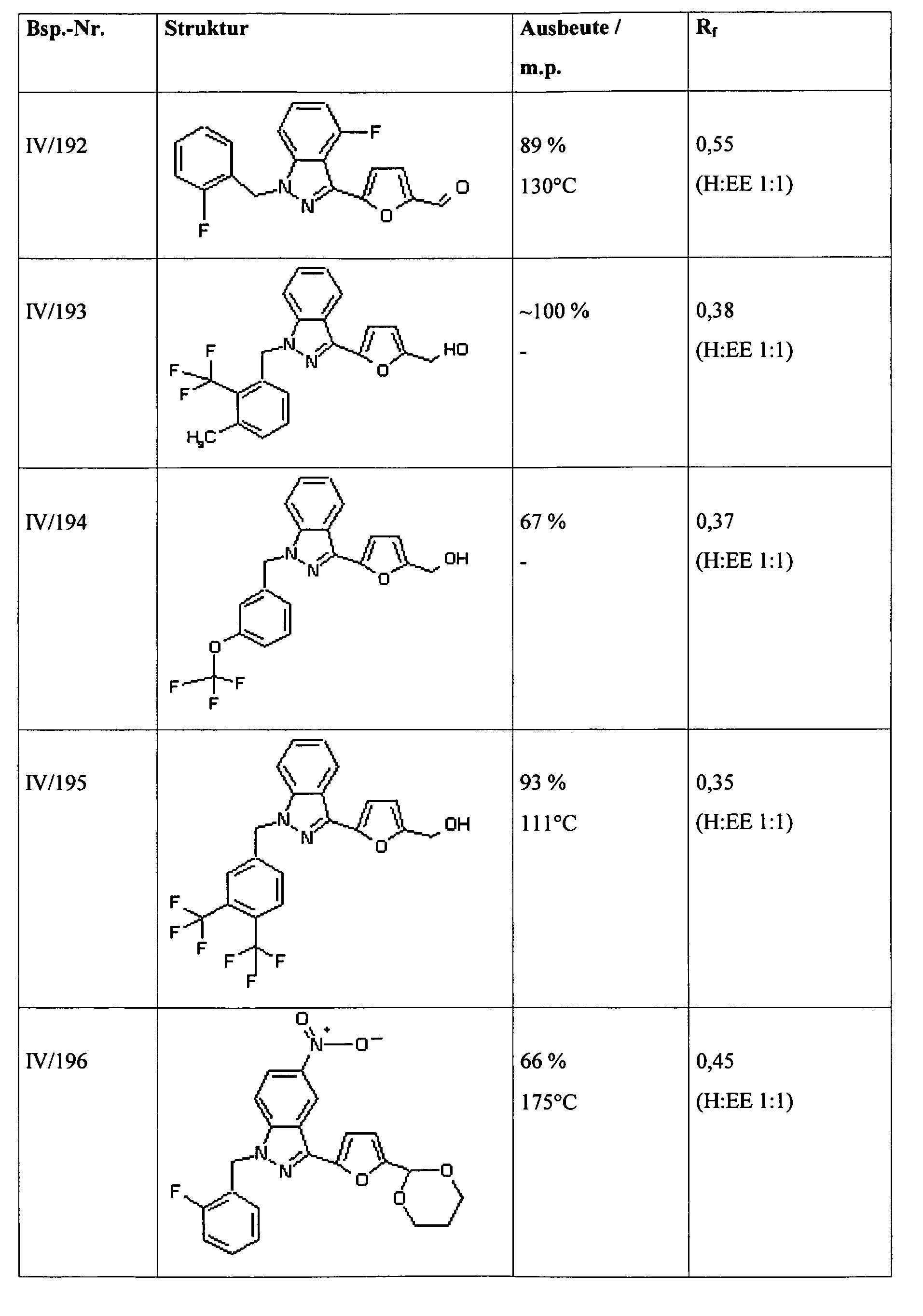

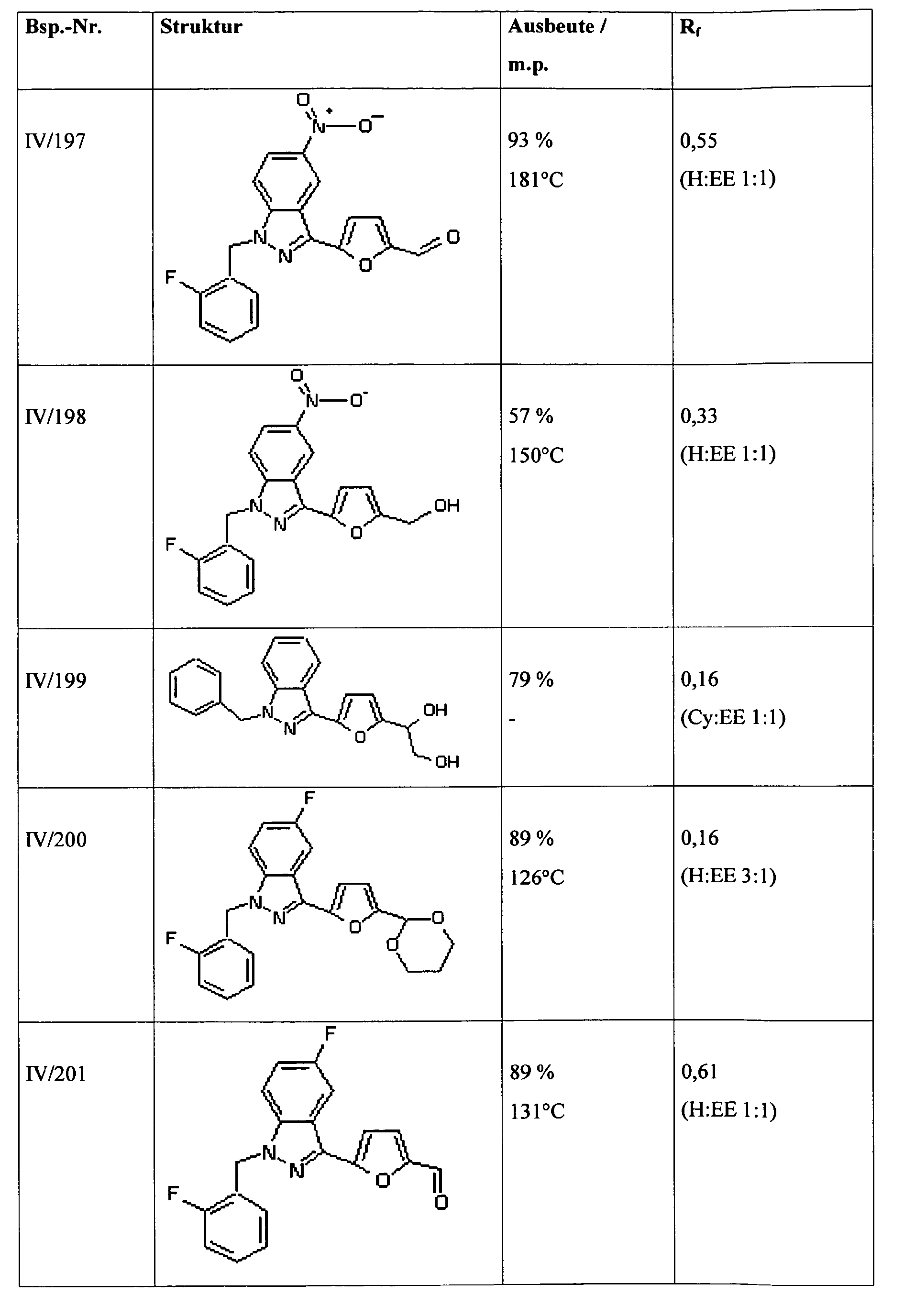

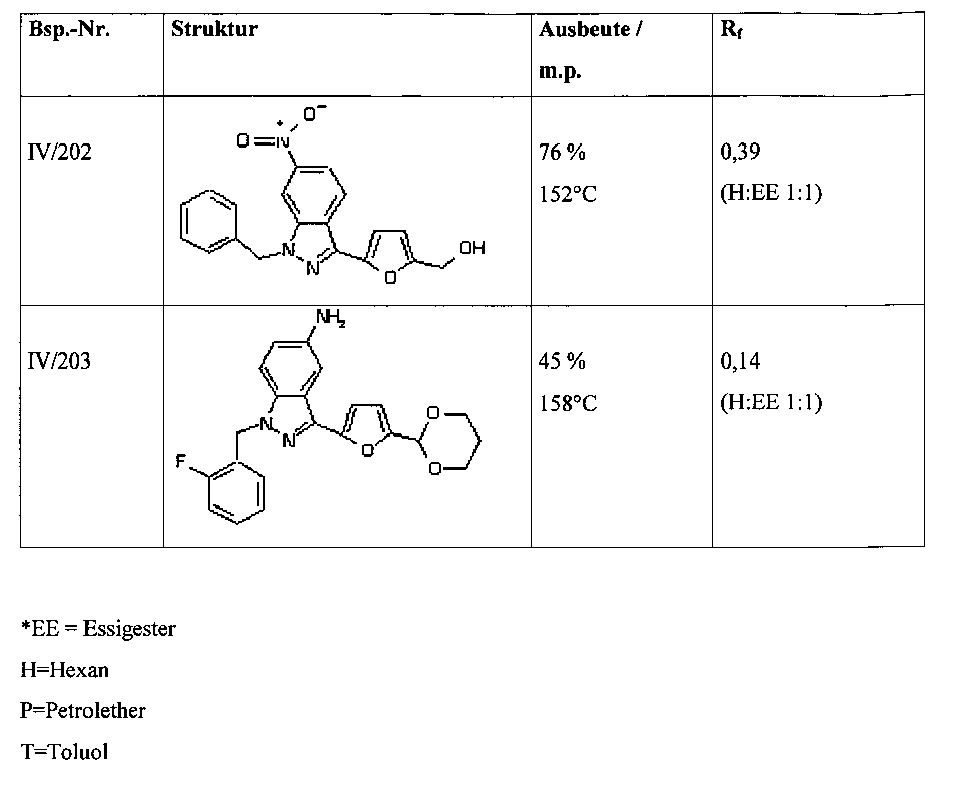

## Patentansprüche

1. Heterocyclylmethyl-substituierte Pyrazolderivate der allgemeinen Formel (I-I), 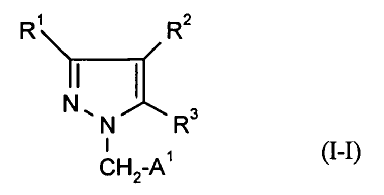 in welcher
R¹ für einen 5- gliedrigen aromatischen Heterocyclus mit 1 Heteroatom aus der Reihe S, N und/oder O oder für Phenyl steht, die gegebenenfalls bis zu 3-fach gleich oder verschieden durch Formyl, Carboxyl, Mercaptyl, Hydroxy, geradkettiges oder verzweigtes Acyl, Alkylthio, Alkoxy oder Alkoxycarbonyl mit jeweils bis zu 6 Kohlenstoffatomen, Nitro, Cyano, Halogen, Phenyl oder geradkettiges oder verzweigtes Alkyl mit bis zu 6 Kohlenstoffatomen substituiert sind, das seinerseits durch Hydroxy, Amino, Carboxyl, geradkettiges oder verzweigtes Acyl, Alkoxy, Alkoxycarbonyl oder Acylamino mit jeweils bis zu 5 Kohlenstoffatomen oder durch einen Rest der Formel -OR⁴ substituiert sein kann,
worin
R⁴ geradkettiges oder verzweigtes Acyl mit bis zu 5 Kohlenstoffatomen oder eine Gruppe der Formel -SiR⁵R⁶R⁷ bedeutet,
worin
R⁵, R⁶ und R⁷ gleich oder verschieden sind und Aryl mit 6 bis 10 Kohlenstoffatomen oder Alkyl mit bis zu 6 Kohlenstoffatomen bedeuten,
und/oder durch einen Rest der Formel 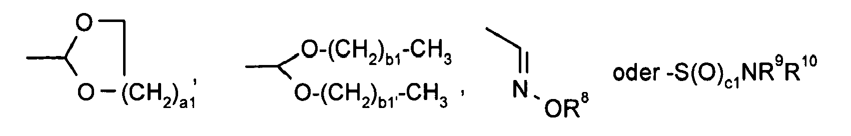 substituiert sind, worin
b1 und b1' gleich oder verschieden sind und eine Zahl 0, 1, 2 oder 3 bedeuten,
a1 eine Zahl 1, 2 oder 3 bedeutet,
R⁸ Wasserstoff oder geradkettiges oder verzweigtes Alkyl mit bis zu 4 Kohlenstoffatomen bedeutet,
c1 eine Zahl 1 oder 2 bedeutet und
R⁹ und R¹⁰ gleich oder verschieden sind und Wasserstoff oder geradkettiges oder verzweigtes Alkyl mit bis zu 10 Kohlenstoffatomen bedeutet, das gegebenenfalls durch Cycloalkyl mit 3 bis 8 Kohlenstoffatomen oder durch Aryl mit 6 bis 10 Kohlenstoffatomen substituiert ist, das seinerseits durch Halogen substituiert sein kann oder
Aryl mit 6 bis 10 Kohlenstoffatomen bedeuten, das gegebenenfalls durch Halogen substituiert ist oder
Cycloalkyl mit 3 bis 7 Kohlenstoffatomen bedeuten oder
worin
R¹¹ Wasserstoff, geradkettiges oder verzweigtes Alkyl mit zu 4 Kohlen-stoffatomen oder einen Rest der Formel 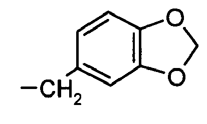
bedeutet,
oder Benzyl oder Phenyl bedeutet, wobei die Ringsysteme gegebenenfalls durch Halogen substituiert sind,
R² und R³ unter Einbezug der Doppelbindung einen 5-gliedrigen aromatischen Heterocyclus mit einem Heteroatom aus der Reihe S, N und/oder O oder einen Phenylring bilden, die gegebenenfalls bis zu 3-fach gleich oder verschieden durch Formyl, Carboxyl, Hydroxy, Amino, geradkettiges oder verzweigtes Acyl, Alkoxy oder Alkoxycarbonyl mit jeweils bis zu 6 Kohlenstoffatomen, Nitro, Cyano, Azido, Halogen, Phenyl oder geradkettiges oder verzweigtes Alkyl mit bis zu 6 Kohlenstoffatomen substituiert sind, wobei das Alkyl seinerseits durch Hydroxy, Amino, Carboxyl, geradkettiges oder verzweigtes Acyl, Alkoxy oder Alkoxycarbonyl mit jeweils bis zu 5 Kohlenstoffatomen substituiert sein kann,
und/oder gegebenenfalls durch einen Rest der Formel -S(O)_{c1'}NR^{9'}R^{10'} substituiert sind, worin c_{1'}, R^{9'} und R^{10'} die oben angegebene Bedeutung von c₁, R⁹ und R¹⁰ haben und mit dieser gleich oder verschieden sind,
A¹ für einen 5- bis 6-gliedrigen aromatischen oder gesättigten Heterocyclus mit bis zu 3 Heteroatomen aus der Reihe S, N und/oder O steht, der gegebenenfalls bis zu 3-fach gleich oder verschieden durch Mercaptyl, Hydroxy, Formyl, Carboxyl, geradkettiges oder verzweigtes Acyl, Alkylthio, Alkyloxyacyl, Alkoxy oder Alkoxycarbonyl mit jeweils bis zu 6 Kohlenstoffatomen, Nitro, Cyano, Trifluormethyl, Azido, Halogen, Phenyl oder geradkettiges oder verzweigtes Alkyl mit bis zu 6 Kohlenstoffatomen substituiert ist, das seinerseits durch Hydroxy, Carboxyl, geradkettiges oder ver-zweigtes Acyl, Alkoxy oder Alkoxycarbonyl mit jeweils bis zu 5 Kohlenstoffatomen substituiert sein kann,
und/oder durch eine Gruppe der Formel -(CO)_{d1}-NR¹²R¹³ substituiert ist,
worin
d1 eine Zahl 0 oder 1 bedeutet,
R¹² und R¹³ gleich oder verschieden sind und Wasserstoff, Phenyl, Benzyl oder geradkettiges oder verzweigtes Alkyl oder Acyl mit jeweils bis zu 5 Kohlenstoffatomen bedeuten,
und deren isomere Formen, Salze und deren N-Oxide.

2. Verbindungen nach Anspruch 1 der allgemeinen Formel (I-I)
in welcher
R¹ für Furyl, Pyrrolyl, Thienyl oder Phenyl steht, die gegebenenfalls bis zu 2-fach gleich oder verschieden durch Formyl, Carboxyl, Hydroxy, geradkettiges oder verzweigtes Acyl, Alkoxy oder Alkoxycarbonyl mit jeweils bis zu 5 Kohlenstoffatomen, Nitro, Cyano, Azido, Fluor, Chlor, Brom, Phenyl oder geradkettiges oder verzweigtes Alkyl mit bis zu 5 Kohlenstoffatomen substituiert sind, das seinerseits durch Hydroxy, Amino, Carboxyl, geradkettiges oder verzweigtes Acyl, Alkoxy, Alkoxycarbonyl oder Acylamino mit jeweils bis zu 4 Kohlenstoffatomen oder durch einen Rest der Formel -OR⁴ substituiert sein kann,
worin
R⁴ geradkettiges oder verzweigtes Acyl mit bis zu 4 Kohlenstoffatomen bedeutet,
und/oder durch einen Rest der Formel 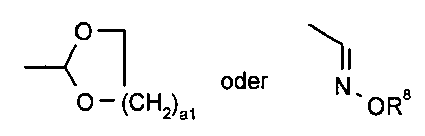 substituiert sind,
worin
a1 eine Zahl 1, 2 oder 3 bedeutet,
R⁸ Wasserstoff oder geradkettiges oder verzweigtes Alkyl mit bis zu 3 Kohlen-stoffatomen bedeutet,
R² und R³ unter Einbezug der Doppelbindung einen Furyl-, Thienyl- oder Phenylring bilden, die gegebenenfalls bis zu 3-fach gleich oder verschieden durch Formyl, Carboxyl, Hydroxy, Amino, geradkettiges oder verzweigtes Acyl, Alkoxy oder Alkoxycarbonyl mit jeweils bis zu 5 Kohlenstoffatomen, Nitro, Cyano, Azido, Fluor, Chlor, Brom, Phenyl oder geradkettiges oder verzweigtes Alkyl mit bis zu 5 Kohlenstoffatomen substituiert sind, das seinerseits durch Hydroxy, Amino, Carboxyl, geradkettiges oder verzweigtes Acyl, Alkoxy oder Alkoxycarbonyl mit jeweils bis zu 4 Kohlen-stoffatomen substituiert sein kann,
A¹ für Tetrahydropyranyl, Thienyl, Furyl, Tetrahydrofuranyl, Pyrazinyl, Morpholinyl, Pyrimidyl, Pyridazinyl oder Pyridyl steht, die gegebenenfalls bis zu 2-fach gleich oder verschieden durch Hydroxy, Formyl, Carboxyl, geradkettiges oder verzweigtes Acyl, Alkylthio, Alkyloxyacyl, Alkoxy oder Alkoxycarbonyl mit jeweils bis zu 4 Kohlenstoffatomen, Fluor, Chlor, Brom, Nitro, Cyano, Trifluormethyl oder gerad-kettiges oder verzweigtes Alkyl mit bis zu 4 Kohlenstoffatomen substituiert sind, das seinerseits durch Hydroxy, Carboxyl, geradkettiges oder verzweigtes Acyl, Alkoxy oder Alkoxycarbonyl mit jeweils bis zu 4 Kohlenstoffatomen substituiert sein kann,
und/oder durch eine Gruppe der Formel -(CO)_{d1}-NR¹²R¹³ substituiert sind,
worin
d1 eine Zahl 0 oder 1 bedeutet,
R¹² und R¹³ gleich oder verschieden sind und Wasserstoff, Phenyl, Benzyl oder geradkettiges oder verzweigtes Alkyl oder Acyl mit jeweils bis zu 4 Kohlen-stoffatomen bedeuten,
und deren isomere Formen und Salze und deren N-Oxide.

3. Verbindungen nach Anspruch 1 der allgemeinen Formel (I-I),
in welcher
R¹ für Furyl, Pyrryl, Thienyl oder Phenyl steht, die gegebenenfalls bis zu 2-fach gleich oder verschieden durch Formyl, geradkettiges oder verzweigtes Acyl, Alkoxy oder Alkoxycarbonyl mit jeweils bis zu 4 Kohlenstoffatomen oder geradkettiges oder verzweigtes Alkyl mit bis zu 4 Kohlenstoffatomen substituiert sind, das seinerseits durch Hydroxy, Carboxyl, Amino, geradkettiges oder verzweigtes Acyl, Alkoxy, Alkoxycarbonyl oder Acylamino mit jeweils bis zu 3 Kohlenstoffatomen substituiert sein kann,
und/oder durch einen Rest der Formel 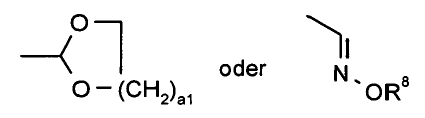 substituiert sind,
worin
a1 eine Zahl 1 oder 2 bedeutet,
R⁸ Wasserstoff oder Methyl bedeutet,
R² und R³ unter Einbezug der Doppelbindung einen Furyl-, Thienyl- oder Phenylring bilden, die gegebenenfalls bis zu 2-fach gleich oder verschieden durch Formyl, Carboxyl, Hydroxy, Amino, geradkettiges oder verzweigtes Acyl, Alkoxy oder Alkoxycarbonyl mit jeweils bis zu 4 Kohlenstoffatomen, Nitro, Cyano, Fluor, Chlor, Phenyl oder geradkettiges oder verzweigtes Alkyl mit bis zu 3 Kohlen-stoffatomen substituiert sind, das seinerseits durch Hydroxy, Amino, Carboxyl, geradkettiges oder verzweigtes Acyl, Alkoxy oder Alkoxycarbonyl mit jeweils bis zu 3 Kohlenstoffatomen substituiert sein kann,
A¹ für Tetrahydropyranyl, Tetrahydrofuranyl, Thienyl, Pyrimidyl, Pyrazinyl, Pyrida-zinyl, Furyl oder Pyridyl steht, die gegebenenfalls bis zu 2-fach gleich oder ver-schieden durch Formyl, Carboxyl, geradkettiges oder verzweigtes Acyl, Alkylthio, Alkyloxyacyl, Alkoxy oder Alkoxycarbonyl mit jeweils bis zu 3 Kohlenstoffatomen, Fluor, Chlor, Brom, Nitro, Cyano, Trifluormethyl, oder geradkettiges oder ver-zweigtes Alkyl mit bis zu 3 Kohlenstoffatomen substituiert sind, das seinerseits durch Hydroxy, Carboxyl, geradkettiges oder verzweigtes Acyl, Alkoxy oder Alkoxycarbonyl mit jeweils bis zu 3 Kohlenstoffatomen substituiert sein kann,
und/oder durch eine Gruppe der Formel -(CO)_{d1}-NR¹²R¹³ substituiert sind,
worin
d1 eine Zahl 0 oder 1 bedeutet,
R¹² und R¹³ gleich oder verschieden sind und Wasserstoff oder geradkettiges oder verzweigtes Alkyl oder Acyl mit jeweils bis zu 3 Kohlenstoffatomen bedeuten,
und deren isomere Formen und Salze und deren N-Oxide.

4. Verbindungen nach Anspruch 1 der allgemeinen Formel (I-I), in welcher
R¹ für Furyl steht, das gegebenenfalls durch Formyl oder durch den Rest der Formel - 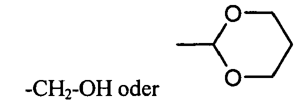 substituiert ist,
R² und R³ unter Einbezug der Doppelbindung einen durch Phenyl, Fluor oder Nitro substituierten Phenylring bilden,
A¹ für Furyl, Pyridyl, Pyrimidyl, Pyridazinyl, Thienyl, Tetrahydrofuranyl oder Tetrahydropyranyl steht, die gegebenenfalls durch Chlor, Brom, Methoxy, Methoxy-carbonyl oder Carboxyl substituiert sind
und deren Salze, isomere Formen und N-Oxide.

5. Verfahren zur Herstellung der Verbindungen nach Anspruch 1 der allgemeinen Formel (I-I), **dadurch gekennzeichnet, dass** man
[A1] Verbindungen der allgemeinen Formel (I-II) 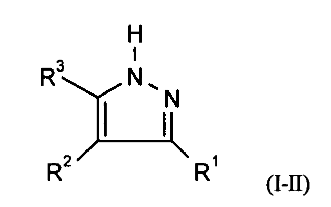 in welcher
R¹, R² und R³ die oben angegebene Bedeutung haben,
mit Verbindungen der allgemeinen Formel (I-III)
D¹-CH₂-A¹ (I-III)
in welcher
A¹ die oben angegebene Bedeutung hat
und
D¹ für Triflat oder Halogen, vorzugsweise für Brom steht, in inerten Lösemitteln, gegebenenfalls in Anwesenheit einer Base umsetzt,
oder
[B1] Verbindungen der allgemeinen Formel (I-IV) 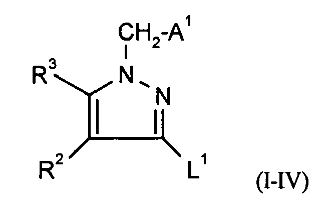 in welcher
A¹, R² und R³ die oben angegebene Bedeutung haben,
und
L¹ für einen Rest der Formel -SnR¹⁴R¹⁵R¹⁶, ZnR¹⁷, Iod oder Triflat steht,
worin
R¹⁴ , R¹⁵ und R¹⁶ gleich oder verschieden sind und geradkettiges oder verzweigtes Alkyl mit bis zu 4 Kohlenstoffatomen bedeuten,
und
R¹⁷ Halogen bedeutet,
mit Verbindungen der allgemeinen Formel (I-V)
R¹-T¹ (I-V)
in welcher
R¹ die oben angegebene Bedeutung hat
und
im Fall L¹ = SnR¹⁴R¹⁵R¹⁶ oder ZnR¹⁷
T¹ für Triflat oder für Halogen, vorzugsweise für Brom steht,
und
im Fall L¹= Jod oder Triflat
T¹ für einen Rest der Formel SnR^{14'}R^{15'}R^{16'} ,ZnR^{17'} oder BR¹⁸R¹⁹steht,
worin
R^{14'}, R^{15'}, R^{16'} und R^{17'} die oben angegebene Bedeutung von R¹⁴, R¹⁵, R¹⁶ und R¹⁷ haben und mit dieser gleich oder verschieden sind,
R¹⁸ und R¹⁹ gleich oder verschieden sind und Hydroxy, Aryloxy mit 6 bis 10 Kohlenstoffatomen oder geradkettiges oder verzweigtes Alkyl oder Alkoxy mit jeweils bis zu 5 Kohlenstoffatomen bedeuten, oder gemeinsam einen 5- oder 6-gliedrigen carbocyclischen Ring bilden,
in einer palladiumkatalysierten Reaktion in inerten Lösemitteln umsetzt,
und im Fall der Reste -S(O)_{c1}NR⁹R¹⁰ und -S(O)_{c1'}NR^{9'}R^{10'} ausgehend von den unsubstituierten Verbindungen der allgemeinen Formel (I-I) zunächst mit Thionylchlorid umsetzt und abschließend die Aminkomponente einsetzt und gegebenenfalls die unter R¹, R², R³ und/oder A¹ aufgeführten Substituenten nach üblichen Methoden, vorzugsweise durch Reduktion, Oxidation, Abspaltung von Schutzgruppen und/oder nucleophiler Substitution variiert oder einführt.

6. Arzneimittel enthaltend mindestens eine Verbindung der allgemeinen Formel (I-I) gemäß Anspruch 1.

7. Arzneizubereitungen enthaltend eine Kombination aus mindestens einer Verbindung der allgemeinen Formel (I-I) gemäß Anspruch 1 und mindestens einem organischen Nitrat oder einem NO-Donator.

8. Arzneizubereitungen enthaltend eine Kombination aus mindestens einer Verbindung der allgemeinen Formel (I-I) gemäß Anspruch 1 und Verbindungen die den Abbau von cyclischem Guanosinmonophosphat (cGMP) inhibieren.

9. Verwendung von Verbindungen der allgemeinen Formel (I-I) gemäß Anspruch 1 bei der Herstellung von Arzneimitteln zur Behandlung von kardiovaskulären Erkrankungen.

10. Verwendung von Verbindungen der allgemeinen Formel (I-I) gemäß Anspruch 1 bei der Herstellung von Arzneimitteln zur Prophylaxe und Bekämpfung der Folgen cerebraler Infarktgeschehen (Apoplexia cerebri) wie Schlaganfall, cerebraler Ischämien und des Schädel-Hirn-Traumas.

11. 1-Heterocyclyl-methyl-substituierte Pyrazole der allgemeinen Formel (II-I), 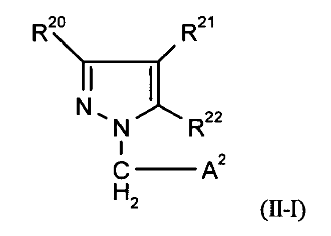 in welcher
R²⁰ für einen 6-gliedrigen aromatischen Heterocyclus mit bis zu 3 Stickstoffatomen steht, der gegebenenfalls bis zu 3-fach gleich oder verschieden durch Formyl, Carboxyl, Hydroxy, Mercaptyl, geradkettiges oder verzweigtes Acyl, Alkoxy, Alkylthio oder Alkoxycarbonyl mit jeweils bis zu 6 Kohlenstoffatomen, Nitro, Cyano, Azido, Halogen, Phenyl und/oder durch eine Gruppe der Formel
-NR²³R²⁴
substituiert ist,
worin
R²³ und R²⁴ gleich oder verschieden sind und Wasserstoff oder geradkettiges oder verzweigtes Acyl mit bis zu 6 Kohlenstoffatomen oder geradkettiges oder verzweigtes Alkyl mit bis zu 6 Kohlenstoffatomen bedeuten, das gegebenenfalls durch Cycloalkyl mit 3 bis 6 Kohlenstoffatomen, Hydroxy, Amino oder durch geradkettiges oder verzweigtes Alkoxy, Acyl oder Alkoxycarbonyl mit jeweils bis zu 5 Kohlenstoffatomen substituiert ist,
oder
R²³ und R²⁴ gemeinsam mit dem Stickstoffatom einen 3- bis 7-gliedrigen gesättigten oder partiell ungesättigten Heterocyclus bilden, der gegebenenfalls zusätzlich ein Sauerstoff- oder Schwefelatom oder einen Rest der Formel -NR²⁵ enthalten kann,
worin
R²⁵ Wasserstoff oder geradkettiges oder verzweigtes Alkyl mit bis zu 4 Kohlenstoffatomen bedeutet,
und/oder durch geradkettiges oder verzweigtes Alkyl mit bis zu 6 Kohlenstoffatomen substituiert ist, das seinerseits durch Hydroxy, Amino, Halogen, Carboxyl, geradkettiges oder verzweigtes Acyl, Alkoxy, Alkoxycarbonyl oder Acylamino mit jeweils bis zu 5 Kohlenstoffatomen oder durch einen Rest der Formel -OR²⁶ substituiert sein kann,
worin
R²⁶ geradkettiges oder verzweigtes Acyl mit bis zu 5 Kohlenstoffatomen oder eine Gruppe der Formel -SiR²⁷R²⁸R²⁹ bedeutet,
worin
R²⁷ , R²⁸ und R²⁹ gleich oder verschieden sind und Aryl mit 6 bis 10 Kohlenstoffatomen oder Alkyl mit bis zu 6 Kohlenstoffatomen bedeuten,
und/oder gegebenenfalls durch einen Rest der Formel 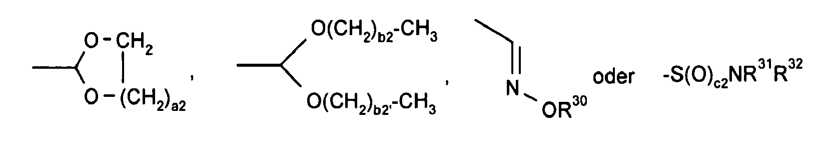 substituiert ist,
worin b2 und b2' gleich oder verschieden sind und eine Zahl 0, 1, 2 oder 3 bedeuten,
a2 eine Zahl 1, 2 oder 3 bedeutet,
R³⁰ Wasserstoff oder geradkettiges oder verzweigtes Alkyl mit bis zu 4 Kohlenstoffatomen bedeutet,
c2 eine Zahl 1 oder 2 bedeutet und
R³¹ und R³² gleich oder verschieden sind und Wasserstoff oder geradkettiges oder verzweigtes Alkyl mit bis zu 10 Kohlenstoffatomen bedeutet, das gegebenenfalls durch Cycloalkyl mit 3 bis 8 Kohlenstoffatomen oder durch Aryl mit 6 bis 10 Kohlenstoffatomen substituiert ist, das seinerseits durch Halogen substituiert sein kann oder
Aryl mit 6 bis 10 Kohlenstoffatomen bedeuten, das gegebenenfalls durch Halogen substituiert ist oder
Cycloalkyl mit 3 bis 7 Kohlenstoffatomen bedeuten oder
R³¹ und R³² gemeinsam mit dem Stickstoffatom einen 5- bis 7-gliedrigen gesättigten Heterocyclus bilden, der gegebenenfalls ein weiteres Sauerstoffatom oder einen Rest -NR³³ enthalten kann,
worin
R³³ Wasserstoff, geradkettiges oder verzweigtes Alkyl mit zu 4 Kohlenstoffatomen oder einen Rest der Formel 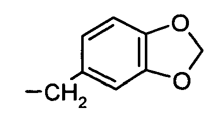
bedeutet,
oder Benzyl oder Phenyl bedeutet, wobei die Ringsysteme gegebenenfalls durch Halogen substituiert sind,
R²¹ und R²² unter Einbezug der Doppelbindung einen 5-gliedrigen aromatischen Heterocyclus mit einem Heteroatom aus der Reihe S, N und/oder O oder einen Phenylring bilden, die gegebenenfalls bis zu 3-fach gleich oder verschieden durch Formyl, Mercaptyl, Carboxyl, Hydroxy, Amino, geradkettiges oder verzweigtes Acyl, Alkylthio, Alkoxy oder Alkoxycarbonyl mit jeweils bis zu 6 Kohlenstoffatomen, Nitro, Cyano, Azido, Halogen, Phenyl oder geradkettiges oder verzweigtes Alkyl mit bis zu 6 Kohlenstoffatomen substituiert sind, das seinerseits durch Hydroxy, Amino, Carboxyl, geradkettiges oder verzweigtes Acyl, Alkoxy oder Alkoxycarbonyl mit jeweils bis zu 5 Kohlenstoffatomen substituiert sein kann, oder gegebenenfalls durch eine Gruppe der Formel -S(O)_{c2}'NR^{31'}R^{32'} substituiert sind, worin c_{2'}, R^{31'} und R^{32'} die oben angegebene Bedeutung von c2, R³¹ und R³² haben und mit dieser gleich oder verschieden sind,
A² für Phenyl oder einen 5- bis 6-gliedrigen aromatischen oder gesättigten Heterocyclus mit bis zu 3 Heteroatomen aus der Reihe S, N und/oder O steht, der gegebenenfalls bis zu 3-fach gleich oder verschieden durch Mercaptyl, Hydroxy, Formyl, Carboxyl, geradkettiges oder verzweigtes Acyl, Alkylthio, Alkyloxyacyl, Alkoxy oder Alkoxycarbonyl mit jeweils bis zu 6 Kohlenstoffatomen, Nitro, Cyano, Trifluormethyl, Azido, Halogen, Phenyl oder geradkettiges oder verzweigtes Alkyl mit bis zu 6 Kohlenstoffatomen substituiert ist, das seinerseits durch Hydroxy, Carboxyl, geradkettiges oder verzweigtes Acyl, Alkoxy oder Alkoxycarbonyl mit jeweils bis zu 5 Kohlenstoffatomen substituiert sein kann,
und/oder durch eine Gruppe der Formel -(CO)_{d2}-NR³⁴R³⁵ substituiert ist,
worin
d2 eine Zahl 0 oder 1 bedeutet,
R³⁴ und R³⁵ gleich oder verschieden sind und Wasserstoff, Phenyl, Benzyl oder geradkettiges oder verzweigtes Alkyl oder Acyl mit jeweils bis zu 5 Kohlenstoffatomen bedeuten,
deren isomere Formen und Salze und deren N-Oxide.

12. Verbindungen nach Anspruch 11 der allgemeinen Formel (II-I),
in welcher
R²⁰ für einen Rest der Formel 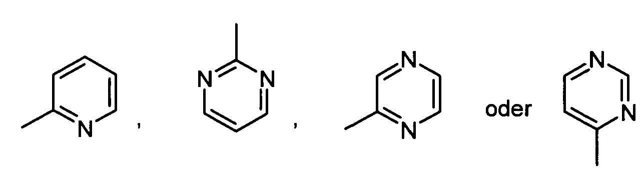
steht, die gegebenenfalls bis zu 3-fach gleich oder verschieden durch Formyl, Carboxyl, Hydroxy, geradkettiges oder verzweigtes Acyl, Alkoxy oder Alkoxycarbonyl mit jeweils bis zu 5 Kohlenstoffatomen, Nitro, Cyano, Azido, Fluor, Chlor, Brom, Phenyl und/oder durch eine Gruppe der Formel -NR²³R²⁴ substituiert sind,
worin
R²³ und R²⁴ gleich oder verschieden sind und Wasserstoff oder geradkettiges oder verzweigtes Acyl mit bis zu 4 Kohlenstoffatomen oder geradkettiges oder verzweigtes Alkyl mit bis zu 4 Kohlenstoffatomen bedeuten, das gegebenenfalls durch Hydroxy, Amino oder durch geradkettiges oder verzweigtes Alkoxy mit bis zu 3 Kohlenstoffatomen substituiert ist, oder
R²³ und R²⁴ gemeinsam mit dem Stickstoffatom einen Morpholinring oder einen Rest der Formel 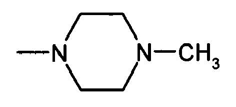
bilden
und/oder durch geradkettiges oder verzweigtes Alkyl mit bis zu 5 Kohlenstoffatomen substituiert sind, das seinerseits durch Hydroxy, Amino, Fluor, Carboxyl, geradkettiges oder verzweigtes Acyl, Alkoxy, Alkoxycarbonyl oder Acylamino mit jeweils bis zu 4 Kohlenstoffatomen oder durch einen Rest der Formel -OR²⁶ substituiert sein kann,
worin
R²⁶ geradkettiges oder verzweigtes Acyl mit bis zu 4 Kohlenstoffatomen bedeutet,
und/oder gegebenenfalls durch einen Rest der Formel 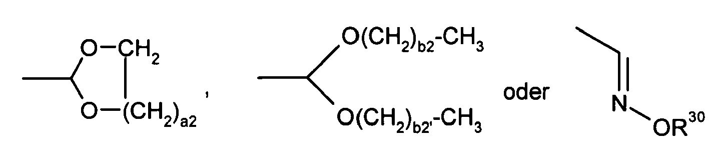
substituiert sind,
worin
b2 und b2' gleich oder verschieden sind und eine Zahl 0, 1, 2 oder 3 bedeuten,
a2 eine Zahl 1, 2 oder 3 bedeutet,
R³⁰ Wasserstoff oder geradkettiges oder verzweigtes Alkyl mit bis zu 4 Kohlenstoffatomen bedeutet,
R²¹ und R²² unter Einbezug der Doppelbindung einen Furyl-, Thienyl- oder Phenylring bilden, die gegebenenfalls bis zu 3-fach gleich oder verschieden durch Formyl, Carboxyl, Hydroxy, Amino, geradkettiges oder verzweigtes Acyl, Alkoxy oder Alkoxycarbonyl mit jeweils bis zu 5 Kohlenstoffatomen, Nitro, Cyano, Azido, Fluor, Chlor, Brom, Phenyl oder geradkettiges oder verzweigtes Alkyl mit bis zu 5 Kohlenstoffatomen substituiert sind, das seinerseits durch Hydroxy, Amino, Carboxyl, geradkettiges oder verzweigtes Acyl, Alkoxy oder Alkoxycarbonyl mit jeweils bis zu 4 Kohlenstoffatomen substituiert sein kann,
A² für Phenyl oder für Tetrahydropyranyl, Furyl, Tetrahydrofuranyl, Morpholinyl, Pyrimidyl, Pyridazinyl oder Pyridyl steht, die gegebenenfalls bis zu 2-fach gleich oder verschieden durch Hydroxy, Formyl, Carboxyl, geradkettiges oder verzweigtes Acyl, Alkylthio, Alkyloxyacyl, Alkoxy oder Alkoxycarbonyl mit jeweils bis zu 4 Kohlenstoffatomen, Fluor, Chlor, Brom, Nitro, Cyano, Trifluormethyl oder geradkettiges oder verzweigtes Alkyl mit bis zu 4 Kohlenstoffatomen substituiert sind, das seinerseits durch Hydroxy, Carboxyl, geradkettiges oder verzweigtes Acyl, Alkoxy oder Alkoxycarbonyl mit jeweils bis zu 4 Kohlenstoffatomen substituiert sein kann,
und/oder durch eine Gruppe der Formel -(CO)_{d2}-NR³⁴R³⁵ substituiert sind,
worin
d2 eine Zahl 0 oder 1 bedeutet,
R³⁴ und R³⁵ gleich oder verschieden sind und Wasserstoff, Phenyl, Benzyl oder geradkettiges oder verzweigtes Alkyl oder Acyl mit jeweils bis zu 4 Kohlenstoffatomen bedeuten,
deren isomere Formen und Salze und deren N-Oxide.

13. Verbindungen nach Anspruch 11 der allgemeinen Formel (II-I),
in welcher
R²⁰ für einen Rest der Formel 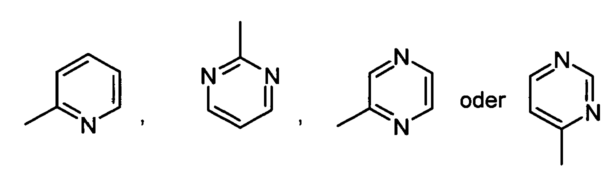
steht,
wobei die Ringsysteme, gegebenenfalls bis zu 3-fach gleich oder verschieden durch Formyl, geradkettiges oder verzweigtes Acyl, Alkoxy oder Alkoxycarbonyl mit jeweils bis zu 4 Kohlenstoffatomen, Methylamino, Amino, Fluor, Chlor, Brom, Cyano, Azido oder geradkettiges oder verzweigtes Alkyl mit bis zu 4 Kohlenstoffatomen substituiert sind, das seinerseits durch Hydroxy, Carboxyl, Amino, geradkettiges oder verzweigtes Acyl, Alkoxy, Alkoxycarbonyl oder Acylamino mit jeweils bis zu 3 Kohlenstoffatomen substituiert sein kann,
und/oder gegebenenfalls durch einen Rest der Formel 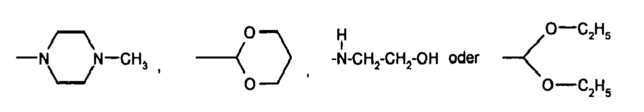 substituiert sind
R²¹ und R²² unter Einbezug der Doppelbindung einen Furyl-, Thienyl- oder Phenylring bilden, die gegebenenfalls bis zu 2-fach gleich oder verschieden durch Formyl, Carboxyl, Hydroxy, Amino, geradkettiges oder verzweigtes Acyl, Alkoxy oder Alkoxycarbonyl mit jeweils bis zu 4 Kohlenstoffatomen, Nitro, Cyano, Fluor, Chlor, Phenyl oder geradkettiges oder verzweigtes Alkyl mit bis zu 3 Kohlenstoffatomen substituiert sind, das seinerseits durch Hydroxy, Amino, Carboxyl, geradkettiges oder verzweigtes Acyl, Alkoxy oder Alkoxycarbonyl mit jeweils bis zu 3 Kohlenstoffatomen substituiert sein kann,
A² für Phenyl, Tetrahydropyranyl, Tetrahydrofuranyl, Furyl oder Pyridyl steht, die gegebenenfalls bis zu 2-fach gleich oder verschieden durch Formyl, Carboxyl, geradkettiges oder verzweigtes Acyl, Alkylthio, Alkyloxyacyl, Alkoxy oder Alkoxycarbonyl mit jeweils bis zu 3 Kohlenstoffatomen, Fluor, Chlor, Brom, Nitro, Cyano, Trifluormethyl, oder für geradkettiges oder verzweigtes Alkyl mit bis zu 3 Kohlenstoffatomen substituiert sind, das seinerseits durch Hydroxy, Carboxyl, geradkettiges oder verzweigtes Acyl, Alkoxy oder Alkoxycarbonyl mit jeweils bis zu 3 Kohlenstoffatomen substituiert sein kann,
und/oder durch eine Gruppe der Formel -(CO)_{d2}-NR³⁴R³⁵ substituiert sind,
worin
d2 eine Zahl 0 oder 1 bedeutet,
R¹⁴ und R³⁵ gleich oder verschieden sind und Wasserstoff oder geradkettiges oder verzweigtes Alkyl oder Acyl mit jeweils bis zu 3 Kohlenstoffatomen bedeuten,
deren isomere Formen, Salze und N-Oxide.

14. Verbindungen nach Anspruch 11 der allgemeinen Formel (II-I), in welcher
R²⁰ für einen Rest der Formel 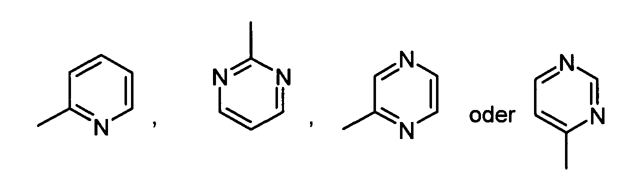
steht,
wobei die oben aufgeführten heterocyclischen Ringsysteme, gegebenenfalls bis zu 3-fach gleich oder verschieden durch Methyl, Fluor, Formyl, Amino, Cyano, Methoxy, Methoxycarbonyl, Methylamino, Chlor oder durch einen Rest der Formel 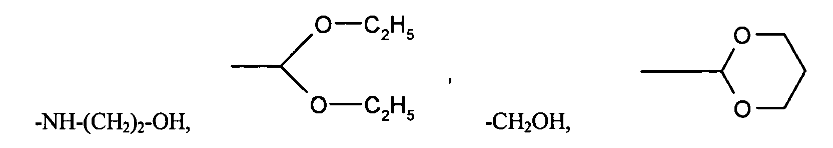 oder 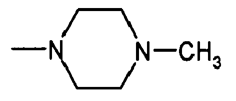 substituiert sind,
R²¹ und R²² unter Einbezug der Doppelbindung gemeinsam einen Phenylring bilden und
A² für Phenyl steht, das gegebenenfalls durch Fluor oder Cyano substituiert ist
und deren isomere Formen, Salze und N-Oxide.

15. Verfahren zur Herstellung von Verbindungen der allgemeinen Formel (II-I) nach Anspruch 1, **dadurch gekennzeichnet, dass** man
**[A2]** Verbindungen der allgemeinen Formel (II-II) 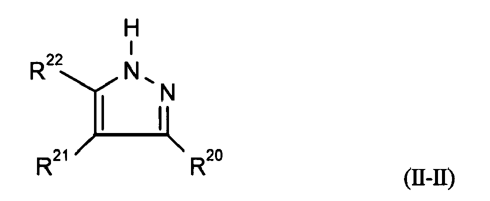 in welcher
R²⁰, R²¹ und R²² die oben angegebene Bedeutung haben,
mit Verbindungen der allgemeinen Formel (II-III)
D²-CH₂-A² (II-III)
in welcher
A² die oben angegebene Bedeutung hat,
und
D² für Triflat oder Halogen, vorzugsweise für Brom steht,
in inerten Lösemitteln, gegebenenfalls in Anwesenheit einer Base umsetzt,
oder
[B2] Verbindungen der allgemeinen Formel (II-IV) 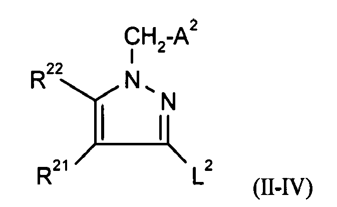 in welcher
A², R²¹ und R²² die oben angegebene Bedeutung haben,
und
L² für einen Rest der Formel -SnR³⁶R³⁷R³⁸, ZnR³⁹, Iod oder Triflat steht,
worin
R³⁶, R³⁷ und R³⁹ gleich oder verschieden sind und geradkettiges oder verzweigtes Alkyl mit bis zu 4 Kohlenstoffatomen bedeuten,
und
R³⁹ Halogen bedeutet,
mit Verbindungen der allgemeinen Formel (II-V)
R²⁰-T² (II-V)
in welcher
R²⁰ die oben angegebene Bedeutung hat,
und
im Fall L² = SnR³⁶R³⁷R³⁸ oder ZnR³⁹
T² für Triflat oder für Halogen, vorzugsweise für Brom steht,
und
im Fall L² = Jod oder Triflat
T² für einen Rest der Formel SnR^{36'}R^{37'}R^{38'}, ZnR^{39'} oder BR⁴⁰R⁴¹steht,
worin
R^{36'}, R^{37'}, R^{38'} und R^{39'} die oben angegebene Bedeutung von R³⁶, R³⁷, R³⁸ und R³⁹ haben und mit dieser gleich oder verschieden sind,
R⁴⁰ und R⁴¹ gleich oder verschieden sind und Hydroxy, Aryloxy mit 6 bis 10 Kohlenstoffatomen oder geradkettiges oder verzweigtes Alkyl oder Alkoxy mit jeweils bis zu 5 Kohlenstoffatomen bedeuten, oder gemeinsam einen 5- oder 6-gliedrigen carbocyclischen Ring bilden, in einer palladiumkatalysierten Reaktion in inerten Lösemitteln umsetzt,
und im Fall der Reste -S(O)_{c2}NR³¹R³² und -S(O)_{c2}'NR^{31'}R^{32'} ausgehend von den unsubstituierten Verbindungen der allgemeinen Formel (II-I) zunächst mit Thionylchlorid umsetzt und abschließend die Aminkomponente einsetzt,
und gegebenenfalls die unter R²⁰, R²¹ R²² und/oder A² aufgeführten Substituenten nach üblichen Methoden, vorzugsweise durch Reduktion, Oxidation, Abspaltung von Schutzgruppen und/oder nucleophiler Substitution variiert oder einführt.

16. Arzneimittel enthaltend mindestens eine Verbindung der allgemeinen Formel (II-I) gemäß Anspruch 11.

17. Arzneizubereitungen enthaltend eine Kombination aus mindestens einer Verbindung aus der allgemeinen Formel (II-I) gemäß Anspruch 11 und mindestens einem organischen Nitrat oder einem NO-Donator.

18. Arzneizubereitungen enthaltend eine Kombination aus mindestens einer Verbindung der allgemeinen Formel (II-I) gemäß Anspruch 11 und Verbindungen die den Abbau von cyclischem Guanosinmonophosphat (cGMP) inhibieren.

19. Verwendung von Verbindungen der allgemeinen Formel (II-I) gemäß Anspruch 11 bei der Herstellung von Arzneimitteln zur Behandlung von kardiovaskulären Erkrankungen.

20. Verwendung von Verbindungen der allgemeinen Formel (II-I) gemäß Anspruch 11 bei der Herstellung von Arzneimitteln zur Prophylaxe und Bekämpfung der Folgen cerebraler Infarktgeschehen (Apoplexia cerebri) wie Schlaganfall, cerebraler Ischämien und des Schädel-Hirn-Traumas.

21. 1-Benzyl-3-(substituierte heteroaryl)-kondensierte Pyrazol-Derivate der allgemeinen Formel (IV-I), 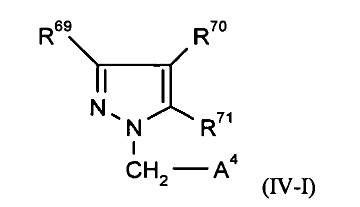 in welcher
A⁴ für Phenyl steht, das gegebenenfalls bis zu 3-fach gleich oder verschieden durch Halogen, Hydroxy, Cyano, Carboxyl, Nitro, Trifluormethyl, Trifluormethoxy, Azido, geradkettiges oder verzweigtes Alkyl, Alkoxy oder Alkoxycarbonyl mit jeweils bis zu 6 Kohlenstoffatomen substituiert ist,
R⁶⁹ für einen Rest der Formel 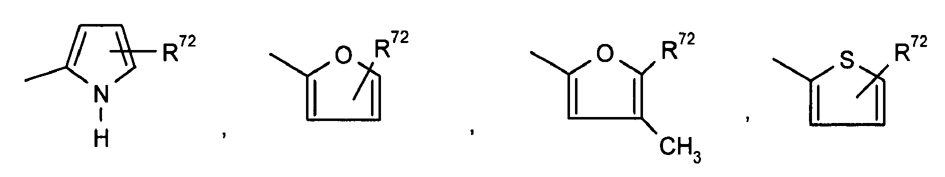
oder 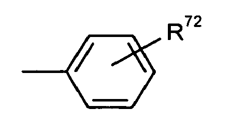 steht,
worin
R⁷² einen Rest der Formel -CH(OH)-CH₃ oder geradkettiges oder verzweigtes Alkyl mit 2 bis 6 Kohlenstoffatomen bedeutet, das 1- bis 2-fach durch Hydroxy oder geradkettiges oder verzweigtes Alkoxy mit bis zu 4 Kohlenstoffatomen substituiert ist, oder
Formyl, geradkettiges oder verzweigtes Acyl mit bis zu 6 Kohlenstoffatomen, Nitro oder geradkettiges oder verzweigtes Alkyl mit bis zu 6 Kohlenstoffatomen bedeutet, das durch Amino, Azido oder durch einen Rest der Formel -OR⁷³ substituiert ist,
worin
R⁷³ geradkettiges oder verzweigtes Acyl mit bis zu 5 Kohlenstoffatomen oder eine Gruppe der Formel -SiR⁷⁴_{R}⁷⁵_{R}⁷⁶, 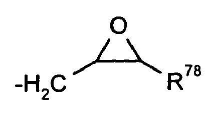 oder -CH₂-OR⁷⁹ bedeutet,
worin
R⁷⁴, R⁷⁵ und R⁷⁶ gleich oder verschieden sind und Aryl mit 6 bis 10 Kohlenstoffatomen oder geradkettiges oder verzweigtes Alkyl mit bis zu 6 Kohlenstoffatomen bedeuten,
R⁷⁸ Wasserstoff oder geradkettiges oder verzweigtes Alkyl mit bis zu 3 Kohlenstoffatomen bedeutet,
und
R⁷⁹ Wasserstoff oder geradkettiges oder verzweigtes Alkyl mit bis zu 4 Kohlenstoffatomen bedeutet,
oder
R⁷² eine Gruppe der Formel 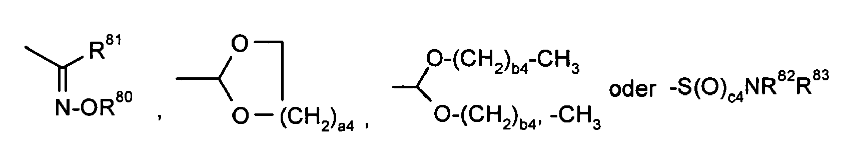
bedeutet, worin
R⁸⁰ Wasserstoff oder geradkettiges oder verzweigtes Alkyl mit bis zu 4 Kohlenstoffatomen bedeutet,
R⁸¹ Wasserstoff oder geradkettiges oder verzweigtes Alkyl mit bis zu 4 Kohlenstoffatomen bedeutet,
und
a4 eine Zahl 1, 2 oder 3 bedeutet,
b4 und b4' gleich oder verschieden sind, eine Zahl 0, 1, 2 oder 3 bedeutet,
c4 eine Zahl 1 oder 2 bedeutet und
R⁸² und R⁸³ gleich oder verschieden sind und Wasserstoff oder geradkettiges oder verzweigtes Alkyl mit bis zu 10 Kohlenstoffatomen bedeutet, das gegebenenfalls durch Cycloalkyl mit 3 bis 8 Kohlenstoffatomen oder durch Aryl mit 6 bis 10 Kohlenstoffatomen substituiert ist, das seinerseits durch Halogen substituiert sein kann oder
Aryl mit 6 bis 10 Kohlenstoffatomen bedeuten, das gegebenenfalls durch Halogen substituiert ist oder
Cycloalkyl mit 3 bis 7 Kohlenstoffatomen bedeuten oder
R⁸² und R⁸³ gemeinsam mit dem Stickstoffatom einen 5- bis 7-gliedrigen gesättigten Heterocyclus bilden, der gegebenenfalls ein weiteres Sauerstoffatom oder einen Rest -NR⁸⁴ enthalten kann,
worin
R⁸⁴ Wasserstoff, geradkettiges oder verzweigtes Alkyl mit zu 4 Kohlenstoffatomen oder einen Rest der Formel 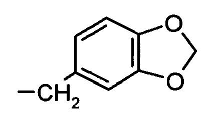
bedeutet,
oder Benzyl oder Phenyl bedeutet, wobei die Ringsysteme gegebenenfalls durch Halogen substituiert sind,
oder
R⁷² eine Gruppe der Formel -CH₂-OR⁸⁵ bedeutet,
worin
R⁸⁵ Wasserstoff oder geradkettiges oder verzweigtes Alkyl mit bis zu 3 Kohlenstoffatomen bedeutet,
R⁷⁰ und R⁷¹ gemeinsam einen Rest der Formel 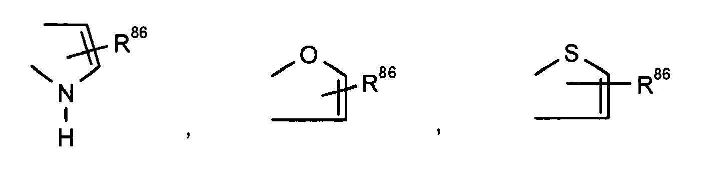
oder 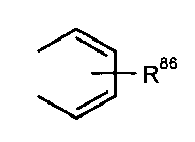 bilden,
worin
R⁸⁶ Wasserstoff, Halogen, Hydroxy, Nitro, Amino, Trifluormethyl oder geradkettiges oder verzweigtes Alkyl oder Alkoxy mit jeweils bis zu 4 Kohlenstoffatomen oder eine Gruppe der Formel -S(O)_{c4}'NR^{82'}R^{83'} bedeutet, worin c4', R^{82'} und R^{83'} die oben angegebene Bedeutung von c4, R⁸² und R⁸³ haben und mit dieser gleich oder verschieden sind,
und deren isomere Formen und Salze,
mit der Maßgabe, dass R⁷² im Fall des Phenylringes und in der direkt zum Heteroatom benachbarten Position nur dann für die Gruppe der Formel -CH₂-OR⁸⁵ stehen darf, wenn A⁴ entweder für Phenyl steht, das durch Cyano, Nitro, Trifluormethyl, Azido, Carboxyl oder geradkettiges oder verzweigtes Alkoxycarbonyl mit bis zu 6 Kohlenstoffatomen substituiert ist oder mindestens 2-fach durch die oben aufgeführten Reste substituiert ist oder R⁸⁶ für Nitro, Amino, Trifluormethyl oder für die Gruppe der Formel -S(O)_{c4}NR^{86'}R^{83'} steht.

22. Verbindungen nach Anspruch 21 der allgemeinen Formel (IV-I),
in welcher
A⁴ für Phenyl steht, das gegebenenfalls bis zu 3-fach gleich oder verschieden durch Fluor, Chlor, Brom, Hydroxy, Cyano, Carboxyl, Nitro, Trifluormethyl, Trifluormethoxy, Azido, geradkettiges oder verzweigtes Alkyl, Alkoxy oder Alkoxycarbonyl mit jeweils bis zu 5 Kohlenstoffatomen substituiert ist,
R⁶⁹ für einen Rest der Formel 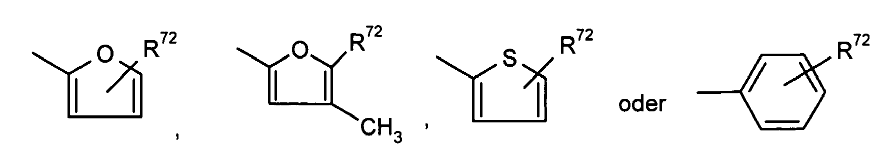
steht,
worin
R⁷² einen Rest der Formel -CH(OH)-CH₃ oder geradkettiges oder verzweigtes Alkyl mit 2 bis 4 Kohlenstoffatomen bedeutet, das 1- bis 2-fach durch Hydroxy oder geradkettiges oder verzweigtes Alkoxy mit bis zu 3 Kohlenstoffatomen substituiert ist, oder
Formyl, geradkettiges oder verzweigtes Acyl mit bis zu 4 Kohlenstoffatomen, Nitro oder geradkettiges oder verzweigtes Alkyl mit bis zu 4 Kohlenstoffatomen bedeutet, das durch Amino, Azido oder durch einen Rest der Formel -OR⁷³ substituiert ist,
worin
R⁷³ geradkettiges oder verzweigtes Acyl mit bis zu 4 Kohlenstoffatomen oder eine Gruppe der Formel 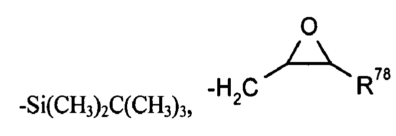
oder -CH₂-OR⁷⁹ bedeutet,
worin
R⁷⁸ Wasserstoff oder geradkettiges oder verzweigtes Alkyl mit bis zu 3 Kohlenstoffatomen bedeutet,
und
R⁷⁹ Wasserstoff oder geradkettiges oder verzweigtes Alkyl mit bis zu 3 Kohlenstoffatomen bedeutet,
oder
R⁷² eine Gruppe der Formel 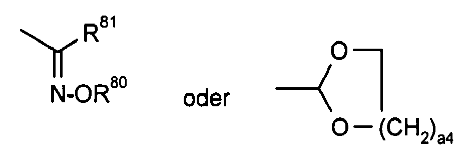
bedeutet,
worin
R⁸⁰ Wasserstoff oder geradkettiges oder verzweigtes Alkyl mit bis zu 3 Kohlenstoffatomen bedeutet,
R⁸¹ Wasserstoff oder geradkettiges oder verzweigtes Alkyl mit bis zu 3 Kohlenstoffatomen bedeutet,
und
a4 eine Zahl 1 oder 2 bedeutet,
oder
R⁷² eine Gruppe der Formel -CH₂-OR⁸⁵ bedeutet,
worin
R⁸⁵ Wasserstoff oder geradkettiges oder verzweigtes Alkyl mit bis zu 3 Kohlenstoffatomen bedeutet,
R⁷⁰ und R⁷¹ gemeinsam einen Rest der Formel 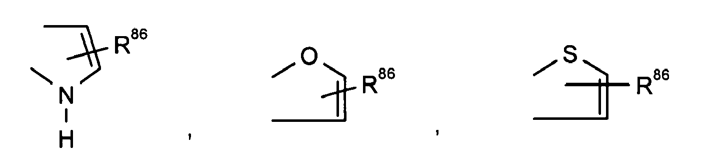
oder 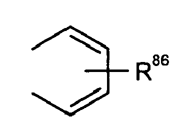 bilden,
worin
R⁸⁶ Wasserstoff, Fluor, Chlor, Brom, Hydroxy, Nitro, Amino, Trifluormethyl oder geradkettiges oder verzweigtes Alkyl oder Alkoxy mit jeweils bis zu 3 Kohlenstoffatomen bedeutet,
und deren isomere Formen und Salze,
mit der Maßgabe, dass R⁷² im Fall des Phenylringes und in der direkt zum Heteroatom benachbarten Position nur dann für die Gruppe der Formel -CH₂-OR⁸⁵ stehen darf, wenn A⁴ entweder für Phenyl steht, das durch Cyano, Nitro, Trifluormethyl, Azido, Carboxyl oder geradkettiges oder verzweigtes Alkoxycarbonyl mit bis zu 6 Kohlenstoffatomen substituiert ist oder mindestens 2-fach durch die oben aufgeführten Reste substituiert ist, oder
R⁸⁶ für Nitro, Amino oder Trifluormethyl steht.

23. Verbindungen nach Anspruch 21 der allgemeinen Formel (IV-I)
in welcher
A⁴ für Phenyl steht, das gegebenenfalls bis zu 3-fach gleich oder verschieden durch Fluor, Chlor, Brom, Trifluormethyl, Trifluormethoxy, Cyano, Nitro, Carboxyl, Azido, geradkettiges oder verzweigtes Alkyl, Alkoxy der Alkoxycarbonyl mit jeweils bis zu 3 Kohlenstoffatomen substituiert ist,
R⁶⁹ für einen Rest der Formel 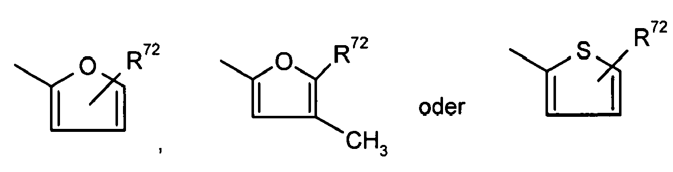
steht,
worin
R⁷² einen Rest der Formel -CH(OH)-CH₃ oder geradkettiges oder verzweigtes Alkyl mit 2 bis 4 Kohlenstoffatomen bedeuten, das 1- bis 2-fach durch Hydroxy, Methyl oder Methoxy substituiert ist, oder
Formyl, geradkettiges oder verzweigtes Acyl mit bis zu 3 Kohlenstoffatomen, Nitro oder geradkettiges oder verzweigtes Alkyl mit bis zu 3 Kohlenstoffatomen bedeuten, das durch Amino, Azido oder durch einen Rest der Formel -OR⁷³ substituiert ist,
worin
R⁷³ geradkettiges oder verzweigtes Acyl mit bis zu 3 Kohlenstoffatomen oder eine Gruppe der Formel 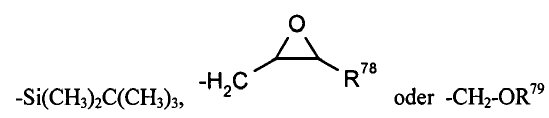
bedeutet,
worin
R⁷⁸ Wasserstoff oder Methyl bedeutet,
und
R⁷⁹ Wasserstoff oder geradkettiges oder verzweigtes Alkyl mit bis zu 3 Kohlenstoffatomen bedeutet,
oder
R⁷² eine Gruppe der Formel 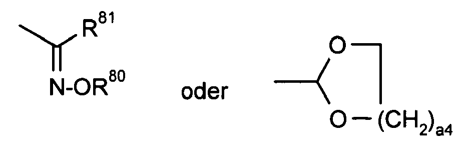
bedeutet,
worin
R⁸⁰ Wasserstoff oder geradkettiges oder verzweigtes Alkyl mit bis zu 3 Kohlenstoffatomen bedeutet,
R⁸¹ Wasserstoff oder Methyl bedeutet,
und
a4 eine Zahl 1 oder 2 bedeutet,
oder
R⁷² gleich oder verschieden ist und die Gruppe der Formel -CH₂-OR⁸⁵ bedeutet,
worin
R⁸⁵ Wasserstoff oder Methyl bedeutet,
R⁷⁰ und R⁷¹ gemeinsam einen Rest der Formel 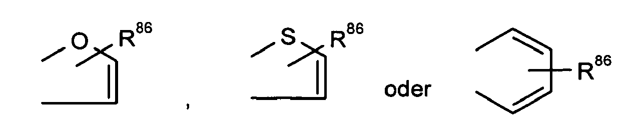 bilden,
worin
R⁸⁶ Wasserstoff, Fluor, Chlor, Brom, Nitro, Trifluormethyl, Amino, Hydroxy oder geradkettiges oder verzweigtes Alkyl oder Alkoxy mit jeweils bis zu 3 Kohlenstoffatomen bedeutet,
und deren isomere Formen und Salze,
mit der Maßgabe, dass R⁷² im Fall des Phenylringes und in der direkt zum Heteroatom benachbarten Position nur dann für die Gruppe der Formel -CH₂-OR⁸⁵ stehen darf, wenn A entweder für Phenyl steht, das durch Cyano, Nitro, Trifluormethyl, Azido, Carboxyl oder geradkettiges oder verzweigtes Alkoxycarbonyl mit bis zu 6 Kohlenstoffatomen substituiert ist oder mindestens 2-fach durch die oben aufgeführten Reste substituiert ist oder R⁸⁶ für Nitro, Amino oder Trifluormethyl steht.

24. Verbindungen nach Anspruch 21 der allgemeinen Formel (IV-I), in welcher
A⁴ für Phenyl steht, das gegebenenfalls bis zu 2-fach gleich oder verschieden durch Fluor, Chlor, Methyl, Methoxy, Cyano, Nitro, Trifluormethyl oder Trifluormethoxy substituiert ist.
R⁷⁰ und R⁷¹ gemeinsam unter Einbezug der Doppelbindung einen Phenylring bilden, der gegebenenfalls durch Nitro, Fluor, Amino oder Methoxy substituiert ist,
mit der Maßgabe, dass R⁷² im Fall des Phenylringes und in der direkt zum Heteroatom benachbarten Position nur dann für die Gruppe der Formel -CH₂OR⁸⁵ stehen darf, wenn A⁴ entweder für Phenyl steht, das durch Cyano, Nitro, Trifluormethyl, Azido, Carboxyl oder geradkettiges oder verzweigtes Alkoxycarbonyl mit bis zu 6 Kohlenstoffatomen substituiert ist oder mindestens 2-fach durch die oben aufgeführten Reste substituiert ist,
oder
R⁸⁶ für Nitro, Amino oder Trifluormethyl steht.

25. Verfahren zur Herstellung der Verbindungen nach Anspruch 21 der allgemeinen Formel (IV-I), **dadurch gekennzeichnet, dass** man
[A4] Verbindungen der allgemeinen Formel (IV-II)
H₂N-NH-CH₂-A⁴ (IV-II)
in welcher
A⁴ die oben angegebene Bedeutung hat,
durch Umsetzung mit Verbindungen der allgemeinen Formel (IV-III) 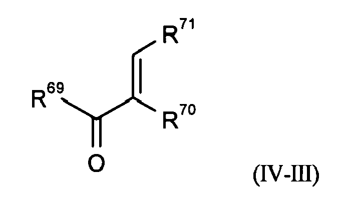 in welcher
R⁶⁹, R7⁰ und R⁷¹ die oben angegebene Bedeutung haben,
in die Verbindungen der allgemeinen Formel (IV-IV) 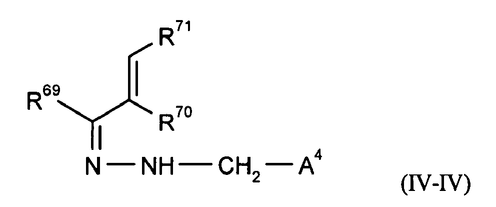 in welcher
A⁴, R⁶⁹, R⁷⁰ und R⁷¹ die oben angegebene Bedeutung haben,
in inerten Lösemitteln, gegebenenfalls in Anwesenheit einer Säure überführt,
und abschließend mit Bleitetraacetat / BF₃ x Ether oxidiert und cyclisiert,
oder
[B4] Verbindungen der allgemeinen Formel (IV-V) 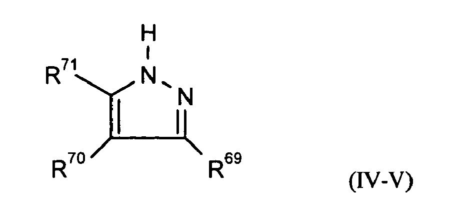 in welcher
R⁶⁹, R⁷⁰ und R⁷¹ die oben angegebene Bedeutung haben,
mit Verbindungen der allgemeinen Formel (IV-VI)
D⁴-CH₂-A⁴ (IV-VI)
in welcher
A⁴ die oben angegebene Bedeutung hat
und
D⁴ für Triflat oder Halogen, vorzugsweise für Brom steht,
in inerten Lösemitteln, gegebenenfalls in Anwesenheit einer Base umsetzt,
oder
[C4] Verbindungen der allgemeinen Formel (IV-VII) 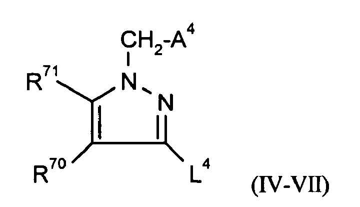 in welcher
A⁴, R⁷⁰ und R⁷¹ die oben angegebene Bedeutung haben,
und
L⁴ für einen Rest der Formel -SnR⁸⁷R⁸⁸R⁸⁹, ZnR⁹⁰, Iod oder Triflat steht,
worin
R⁸⁷, R⁸⁸ und R⁸⁹ gleich oder verschieden sind und
geradkettiges oder verzweigtes Alkyl mit bis zu 4 Kohlenstoffatomen bedeuten,
und
R⁹⁰ Halogen bedeutet,
mit Verbindungen der allgemeinen Formel (IV-VIII)
R⁶⁹-T⁴ (IV-VIII)
in welcher
R⁶⁹ die oben angegebene Bedeutung hat
und
im Fall L⁴ = SnR⁸⁷R⁸⁸R⁸⁹ oder ZnR⁹⁰
T⁴ für Triflat oder für Halogen, vorzugsweise für Brom steht,
und
im Fall L⁴ = Jod oder Triflat
T⁴ für einen Rest der Formel SnR^{87'}R^{88'}R^{89'}, ZnR^{90'} oder BR⁹¹R⁹²steht,
worin
R^{87'}, R^{88'}, R^{89'} und R^{90'} die oben angegebene Bedeutung von R⁸⁷, R⁸⁸, R⁸⁹ und R⁹⁰ haben und mit dieser gleich oder verschieden sind,
R⁹¹ und R⁹² gleich oder verschieden sind und
Hydroxy, Aryloxy mit 6 bis 10 Kohlenstoffatomen oder geradkettiges oder verzweigtes Alkyl oder Alkoxy mit jeweils bis zu 5 Kohlenstoffatomen bedeuten, oder gemeinsam einen 5- oder 6-gliedrigen carbocyclischen Ring bilden,
in einer palladiumkatalysierten Reaktion in inerten Lösemitteln umsetzt,
oder
[D4] im Fall, dass R⁷² für einen Alkyl mit 2 bis 6 Kohlenstoffatomen steht, das 2-fach durch Hydroxy substituiert ist
Verbindungen der allgemeinen Formel (IV-Ia) 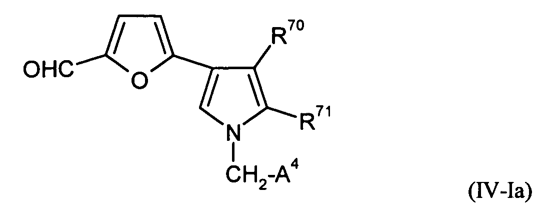 in welcher
A⁴, R⁷⁰ und R⁷¹ die oben angegebene Bedeutung haben,
zunächst durch eine Wittig-Reaktion im System (C₆H₅)₃P^{⊕}-CH₂- in die Verbindungen der allgemeinen Formel (IV-IX) 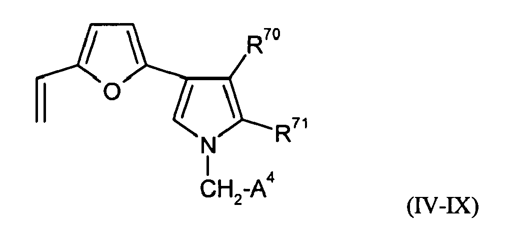 in welcher
R⁷⁰, R⁷¹ und A⁴ die oben angegebene Bedeutung haben,
überführt,
und abschließend mit Osmiumtetroxid die Hydroxyfunktionen einführt
und gegebenenfalls die unter R⁶⁹, R⁷⁰, R⁷¹ und/oder A⁴ aufgeführten Substituenten nach üblichen Methoden, vorzugsweise durch Reduktion, Oxidation, Abspaltung von Schutzgruppen und/oder nucleophiler Substitution variiert oder einführt.

26. Arzneimittel enthaltend mindestens eine Verbindung der allgemeinen Formel (IV-I) gemäß Anspruch 21.

27. Arzneizubereitungen enthaltend eine Kombination aus mindestens einer Verbindung aus der allgemeinen Formel (IV-I) gemäß Anspruch 21 und mindestens einem organischen Nitrat oder einem NO-Donator.

28. Arzneizubereitungen enthaltend eine Kombination aus mindestens einer Verbindung der allgemeinen Formel (IV-I) gemäß Anspruch 21 und Verbindungen die den Abbau von cyclischem Guanosinmonophosphat (cGMP) inhibieren.

29. Verwendung von Verbindungen der allgemeinen Formel (IV-I) gemäß Anspruch 21 bei der Herstellung von Arzneimitteln zur Behandlung von kardiovaskulären Erkrankungen.

30. Verwendung von Verbindungen der allgemeinen Formel (IV-I) gemäß Anspruch 21 bei der Herstellung von Arzneimitteln zur Prophylaxe und Bekämpfung der Folgen cerebraler Infarktgeschehen (Apoplexia cerebri) wie Schlaganfall, cerebraler Ischämien und des Schädel-Hirn-Traumas.
